# EUROPEAN PATENT SPECIFICATION

(11) **EP 4 031 520 B1**
(45) Date of publication and mention of the grant of the patent: **28.10.2026**
(21) Application number: 20864677.8
(22) Date of filing: 18.09.2020
(51) Int. Cl.: C07C 49/593, A61P 35/00, A61K 31/165, A61K 31/404, A61K 47/66, C07C 49/15, C07C 231/02, C07C 233/62, C07D 471/06

(54) **IMAGING AND THERAPEUTIC COMPOSITIONS**
BILDGEBUNGS- UND THERAPEUTISCHE ZUSAMMENSETZUNGEN
COMPOSITIONS D'IMAGERIE ET THÉRAPEUTIQUES

(30) Priority: 20.09.2019 AU 2019903504
(43) Date of publication of application: 27.07.2022
(73) Proprietor: The University of Melbourne, Victoria 3010 (AU)
(72) Inventor: DONNELLY, Paul Stephen, Melbourne, Victoria 3010 (AU); NOOR, Asif, Melbourne, Victoria 3010 (AU); RUDD, Stacey Erin, Melbourne, Victoria 3010 (AU)
(74) Representative: HGF
(86) International application number: PCT/AU2020/050992
(87) International publication number: WO 2021/051168

(56) References cited:
- WO-A1-2016/058056
- WO-A1-2016/058056
- STACEY E. RUDD ET AL: "A desferrioxamine B squaramide ester for the incorporation of zirconium-89 into antibodies", CHEMICAL COMMUNICATIONS, vol. 52, no. 80, 1 January 2016 (2016-01-01), UK, pages 11889 - 11892, XP055663525, ISSN: 1359-7345, DOI: 10.1039/C6CC05961A
- RUDD, S.E. ET AL.: "A desferrioxamine B squaramide ester for the incorporation of zirconium-89 into antibodies", CHEMICAL COMMUNICATIONS, vol. 52, no. 80, pages 11889 - 11892, XP055663525, DOI: 10.1039/C6CC05961A

## Description

### Field of the invention

The present invention relates to hydroxamic acid-based compounds that are useful as imaging and therapeutic agents when bound to an appropriate metal centre, particularly for the imaging and treatment of tumours. The present invention also relates to compositions including the compounds, for use in methods of imaging and treating patients using the compounds.

### Background of the invention

Zirconium-89 (⁸⁹Zr) is a positron-emitting radionuclide that is used in medical imaging applications. In particular, it is used in positron emission tomography (PET) for cancer detection and imaging. It has a longer half-life (t_{1/2} = 79.3 hours) than other radionuclides used for medical imaging, such as ¹⁸F. For example, ¹⁸F has a t_{1/2} of 110 minutes, which means that its use requires close proximity to a cyclotron facility and rapid and high-yielding synthesis techniques for the preparation of the agents into which it is incorporated. ⁸⁹Zr is not plagued by these same problems, which makes ⁸⁹Zr particularly attractive for use in medical imaging applications.

Desferrioxamine (DFO) is a bacterial siderophore that has been used since the late 1960s to treat iron overload. The three hydroxamic acid groups in DFO form co-ordination bonds with Fe³⁺ ions, essentially making DFO a hexadentate ligand that chelates the Fe³⁺ ions. Due to the co-ordination geometry of ⁸⁹Zr, DFO has also been used as a chelator for ⁸⁹Zr in PET imaging applications (Holland, J. P. et al (2012) Nature 10:1586).

Other DFO-based radioisotope chelators have also been prepared for use in PET imaging applications. These include N-succinyl-desferrioxamine-tetrafluorophenol ester (N-suc-DFO-TFP ester) p-isothiocyanatobenzyl-desferrioxamine (DFO-Bz-NCS, also known as DFO-Ph-NCS), desferrioxamine-maleimide (DFO-maleimide) and desferrioxamine-squaramide (DFO-sq). All of these chelators can be conjugated with antibodies or antibody fragments to provide a means of targeting the imaging agent to the tumour to be imaged.

WO2016/058056 describes hydroxamic acid-based compounds that are useful as imaging agents when bound to an appropriate metal centre, particularly for the imaging of tumours. Rudd et al. (ChemComm. 52 (2016) 11889-11892) describe a desferrioxamine B squaramide ester for the incorporation of zirconium-89 into antibodies.

However, there remains a need to develop new agents for use with radioisotopes, with improved biocompatibility properties, including enhanced tumour affinity and low uptake in off-target tissues.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

The present inventors have found that the compound of formula (I) or pharmaceutically acceptable derivative thereof set out below, and its conjugate with a prostate specific membrane antigen (PSMA) targeting agent (when complexed to a radionuclide such as ⁸⁹Zr or ⁶⁸Ga), is an effective PET imaging or therapeutic agent: 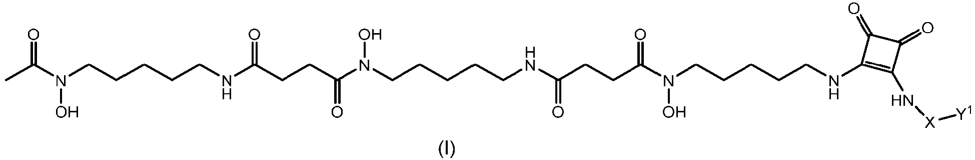 wherein X is selected from the group consisting of:
aryl optionally substituted with C₁-C₁₀ alkyl, ethylenediaminetetraacetic acid (EDTA); and
(C₁-C₁₀ alkyl)NR⁷(Y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
Y¹ and Y² are independently selected from the group consisting of: carboxylic acid, ester, anhydride, and amine; and
wherein the pharmaceutically acceptable derivative of formula (I) is:

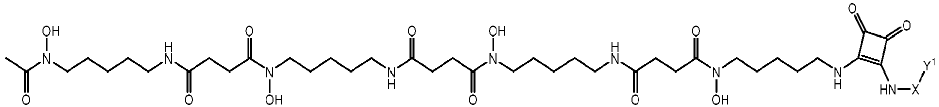

Therefore, in a first aspect, the present invention relates to a compound of formula (I) or pharmaceutically acceptable derivative thereof, as defined herein, or a pharmaceutically acceptable salt thereof: 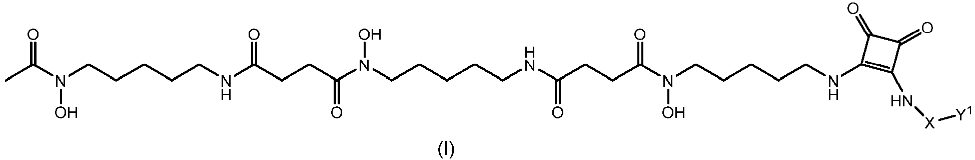 or a pharmaceutically-acceptable salt thereof, wherein X is selected from the group consisting of:
aryl optionally substituted with C₁-C₁₀ alkyl, ethylenediaminetetraacetic acid (EDTA); and
(C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
Y¹ and Y² are independently selected from the group consisting of: carboxylic acid, ester, anhydride, and amine; and
wherein the pharmaceutically acceptable derivative of formula (I) is:

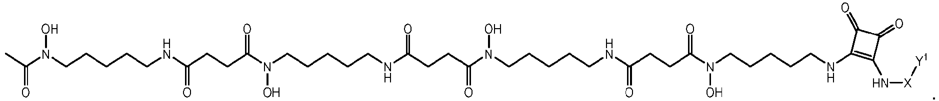

In a second aspect, the present invention relates to a conjugate comprising:
- a compound of formula (I) or pharmaceutically acceptable derivative thereof according to the first aspect, and
- a PSMA targeting agent.

In a third aspect, the present invention relates to conjugate of formula (II) or pharmaceutically acceptable derivative thereof: 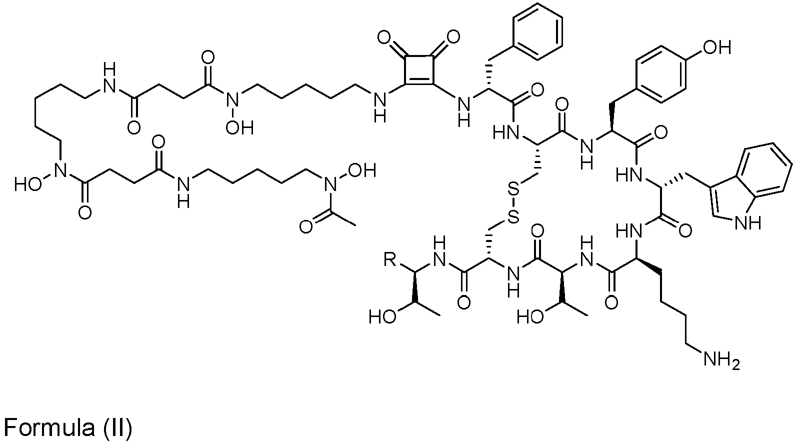 or a pharmaceutically acceptable salt thereof, wherein R is CH₂OH or COOH; and wherein the pharmaceutically acceptable derivative of formula (II) is

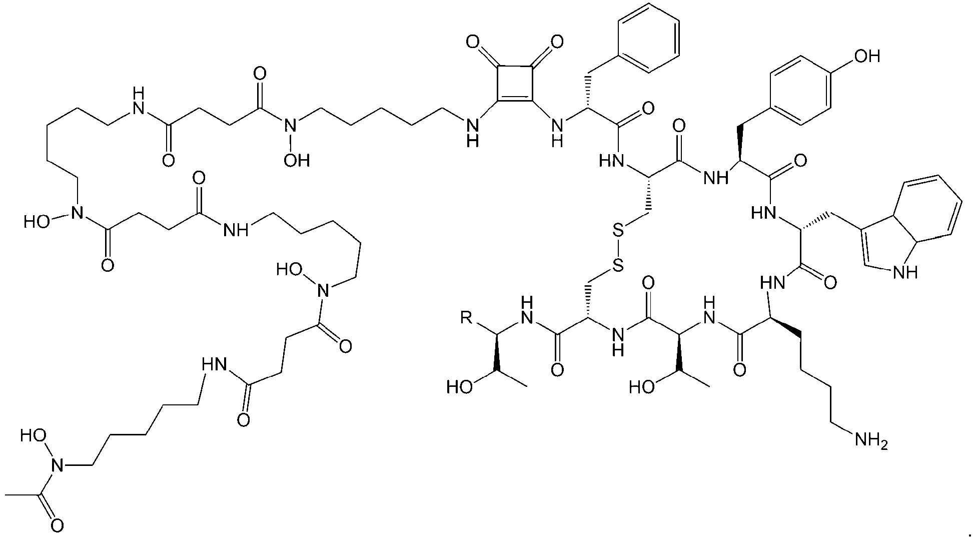

**In** a fourth aspect, the present invention relates to a radionuclide-labelled conjugate comprising:
- a conjugate or pharmaceutically acceptable derivative thereof according to the second or third aspect, and
- a radionuclide complexed thereto,
wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).

**In** a fifth aspect, the present invention relates to a pharmaceutical composition comprising the radionuclide-labelled conjugate of the fourth aspect, and one or more pharmaceutically acceptable carrier substances, excipients and/or adjuvants, preferably wherein the pharmaceutical composition further comprises a drug having anti-cancer activity.

**In** a sixth aspect, the present invention relates to a radionuclide-labelled conjugate of the fourth aspect, for use in a method of imaging a patient, the method including:
- administering to a patient the radionuclide-labelled conjugate; and
- imaging said patient.

**In** a seventh aspect, the present invention relates to a radionuclide-labelled conjugate of the fourth aspect, for use in a method of imaging a cell or *in vitro* biopsy sample, the method including:
- administering to a cell or *in vitro* biopsy sample the radionuclide-labelled conjugate; and
- imaging the cell or *in vitro* biopsy sample.
**In** an eighth aspect, the present invention relates to a radionuclide-labelled conjugate of the fourth aspect, for use in the treatment of cancer in a patient.
**In** a ninth aspect, the present invention relates to a kit for use in the method or use of the sixth, seventh or eighth aspect, the kit comprising:
- a compound according to the first aspect, a conjugate according to the second or third aspect, a radionuclide-labelled conjugate of the fourth aspect, or a pharmaceutical composition of the fifth aspect; and
- optionally, a label or package insert with instructions for use in the method or use of the sixth, seventh or eighth aspect.

**In** a preferred embodiment, X is selected from the group consisting of: phenyl, benzyl, and EDTA functionalised phenyl. More preferably, X is selected from the group consisting of: 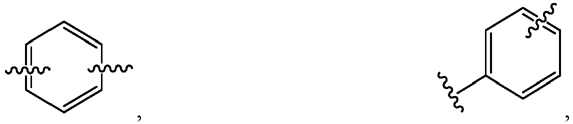 and

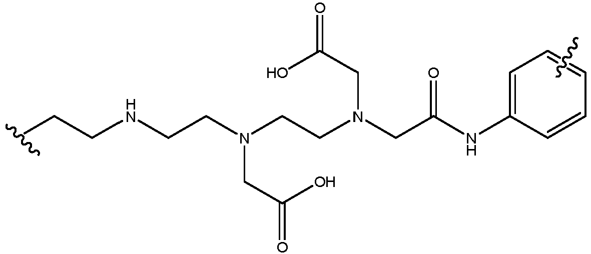

In a preferred aspect of this embodiment, Y¹ is carboxylic acid.

In another preferred embodiment, X is (C₁-C₁₀ alkyl)NR⁷(Y²)R⁸ More preferably, X is selected from the group consisting of: 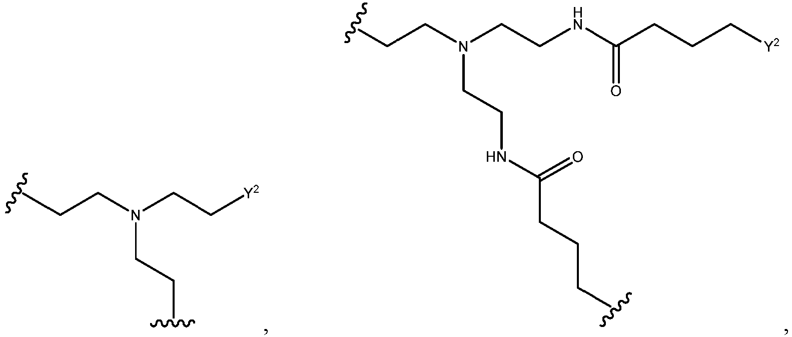 and

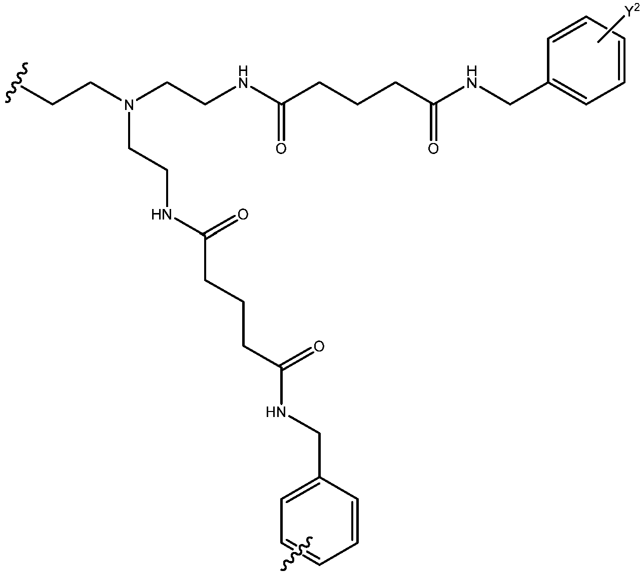

In a preferred aspect of this embodiment, Y¹ and Y² are independently amine or carboxylic acid. Preferably, Y¹ and Y² are the same.

Preferably, a compound of formula (I) or pharmaceutically acceptable derivative thereof, or a pharmaceutically acceptable salt thereof, may be selected from the group consisting of: 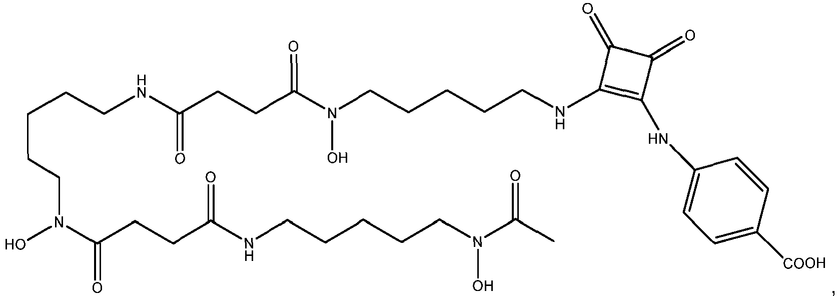 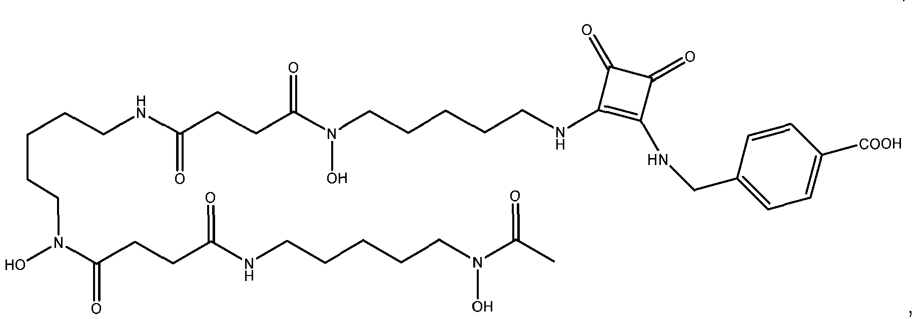 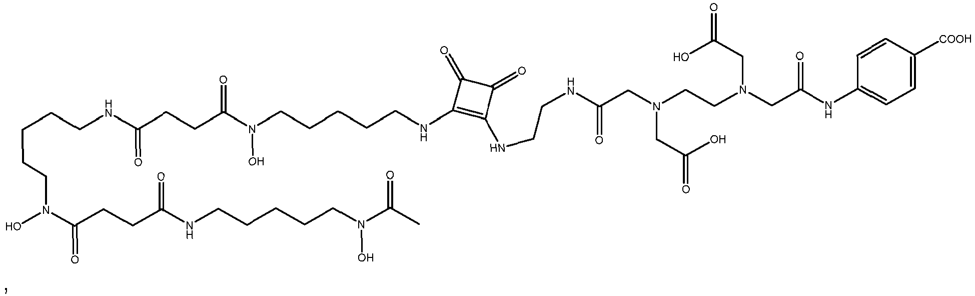 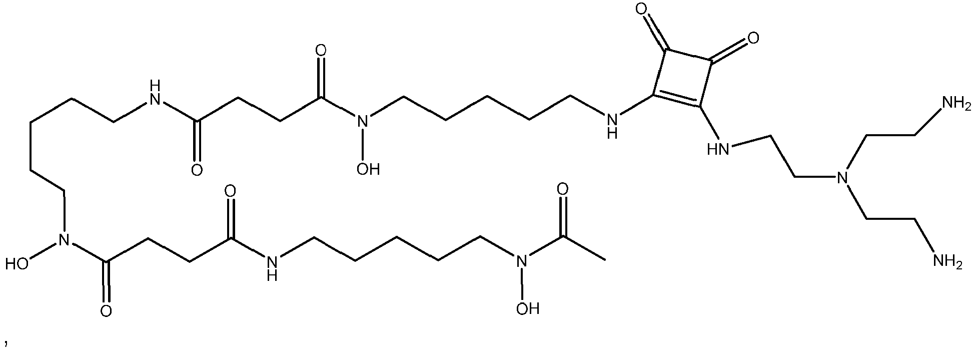 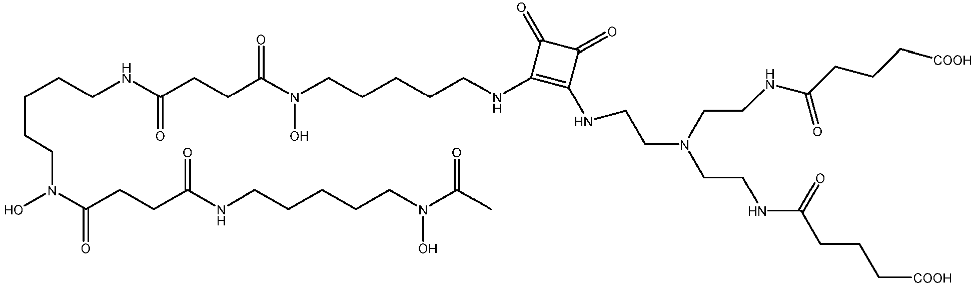 , and

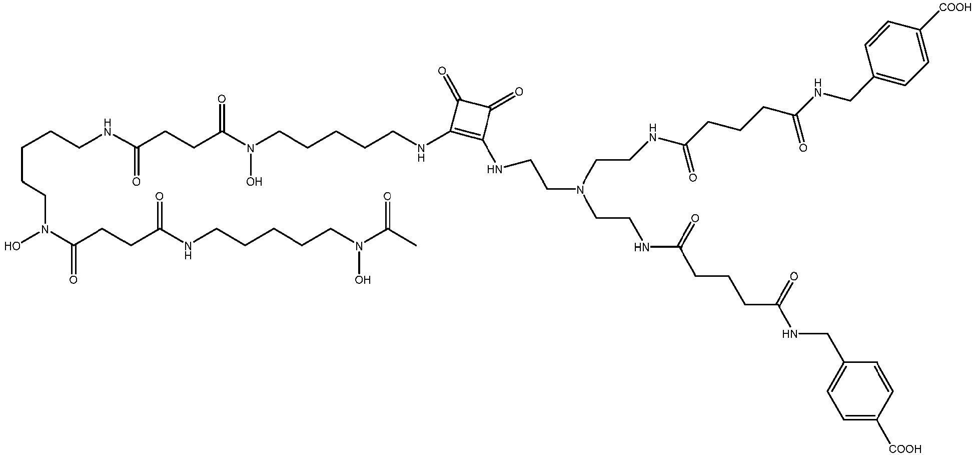

In another aspect, the present invention also relates to a conjugate of:
- a compound of formula (I) or pharmaceutically acceptable derivative thereof: 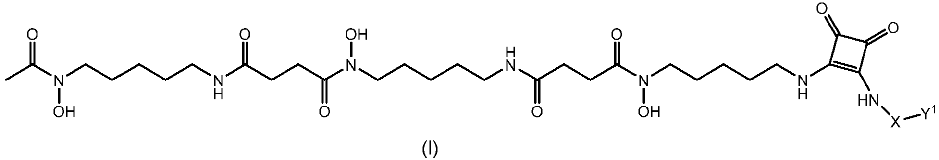 or a pharmaceutically-acceptable salt thereof, wherein X is selected from the group consisting of:
   aryl optionally substituted with C₁-C₁₀ alkyl, ethylenediaminetetraacetic acid (EDTA); and
   (C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
   Y¹ and Y² are independently selected from the group consisting of: carboxylic acid, ester, anhydride, and amine; and
   wherein the pharmaceutically acceptable derivative of formula (I) is: 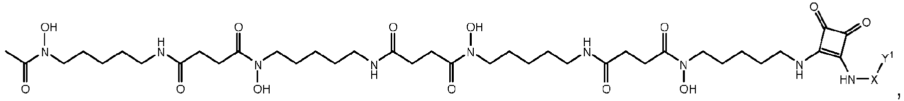 and
- a PSMA targeting agent.

The PSMA targeting agent is a moiety with selectivity to prostate specific membrane antigen. Preferably the PSMA targeting agent is a peptide or a urea-linked dipeptide, more preferably Lys-Urea-Glu.

Preferably, conjugates of a compound of formula (I) and a PSMA targeting agent, or a pharmaceutically acceptable salt thereof, may be selected from the group consisting of: 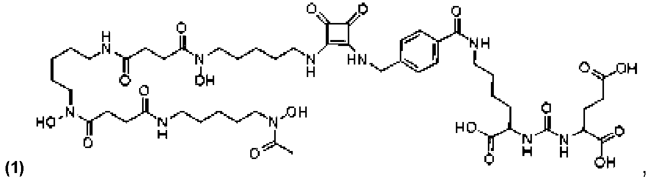 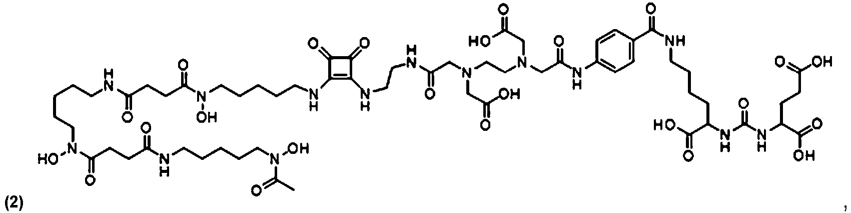 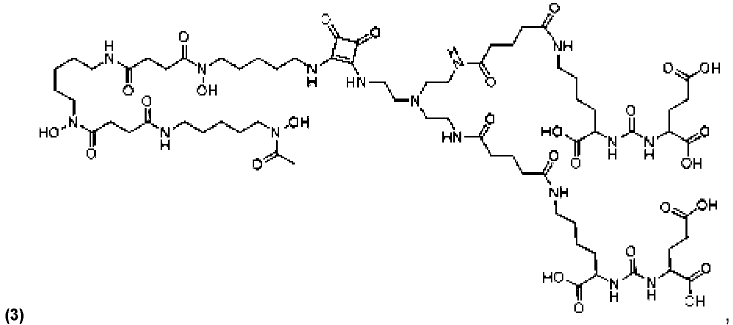 and

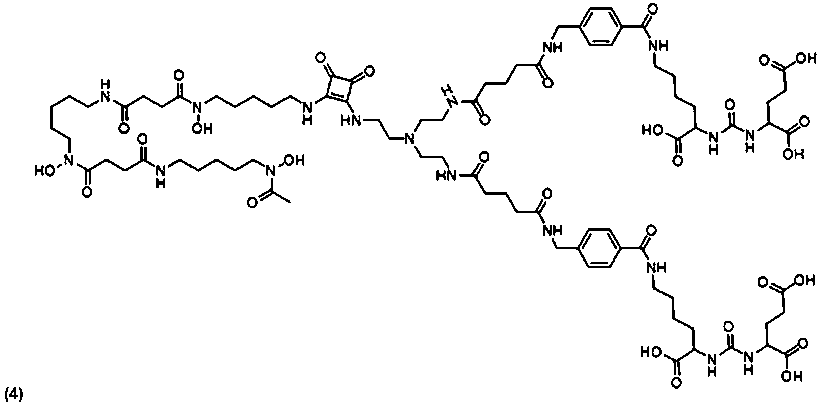

In another aspect, the present invention relates to a radionuclide-labelled conjugate of:
- a compound of formula (I) or pharmaceutically acceptable derivative thereof: 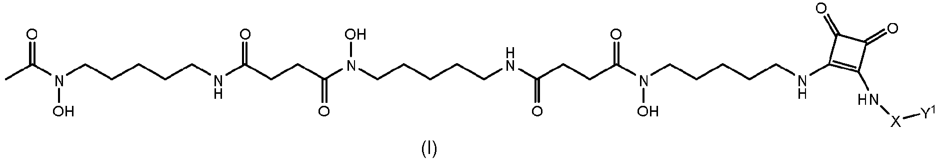 or a pharmaceutically-acceptable salt thereof, wherein X is selected from the group consisting of:
   aryl optionally substituted with C₁-C₁₀ alkyl, ethylenediaminetetraacetic acid (EDTA); and
   (C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
   Y¹ and Y² are independently selected from the group consisting of: carboxylic acid, ester, anhydride, and amine; and
   wherein the pharmaceutically acceptable derivative of formula (I) is: 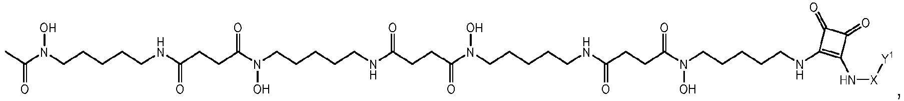
- a PSMA targeting agent, and
- a radionuclide complexed thereto,
wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).

The PSMA targeting agent is a moiety with selectivity to prostate specific membrane antigen. The PSMA targeting agent may be a peptide. Preferably the PSMA targeting agent is a urea-linked dipeptide, more preferably Lys-Urea-Glu.

In another aspect, the invention provides a conjugate of formula (II) or pharmaceutically acceptable derivative thereof: 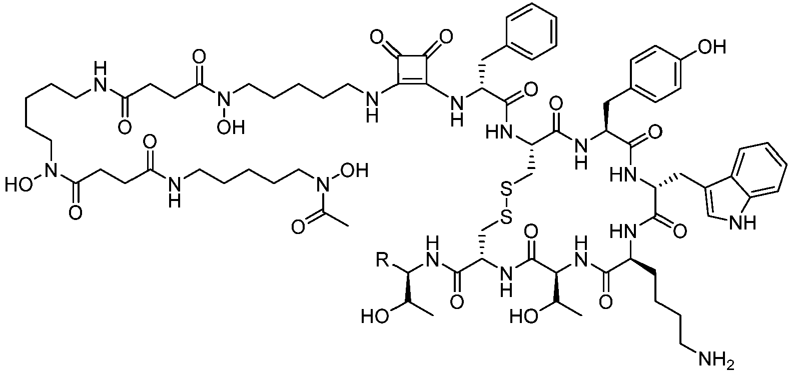 or a pharmaceutically-acceptable salt thereof, wherein R is CH₂OH or COOH; and wherein the pharmaceutically acceptable derivative of formula (II) is

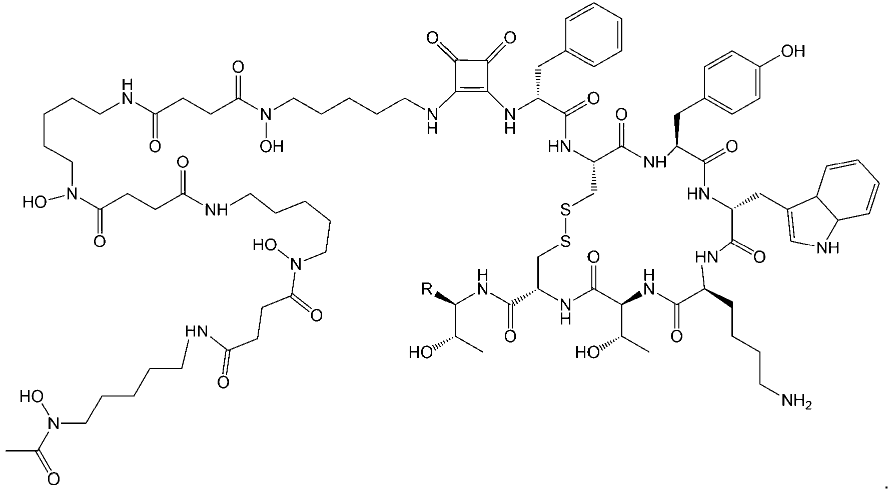

In one embodiment, R is CH₂OH. In another embodiment, R is COOH.

In another aspect, the present invention relates to a radionuclide-labelled conjugate of:
- a conjugate of formula (II) or pharmaceutically acceptable derivative thereof: 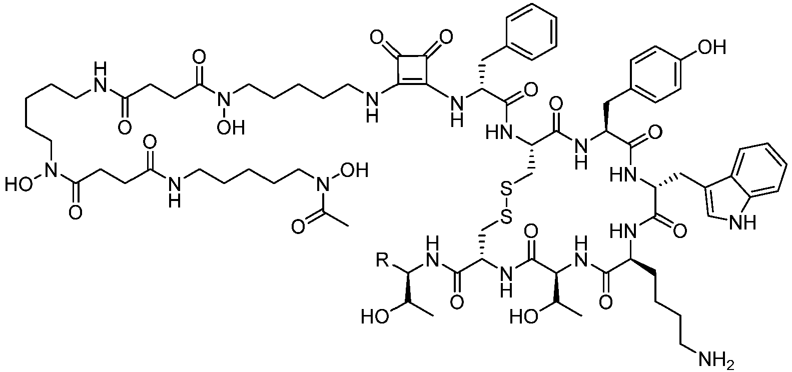 or a pharmaceutically-acceptable salt thereof, wherein R is CH₂OH or COOH; and wherein the pharmaceutically acceptable derivative of formula (II) is 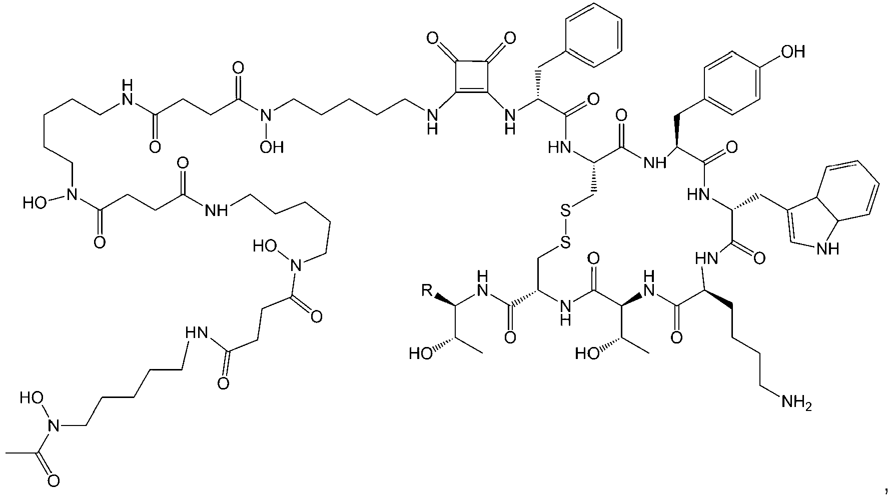 and
- a radionuclide complexed thereto,
wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).

In any aspect or embodiment of the invention, the DFO in formula (I) or (II) of a compound, conjugate or radionuclide conjugate of the invention, or any structure shown herein, may be substituted for a pharmaceutically acceptable derivative thereof, for example, DFO-analogues such as DFO*. Pharmaceutically acceptable derivatives of formula (I) and formula (II) are: 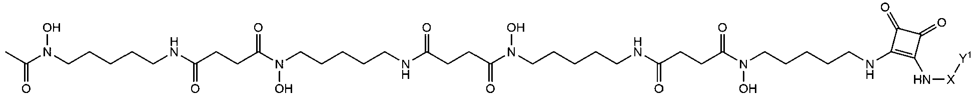 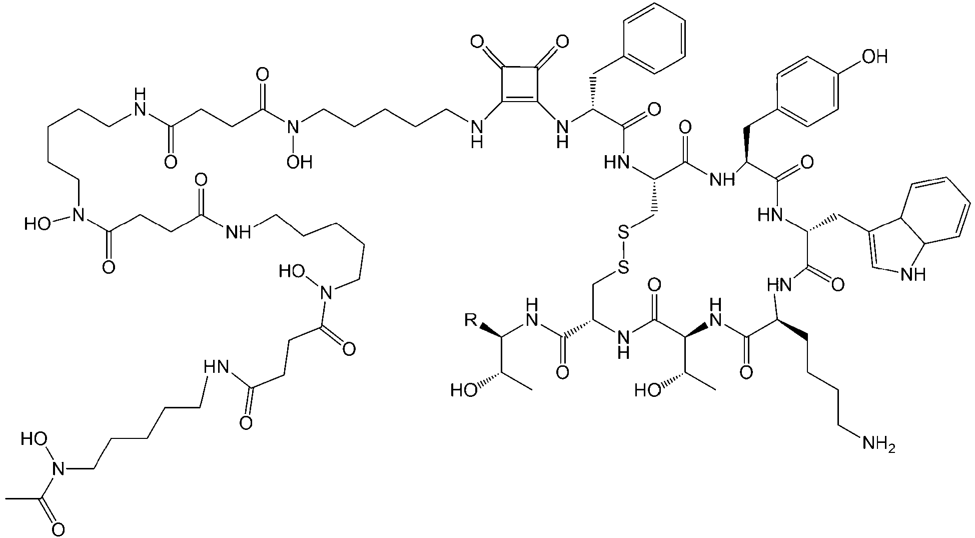 In any embodiment of the above aspects, the radioisotope may be a diagnostic radioisotope suitable for PET imaging. The radionuclide may be a radioisotope of zirconium, gallium or indium. The radioisotope of zirconium may be ⁸⁹Zr. The radioisotope of gallium may be ⁶⁸Ga. The radioisotope of indium may be ¹¹¹1n.

In any embodiment of the above aspects, the radioisotope may be a therapeutic radioisotope. The radionuclide may be a radioisotope of gallium, lutetium, scandium, titanium, manganese or indium. The radioisotope of gallium may be ⁶⁷Ga. The radioisotope of lutetium may be ¹⁷⁷Lu. The radioisotope of scandium may be ^{43/44}Sc. The radioisotope of titanium may be ⁴⁵Ti. The radioisotope of manganese may be ⁵²Mn. The radioisotope of indium may be ¹¹¹In.

In another aspect, the present invention relates to a radionuclide-labelled conjugate, as defined herein, for use in a method of imaging a patient, the method including:
- administering to a patient the radionuclide-labelled conjugate, as defined herein, and
- imaging the patient.

In another aspect, the present invention relates to a radionuclide-labelled conjugate, as defined herein, for use in a method of imaging a cell or *in vitro* biopsy sample, the method including:
- administering to a cell or *in vitro* biopsy sample the radionuclide-labelled conjugate, as defined herein, and
- imaging the cell or *in vitro* biopsy sample.

In another aspect, the present invention relates to a radionuclide-labelled conjugate, as defined herein, for use in the treatment of cancer in a patient.

As used herein, except where the context requires otherwise, the term "comprise" and variations of the term, such as "comprising", "comprises" and "comprised", are not intended to exclude further additives, components, integers or steps. As used herein "including" and "comprising" are used interchangeably.

Further aspects of the present invention and further embodiments of the aspects described in the preceding paragraphs will become apparent from the following description, given by way of example and with reference to the accompanying drawings.

### Brief description of the drawings

**Figure 1****:** Structures of illustrative mono- and dimeric PSMA ligands.
**Figure 2****:** Representative whole body microPET and CT images of mice bearing LNCaP xenograft tumours after 1 and 18 hours post injection of ⁸⁹Zr-DFOSq PSMA tracers (CT was not performed for ⁸⁹Zr-(**2**)).
**Figure 3****:** a) Time activity curve of tracer uptake into LNCaP tumours. PET images from each study were analysed and results are shown as mean ± SEM, n = 3. Data were analysed (t-test) for differences in tumour SUVₘₐₓ at each time point. n.s. P = not significant, *P<0.05; curves from top to bottom: ⁸⁹Zr-(4), ⁸⁹Zr-(3), ⁸⁹Zr-(1) and ⁸⁹Zr-(2), b) Ex-vivo biodistribution analysis. Mice were euthanised at 1 and 18 hr after injection with and tracer uptake is expressed as percent injected dose/gram tissue. Data represents mean ± SEM of n = 3 mice/group.
**Figure 4****:** *Ex-vivo* biodistribution analysis. Mice were euthanised at 1 and 18 hr after injection. Tissues were harvested, weighed and counted on a gamma counter. Tracer uptake is expressed as percent injected dose/gram tissue. Data represents mean ± SEM of n = 3 mice/group.
**Figure 5****:** Representative whole body microPET and CT images of mice bearing LNCaP xenograft tumours after 1 and 2 hours post injection of ⁶⁸Ga-DFOSq PSMA tracers.

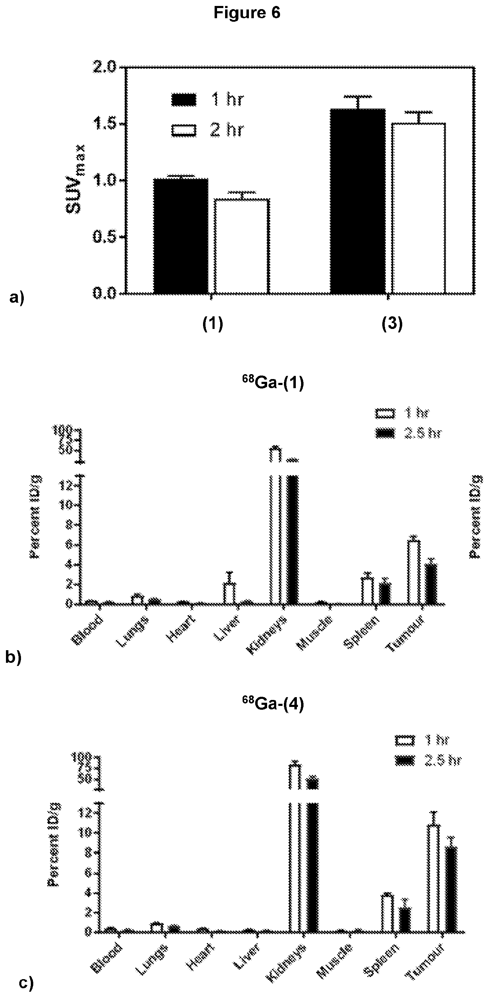

**Figure 6****:** (left) Uptake of PSMA tracers into LNCaP tumours *in vivo.* PET images from each study were analysed and results are shown as mean ± SEM, n = 3, (right) *Ex-vivo* biodistribution analysis. Mice were euthanised at 1 and 2.5 hr p.i. with **(1)** (upper panel) or **(3)** (lower panel). Tissues were harvested, weighed and counted using a gamma counter. Tracer uptake is expressed as percent injected dose/gram tissue. Data represents mean ± SEM of n = 3 mice/group.

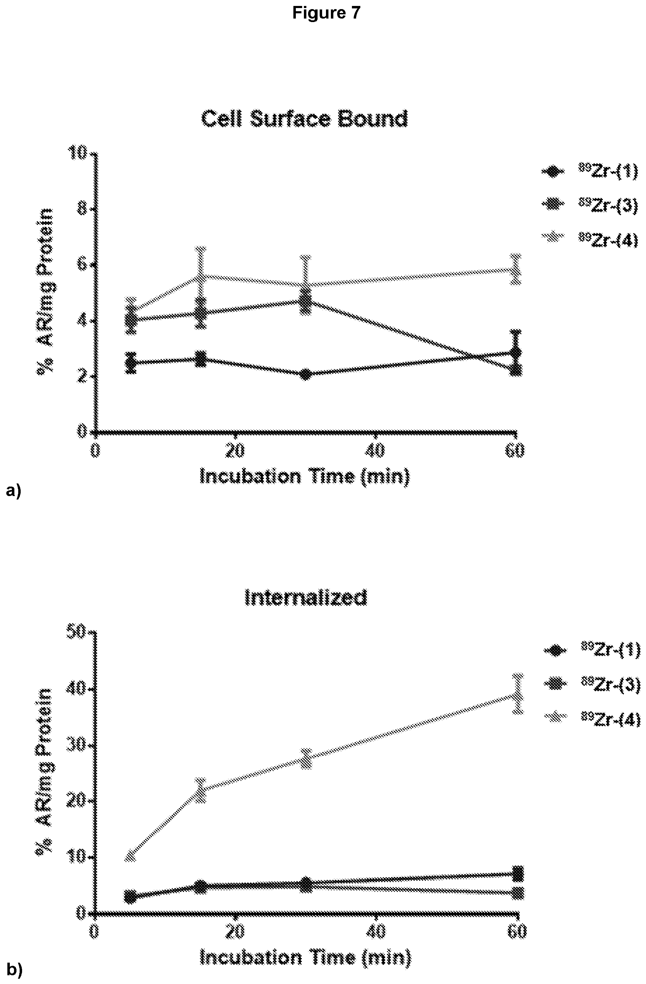

**Figure 7****:** PSMA-positive LNCap cell uptake of ⁸⁹Zr-PSMA tracers, Left panel, cell surface bound activity and right panels, internalised activity. Uptake is expressed as the percentage of added radioactivity (AR)/mg protein. The results represent the mean ± SEM, n= 3 technical replicates.

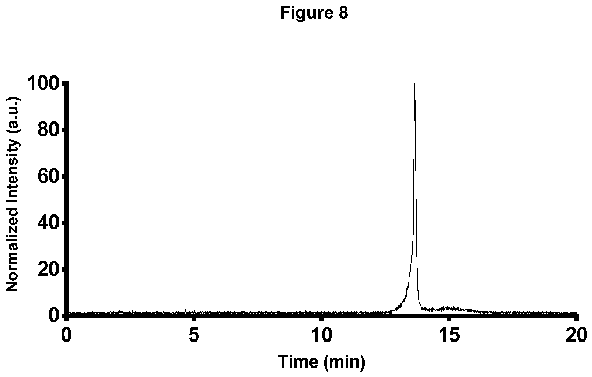

**Figure 8****:** Radio-HPLC trace of ⁸⁹Zr-(**3)**.

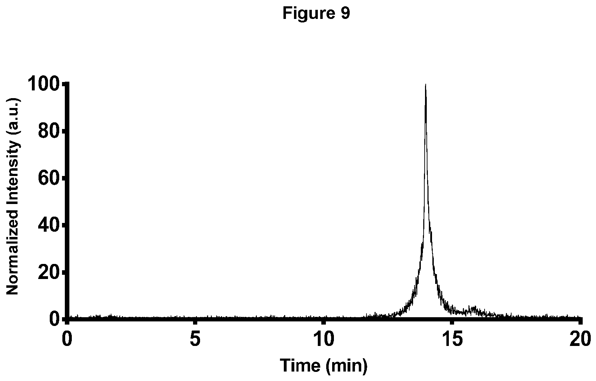

**Figure 9****:** Radio-HPLC trace of ⁸⁹Zr-(**4)**.
**Figure 10****:** Structures of DFO-Sq conjugated octreotate and octreotide molecules.
**Figure 11****:** Radio-HPLC traces of ⁸⁹Zr-DFOSqTIDE/TATE peptides (Method, ⁸⁹Zr-DFOSqTIDE: 20-100% Buffer B (0.05% TFA ACN) to A (0.05% TFA in MilllQ) over 15 min ⁸⁹Zr-DFOSqTATE: 0-95% Buffer B (0.05% TFA ACN) to A (0.05% TFA in MilllQ) over 15 min. Top trace is ⁸⁹Zr-DFOSqTIDE, bottom trace is ⁸⁹Zr-DFOSqTATE.
**Figure 12****:** Representative PET images of mice injected with ⁸⁹Zr-DFOSqTIDE and ⁸⁹Zr-DFOSqTATE imaged at different time points.
**Figure 13****:** a) Time activity curve of tracer uptake into AR42J Tumours and tumour to liver ratio. PET images from each study were analysed and results are shown as mean ± SEM, n = 3. Data were analysed (t-test) for differences in tumour SUVₘₐₓ at time point. ** P<0.01; b) *Ex-vivo* biodistribution analysis. Mice were euthanized at 1 and 18 hr after injection with DFOSqTIDE or DFOSqTATE. Tissues were harvested, weighed and counted on a gamma counter. Tracer uptake is expressed as percent injected dose/gram tissue. Data represents mean ± SEM of n = 3 mice/group.
**Figure 14****:** Radio-HPLC traces of ⁶⁸Ga-DFOSqTIDE/TATE peptides (Method, 20-100% Buffer B (0.05% TFA ACN) to A (0.05% TFA in MilllQ) over 15 min. Top trace is ⁶⁸Ga-DFOSqTIDE, bottom trace is ⁶⁸Ga-DFOSqTATE.
**Figure 15****:** Representative PET images of mice injected with ⁶⁸GaDFOSq-TIDE and ⁶⁸GaDFOSq-TATE tracers imaged at different time points. Quantification of the uptake in the images using Standard Uptake Value (SUVₘₐₓ = radioactivity in a tissue/injected activity/BW) is shown in (c).
**Figure 16****:** Ex vivo biodistribution study in mouse model where mice were injected with either ⁶⁸GaDFOSq-TIDE and ⁶⁸GaDFOSq-TATE (2-3 MBq, 1 µg, 0.5 nmol) and then euthanised at either 1 h or 2 h after administration. The amount of injected activity per gram of tissue (%IA/g) in the tumor and major organs was quantified.

### Detailed description of the embodiments

It will be understood that the invention disclosed and defined in this specification extends to all alternative combinations of two or more of the individual features mentioned or evident from the text or drawings. All of these different combinations constitute various alternative aspects of the invention.

Further aspects of the present invention and further embodiments of the aspects described in the preceding paragraphs will become apparent from the following description, given by way of example and with reference to the accompanying drawings.

Reference will now be made in detail to certain embodiments of the invention. While the invention will be described in conjunction with the embodiments, it will be understood that the intention is not to limit the invention to those embodiments.

The present invention relates to compounds of formula (I) or pharmaceutically acceptable derivative thereof as defined herein, its conjugate with a prostate specific membrane antigen (PSMA) targeting agent, and radionuclide conjugates thereof.

In one aspect, the invention provides compounds of formula (I) or pharmaceutically acceptable derivatives thereof: 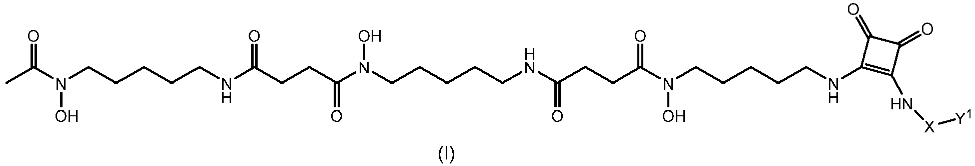 wherein X is selected from the group consisting of:
aryl optionally substituted with C₁-C₁₀ alkyl, ethylenediaminetetraacetic acid (EDTA); and
(C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
Y¹ and Y² are independently selected from the group consisting of: carboxylic acid, ester, anhydride, and amine; and
wherein the pharmaceutically acceptable derivative of formula (I) is:

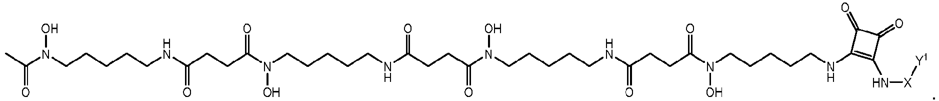

Compounds of formula (I) are derivatives of "DFO-squaramide" (also herein referred to as "DFOSq") suitable for conjugation with a PSMA targeting agent. The inventors have surprisingly found that including an aromatic or aryl linker group between the DFO-based moiety and the targeting agent improves tumour targeting. Improved tumour targeting was also achieved by conjugating multiple targeting agents to a DFO-based moiety. Surprisingly, the tumour targeting improved further by conjugating multiple targeting agents to a DFO-based moiety via an aromatic linker group.

Compounds of formula (I) that include a PSMA targeting agent in a dimeric arrangement surprisingly showed improved tumour targeting. Advantageously compounds of formula (I) that include a PSMA targeting agent in a dimeric arrangement showed both a higher total uptake of the compound in the tumour and increase specificity towards the targeted tumour. Compounds of formula (I) that include a PSMA targeting agent in a dimeric arrangement showed longer retention times in the tumour up to 2 hours post injection compared to targeting agents with a monomeric arrangement.

Pharmaceutically acceptable derivatives thereof of formula (I) may be prepared from DFO-analogues, such as DFO* (Patra et al. Chem. Commun., 2014, 50, 11523-11525). DFO* is a DFO-analogue and is an extended version of DFO that has an additional hydroxamic acid and is readily prepared from DFO. DFO* is an octadentate chelator of Zn⁴⁺, and ⁸⁹Zn-DFO* complexes have been shown to exhibit increased in *vitro* stability compared to DFO complexes. DFO* and DFO*-squaramide are suitable for conjugation with a PSMA targeting agent. Additionally the DFO* derivatives coupled to model proteins have shown good tumour uptake and significantly lower bone uptake in tumour bearing mice. The improved *in vitro* stability and *in vivo* properties indicate that DFO*, DFO*-squaramide or derivatives thereof may also be suitable to be incorporated into compounds of the invention.

In any aspect or embodiment of the invention, the DFO in formula (I) or (II) of a compound, conjugate or radionuclide conjugate of the invention, or any structure shown herein, may be substituted for a pharmaceutically acceptable derivative thereof, for example, DFO-analogues such as DFO*. The structure of DFO* is shown below:

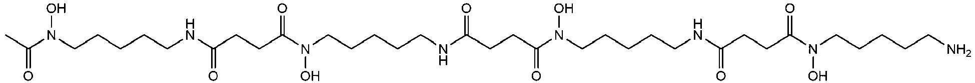

The DFO* analogue of the compound of formula (I) has a structure according the formula below:

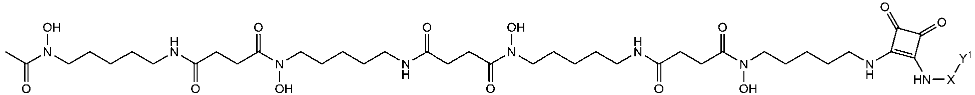

The DFO* analogue of the compound of formula (II) has a structure according the formula below:

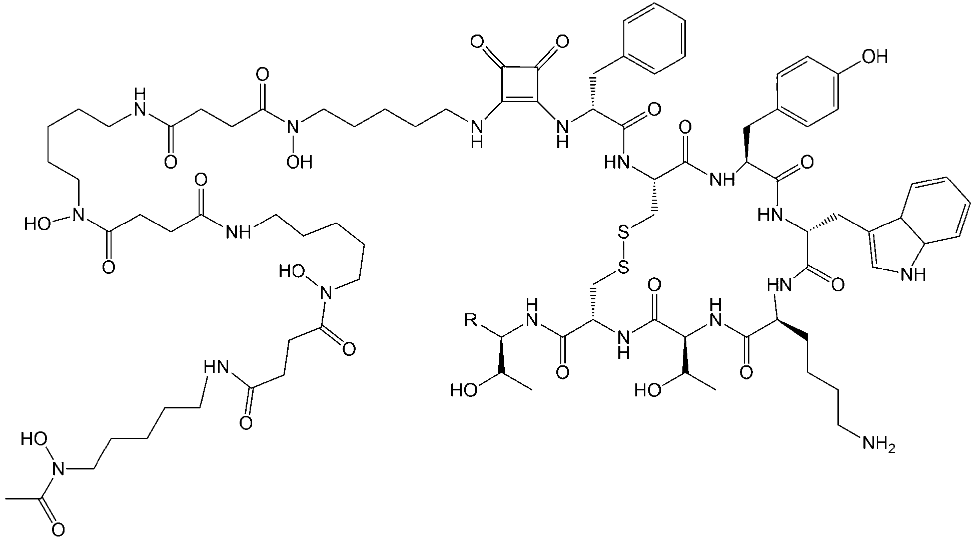

In any aspect or embodiment of the invention, the DFO* analogue of the compound of formula (I) or pharmaceutically acceptable derivative thereof, or a pharmaceutically acceptable salt thereof, may be selected from the group consisting of: 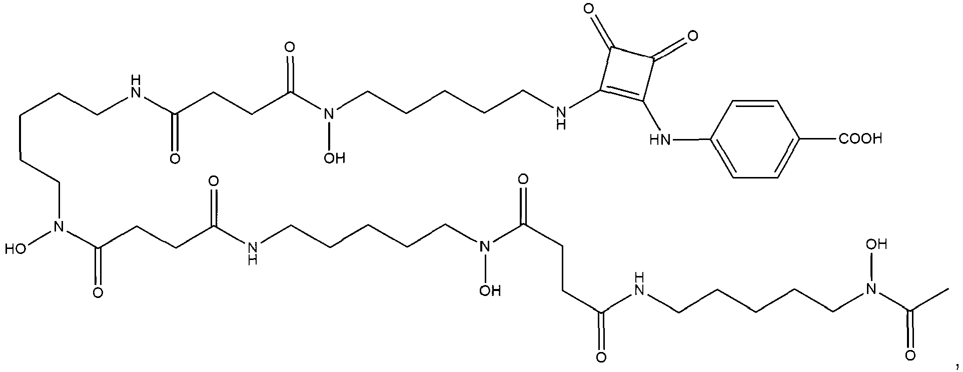 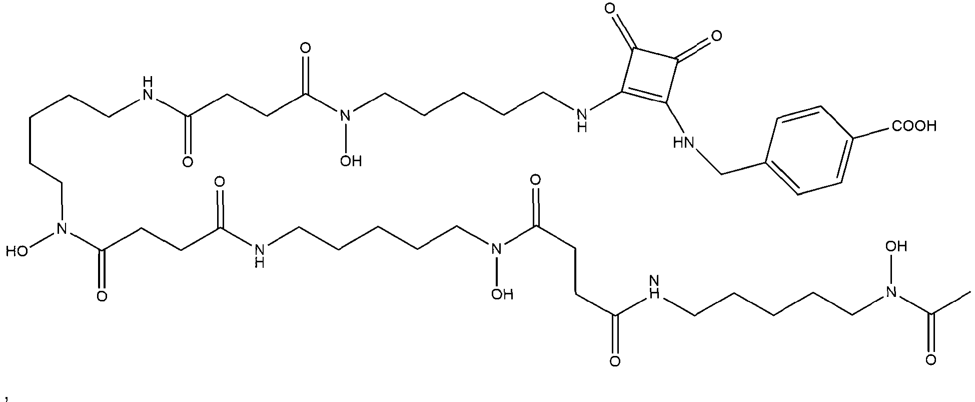 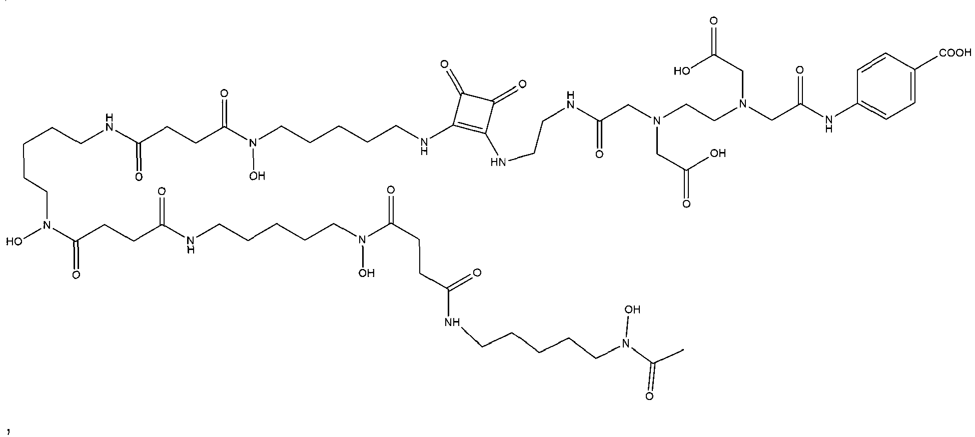 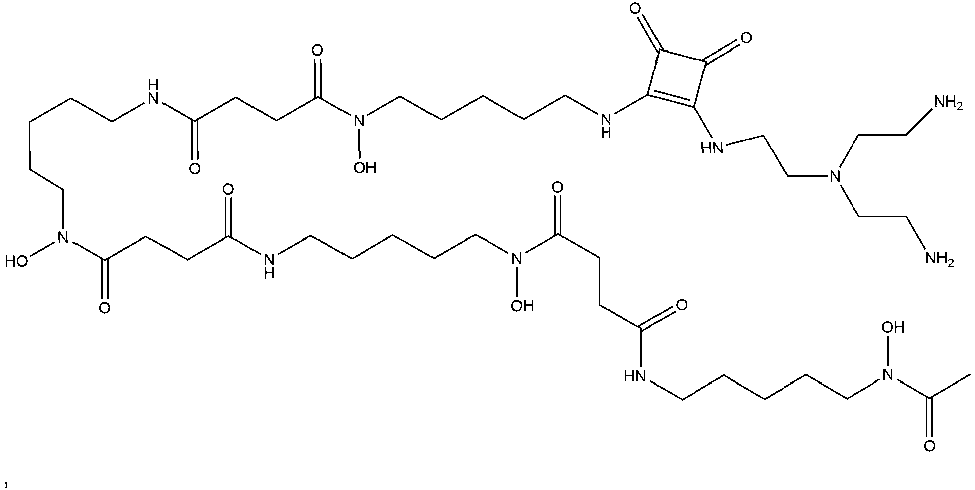 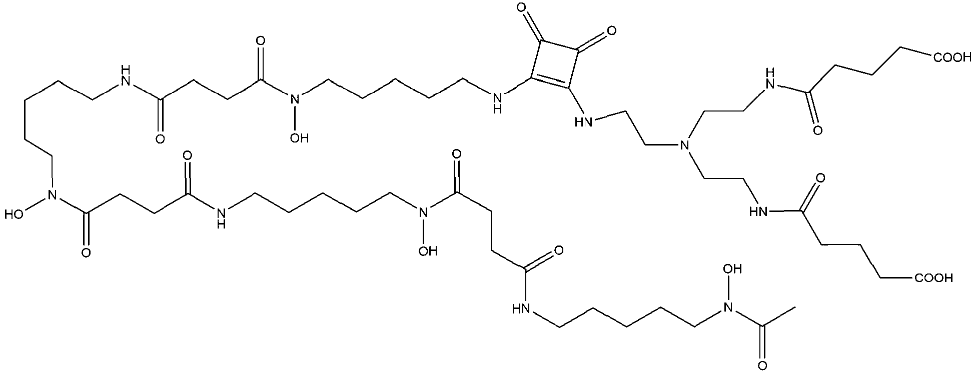 , and

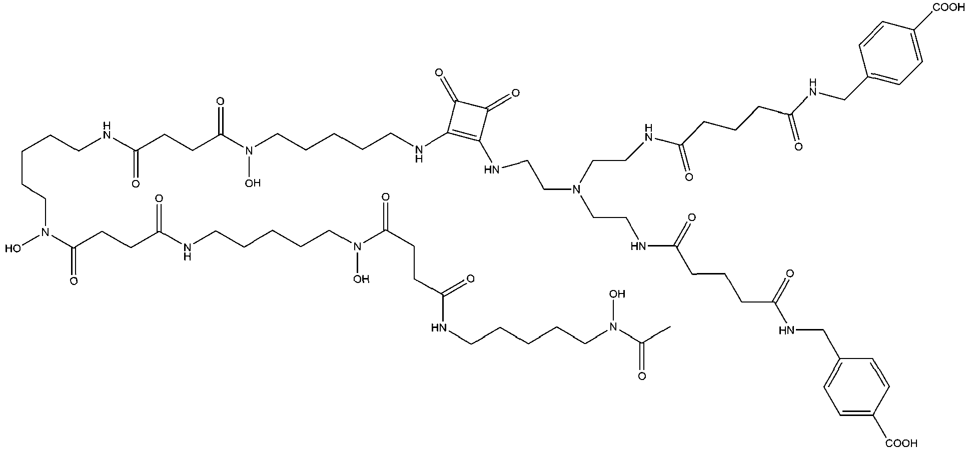

Compounds of formula (I) or pharmaceutically acceptable derivatives thereof that include both an aryl linker and a PSMA targeting agent in a dimeric arrangement surprisingly showed improved tumour targeting compared to compounds of formula (I) that include only the PSMA targeting agent in a dimeric arrangement.

Compounds of formula (I) or pharmaceutically acceptable derivatives thereof that include both an aryl linker and a PSMA targeting agent in a dimeric arrangement showed increased cell internalisation compared to compounds that include only the PSMA targeting agent in a dimeric arrangement.

Compounds of formula (I) or pharmaceutically acceptable derivatives thereof of the invention showed significant clearance (50% or greater) 18 hours post injection.

The present inventors have found that the radiolabelled conjugates of the compounds of formula (I) or pharmaceutically acceptable derivatives thereof with PSMA targeting agents exhibit improved tumour targeting and tissue selectivity over a number of the known radionuclide chelators (particularly other DFO-based chelators) that are used as PET imaging agents.

The present invention also provides conjugates of formula (II) or pharmaceutically acceptable derivatives thereof: 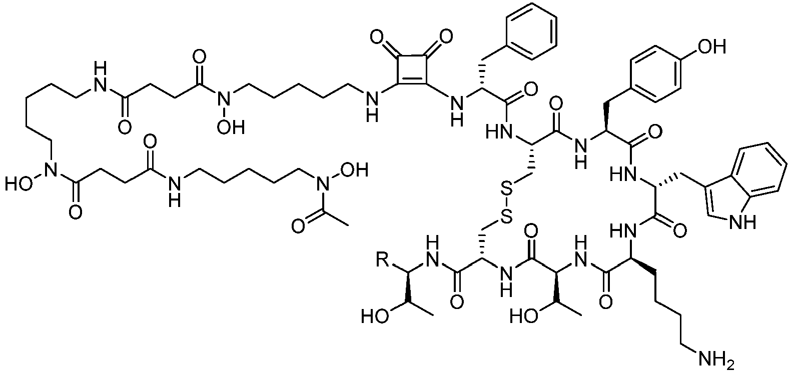
or pharmaceutically-acceptable salts thereof, wherein R is CH₂OH or COOH; and
wherein the pharmaceutically acceptable derivative of formula (II) is 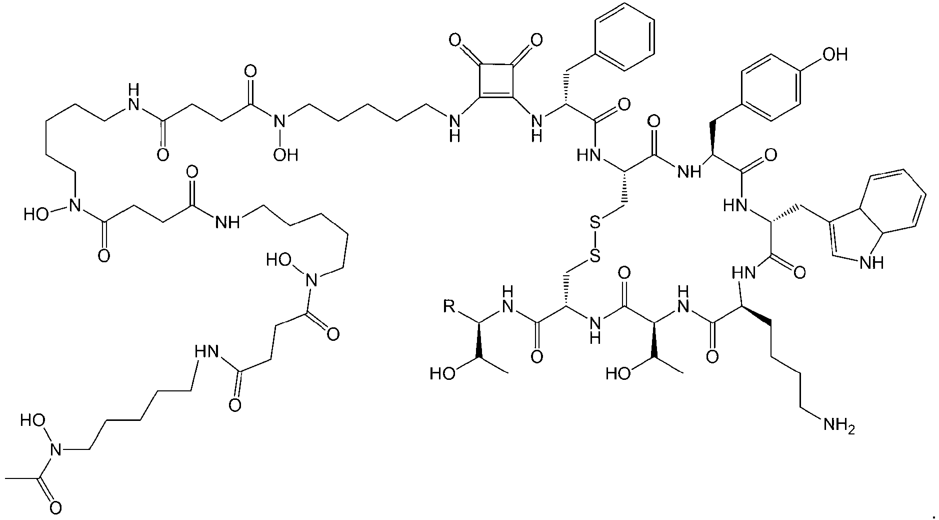
In one embodiment, R is CH₂OH. In another embodiment, R is COOH.

Conjugates of formula (II) are derivatives of "DFO-squaramide" (also herein referred to as "DFOSq") comprising a somatostatin subtype 2 receptor (sstr2) targeting agent. The term "sstr2 targeting agent" refers to a peptide that has the ability to target somatostatin subtype 2 receptor. The agent may be a peptide. In one embodiment, wherein R is CH₂OH, the sstr2 targeting agent is octreotide. In another embodiment, wherein R is COOH, the sstr2 targeting agent is octreotate.

Compounds of formula (II) or pharmaceutically acceptable derivatives thereof advantageously showed increased retention in tumors 2 hours post injection in the presence of sstr protein compared to tumours absent sstr protein.

Compounds of formula (II) wherein R is COOH advantageously provide even further improved tumour targeting. Compounds of formula (II) wherein R is COOH showed increased uptake in targeted tumours and increased retention 2 hours post injection. Compounds of formula (II) showed significant (50% or more) clearance 18 hours post injection.

A "pharmaceutically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzenesulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaieic, hydroiodic, phenylacetic, alkanoic (such as acetic, HOOC-(CH₂)ₙ-COOH where n is any integer from 0 to 6, i.e. 0, 1, 2, 3, 4, 5 or 6), and the like. Similarly, pharmaceutically acceptable cations include, but are not limited to sodium, potassium, calcium, aluminum, lithium and ammonium. A person skilled in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent (such as ether, ethyl acetate, ethanol, isopropanol or acetonitrile), or in a mixture of the two.

It will be apparent that the compounds or conjugates of formula (I) or (II) may, but need not, be present as a hydrate, solvate or non-covalent complex (to a metal other than the radionuclide). In addition, the various crystal forms and polymorphs are within the scope of the present invention, as are prodrugs of the compounds provided herein.

A "prodrug" is a compound that may not fully satisfy the structural requirements of the compounds provided herein, but is modified *in vivo,* following administration to a subject or patient, to produce a radiolabelled conjugate as provided herein. For example, a prodrug may be an acylated derivative of a radiolabelled conjugate. Prodrugs include compounds wherein hydroxyl or amine groups are bonded to any group that, when administered to a mammalian subject, cleaves to form a free hydroxyl or amine group, respectively. Examples of prodrugs include, but are not limited to, acetate, formate, phosphate and benzoate derivatives of amine functional groups within the radiolabelled conjugate. Prodrugs of the may be prepared by modifying functional groups present in the compounds in such a way that the modifications are cleaved in *vivo* to generate the parent compounds.

A "substituent" as used herein, refers to a molecular moiety that is covalently bonded to an atom within a molecule of interest. The term "substituted," as used herein, means that any one or more hydrogens on the designated atom is replaced with a selection from the indicated substituents, provided that the designated atom's normal valence is not exceeded, and that the substitution results in a stable compound, i.e., a compound that can be isolated, characterized and tested for biological activity. When a substituent is oxo, i.e., =O, then two hydrogens on the atom are replaced. An oxo group that is a substituent of an aromatic carbon atom results in a conversion of -CH- to - C(=O)- and a loss of aromaticity. For example a pyridyl group substituted by oxo is a pyridone. Examples of suitable substituents are alkyl, heteroalkyl, halogen (for example, fluorine, chlorine, bromine or iodine atoms), OH, =O, SH, SO₂, NH₂, NHalkyl, =NH, N₃ and NO₂ groups.

The term "optionally substituted" refers to a group in which one, two, three or more hydrogen atoms have been replaced independently of each other by alkyl, halogen (for example, fluorine, chlorine, bromine or iodine atoms), OH, =O, SH, =S, SO₂, NH₂, NHalkyl, =NH, N₃ or NO₂ groups.

Multiple degrees of substitution may be allowed, provided that the designated atom's normal valence is not exceeded, and that the substitution results in a stable compound, i.e., a compound that can be isolated, characterised and tested for biological activity.

As used herein a wording defining the limits of a range of length such as, for example, "from 1 to 10" means any integer from 1 to 10, i.e. 1, 2, 3, 4, 5, 6, 7, 8, 9 or 10. In other words, any range defined by two integers explicitly mentioned is meant to comprise and disclose any integer defining said limits and any integer comprised in said range.

The term "alkyl" refers to a saturated, straight-chain or branched hydrocarbon group that contains from 1 to 10 carbon atoms, for example a n-octyl group, especially from 1 to 6, i.e. 1, 2, 3, 4, 5, or 6, carbon atoms. Examples of alkyl as used herein include, but are not limited to, are methyl, ethyl, propyl, iso-propyl, n-butyl, iso-butyl, sec-butyl, tert-butyl, n-pentyl, iso-pentyl, n-hexyl, 2,2-dimethylbutyl. The alkyl group may be optionally substituted with substituents, multiple degrees of substitution being allowed. The alkyl group may be interrupted by non-carbon atoms such as N, S or O atoms. In one embodiment, a C₁ - C₁₀ alkyl group may be interrupted by n amido groups, wherein n is 0 - 3. A C₁ - C₁₀ alkyl group interrupted by n amido groups includes (CH₂)₂NHC(O)(CH₂)₃ and (CH₂)₂NHC(O)(CH₂)₃C(O)NHCH₂.

As used herein, the term "alkyl" may also refer to a group comprising a longest linear chain length of 1 - 20 atoms, i.e. 1, 2, 3, 4, 5, 6, 7, 8, 9, 10, 11, 12, 13, 14, 15, 16, 17, 18, 19 or 20 atoms, wherein the linear chain may comprise non-carbon atoms such as N, S or O atoms. In one embodiment, an alkyl group comprising a longest linear chain length of 1 - 20 atoms may comprise n amido groups, wherein n is 0 - 3. An alkyl group comprising a longest linear chain length of 1 - 20 atoms comprising n amido groups includes (CH₂)₂NHC(O)(CH₂)₃ and (CH₂)₂NHC(O)(CH₂)₃C(O)NHCH₂.

The term "aryl" refers to an aromatic group that contains one or more rings containing from 6 to 14 ring carbon atoms, preferably from 6 to 10 (especially 6) ring carbon atoms. Examples are phenyl, naphthyl and biphenyl groups. Examples of substituted aryl groups suitable for use in the present invention include p-toluenesulfonyl (Ts), benzenesulfonyl (Bs) and m-nitrobenzenesulfonyl (Ns).

X is a linker group that is covalently attached to the DFOSq molecule at one attachment site and to Y¹ at a second attachment site. Y¹ may be any suitable functional group for conjugation of the PSMA targeting agent to the DFOSq molecule via linker group X.

Preferred compounds of the present invention are those where X is selected from the group consisting of:
aryl optionally substituted with C₁-C₁₀ alkyl, ethylenediaminetetraacetic acid (EDTA); and
(C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3 ; and
Y¹ and Y² are independently selected from the group consisting of: carboxylic acid, ester, anhydride, and amine.

In one embodiment, the compound of formula (I) is monomeric. A monomeric form of the compound of formula (I) includes one Y functional group (Y¹), and is capable of conjugating one PSMA targeting agent. In a preferred form of this embodiment, X comprises an aromatic group. More preferably X is selected from the group consisting of: phenyl, benzyl, and EDTA functionalised phenyl. Preferably Y¹ is a carboxyl group.

In another embodiment, the compound of formula (I) is dimeric. A dimeric form of the compound of formula (I) includes two Y functional groups (Y¹ and Y²), and is capable of conjugating two PSMA targeting agents. In a preferred form of this embodiment, X is (C₁-C₁₀ alkyl)NR⁷(Y²)R⁸, preferably (CH₂)₂NR⁷(Y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3. Preferably, R⁷ and R⁸ are independently selected from (CH₂)₂, (CH₂)₂NHC(O)(CH₂)₃ and (CH₂)₂NHC(O)(CH₂)₃C(O)NHCH₂-phenyl. R⁷ and R⁸ may be identical or different, preferably identical. In a preferred aspect of this embodiment, Y¹ and Y² are amine or carboxylic acid. Preferably Y¹ and Y² are identical.

As used herein, the term "radionuclide-labelled conjugate" refers to:
- a compound of formula (I), as defined herein, conjugated to a PSMA targeting agent which has formed a co-ordination complex with a radionuclide; or
- a conjugate of formula (II) which has formed a co-ordination complex with a radionuclide.

Generally, this occurs as a result of the formation of co-ordination bonds between the electron donating groups (such as the hydroxamate groups) of the compound or conjugate of formula (I) or (II) and the radionuclide.

In the compounds of the present invention, co-ordination bonds are postulated to form between the hydroxamic acid groups of the DFO, or DFO*, and the radionuclide. However, without wishing to be bound by theory, the present inventors also believe that the oxo groups on the squarate moiety (in addition to the hydroxamic acid groups of DFO or DFO*) also act as donor atoms, providing one or two additional sites by which the compound of formula (I) can bind to the radionuclide. This results in an eight-coordinate complex, which is very favourable from a stability perspective for radionuclides that have eight-coordinate geometry (such as ⁸⁹Zr), and may explain the stability observed in respect of the complexes of the present invention. In particular, it may explain why the radionuclide does not as readily leach out of the target tissue (into other tissue, such as bone) therefore resulting in improved imaging quality when compared to other DFO, or DFO*,-based imaging agents. These advantages of the compounds of the present invention over the currently-used chelators are illustrated in the Figures and Examples.

The squarate moiety, for example the squaramide, may also play a role in assisting the targeting of the targeting agent. Further, the squarate moiety also provides superior solubility when compared to isothiocyanato DFO derivatives.

As used herein, the term "radionuclide" (also commonly referred to as a radioisotope or radioactive isotope), is an atom with an unstable nucleus. It radioactively decays resulting in the emission of nuclear radiation (such as gamma rays and/or subatomic particles such as alpha or beta particles). In one embodiment, the radionuclide is one that is also useful in radioimmunotherapy applications (e.g. a beta particle emitter). Preferably, the radionuclide has eight-coordinate geometry. Examples of radionuclides suitable for use in the present invention include radioisotopes of zirconium (e.g. ⁸⁹Zr), gallium (e.g. ⁶⁷Ga and ⁶⁸Ga), lutetium (e.g. ¹⁷⁶Lu and ¹⁷⁷Lu), holmium (e.g. ¹⁶⁶Ho), scandium (e.g. ⁴³Sc, ⁴⁴Sc and ⁴⁷Sc), titanium (e.g. ⁴⁵Ti), manganese (e.g. ⁵²Mn), indium (e.g. ¹¹¹In and ¹¹⁵In), yttrium (e.g. ⁸⁶Y and ⁹⁰Y), terbium e.g. (¹⁴⁹Tb, ¹⁵²Tb, ¹⁵⁵Tb and ¹⁶¹Tb), technetium (e.g. ^{99m}Tc), samarium (e.g. ¹⁵³Sm) and niobium (e.g. ⁹⁵Nb and ⁹⁰Nb). The radionuclide for use in the present invention may be selected from gallium (specifically, ⁶⁷Ga and ⁶⁸Ga), indium (specifically, ¹¹¹In), zirconium (specifically, ⁸⁹Zr) and aluminium fluoride (specifically, Al¹⁸F where ¹⁸F is the radioisotope and Al is the carrier. The radionuclide for use in the present invention may be selected from ⁶⁸Ga, ¹¹¹In and ⁸⁹Zr. For example, ⁶⁸Ga has been shown to bind with DFO (see Ueda et al (2015) Mol Imaging Biol, vol. 17, pages 102-110), and indium has similar co-ordination chemistry to zirconium (and therefore would be expected to bind to the compound of formula (I) or (II) in a similar way).

It will be understood by a person skilled in the art that the compounds or conjugates of the present invention can also complex non-radioactive metals used in imaging applications, such as MRI. An example of such a metal is gadolinium (e.g. ¹⁵²Gd).

As mentioned above, the present invention also relates to a conjugate of a compound of formula (I), or a pharmaceutically-acceptable salt thereof, and a PSMA targeting agent.

As used herein, the term "PSMA targeting agent" refers to a moiety that has the ability to target prostate specific membrane antigen. Preferably the PSMA targeting agent is a peptide or is a urea-linked dipeptide, more preferably Lys-Urea-Glu. The targeting agent will have a functional group (such as an amine group of a lysine residue) that will react with the functional group Y (Y¹ and/or Y²) to form a covalent link between the targeting agent and the compound of formula (I). This results in formation of the conjugate. The conjugate may also include a radionuclide complexed thereto. This produces a radionuclide-labelled conjugate of a compound of formula (I), or a pharmaceutically-acceptable salt thereof, a targeting agent, and a radionuclide complexed thereto. In one embodiment, the radionuclide is a radioisotope of zirconium (e.g. ⁸⁹Zr).

The compounds of formula (I), conjugates, and radionuclide complexes can be synthesised by any suitable method known to a person skilled in the art. It will be clear to a person skilled in the art that conjugates according to the invention may be prepared by reacting compounds of formula (I) with a PSMA targeting agent. It will also be clear to a person skilled in the art that conjugates according to the invention may be prepared by functionalising the PSMA targeting agent with linker group X (or a portion thereof) via functional group Y (Y¹ and/or Y²) and reacting the functionalised PSMA targeting agent with DFOSq.

An example of a synthetic method for monomeric conjugates of compounds of formula (I) is given below in Scheme 1.

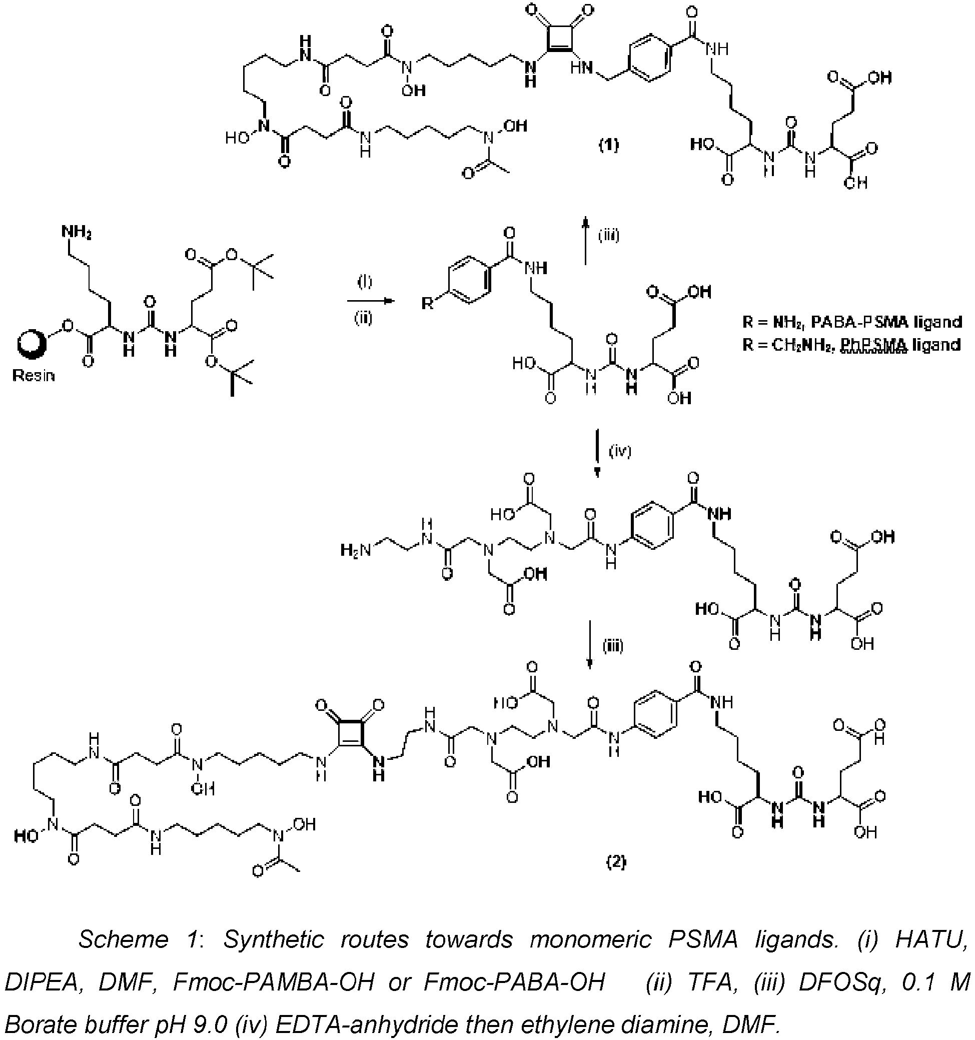

An example of a synthetic method for dimeric conjugates of compounds of formula (I) is given below in Scheme 2.

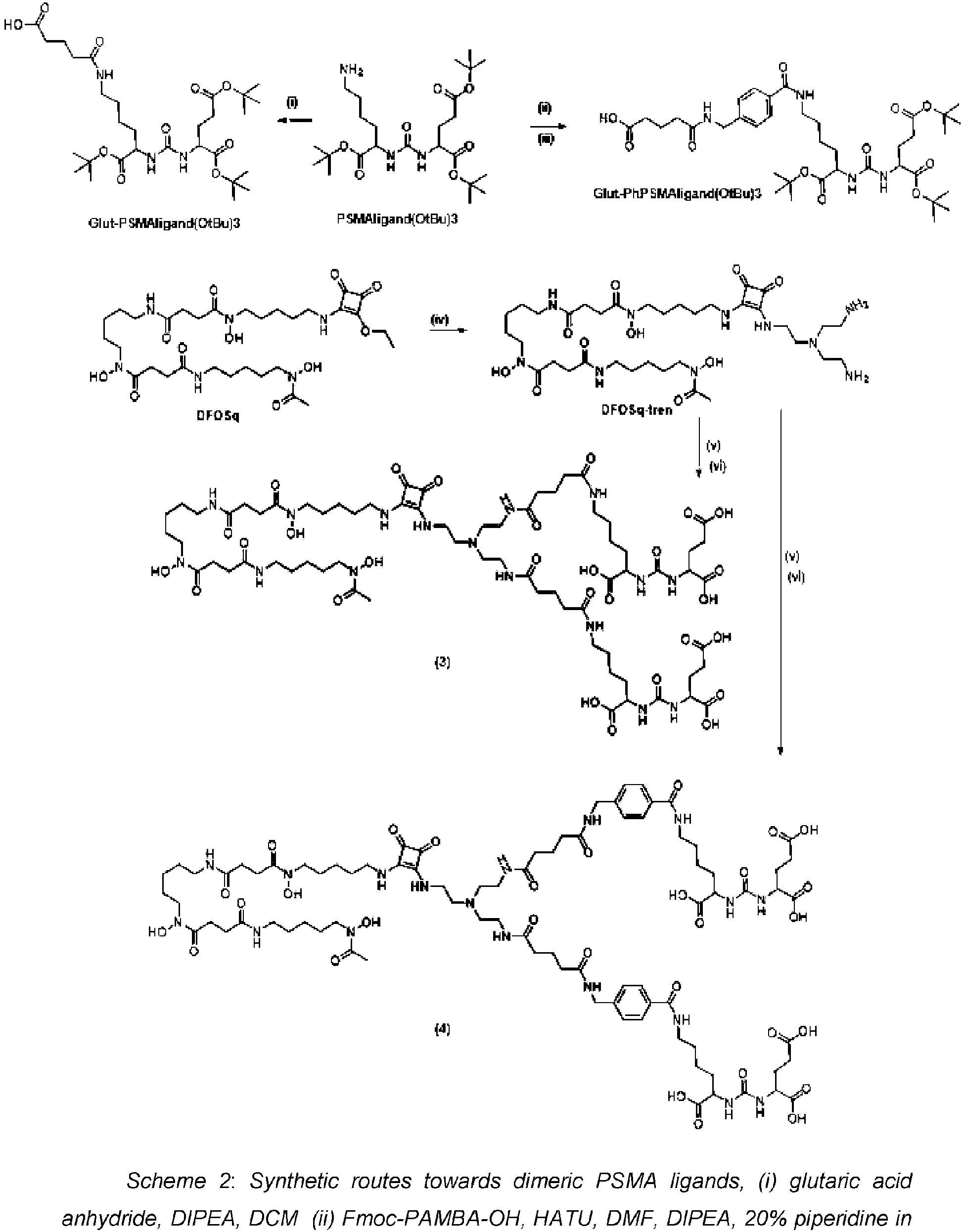

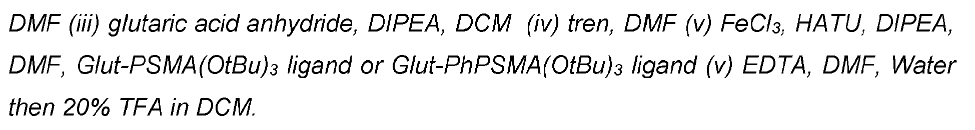

It will also be clear to a person skilled in the art that the conjugates of formula (I) or (II) can be prepared in the absence of the radionuclide. In this embodiment, the radionuclide is added to the conjugate once the conjugate has been prepared. Alternatively, conjugates of formula (I) or (II) without a radionuclide may be useful in targeted iron chelation treatment of cancer by depriving cancer cells of (Fe), an essential nutrient.

The present invention also relates to pharmaceutical compositions including a radionuclide-labelled conjugate of:
- a compound of formula (I): 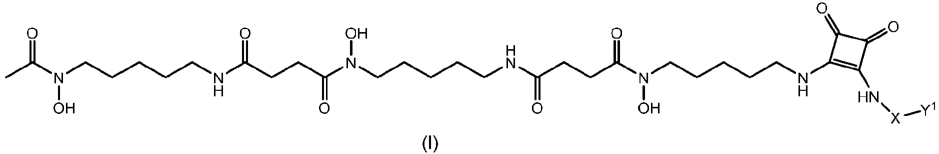 or a pharmaceutically-acceptable salt thereof, wherein X is selected from the group consisting of:
   aryl optionally substituted with C₁-C₁₀ alkyl, ethylenediaminetetraacetic acid (EDTA); and
   (C₁-C₁₀ alkyl)NR⁷(y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
   Y¹ and Y² are independently selected from the group consisting of: carboxylic acid, ester, anhydride, and amine; and
   wherein the pharmaceutically acceptable derivative of formula (I) is: 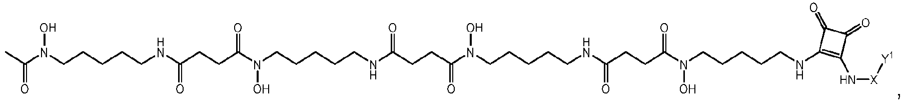
- a PSMA targeting agent, and
- a radionuclide complexed thereto,

wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).
and one or more pharmaceutically acceptable carrier substances, excipients and/or adjuvants.

The present invention also relates to pharmaceutical compositions including a radionuclide-labelled conjugate of:
- a conjugate of formula (II): 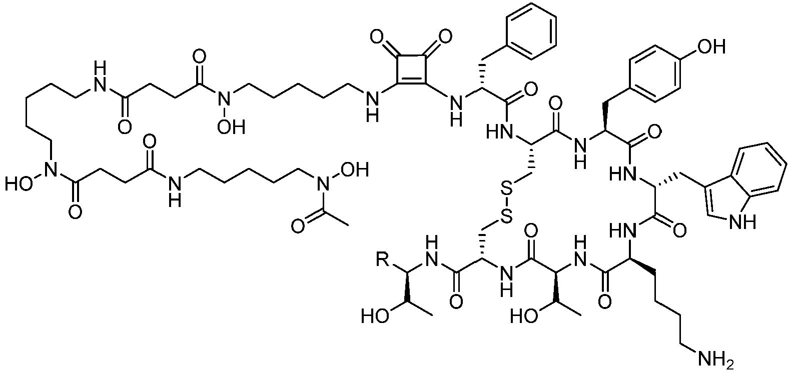 or a pharmaceutically-acceptable salt thereof, wherein R is as defined herein, and
- a radionuclide complexed thereto,

wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).
and one or more pharmaceutically acceptable carrier substances, excipients and/or adjuvants.

Pharmaceutical compositions may include, for example, one or more of water, buffers (for example, neutral buffered saline, phosphate buffered saline, citrates and acetates), ethanol, oil, carbohydrates (for example, glucose, fructose, mannose, sucrose and mannitol), proteins, polypeptides or amino acids such as glycine, antioxidants (e.g. sodium bisulfite), tonicity adjusting agents (such as potassium and calcium chloride), chelating agents such as EDTA or glutathione, vitamins and/or preservatives.

Pharmaceutical compositions will preferably be formulated for parenteral administration. The term "parenteral" as used herein includes subcutaneous, intradermal, intravascular (for example, intravenous), intramuscular, spinal, intracranial, intrathecal, intraocular, periocular, intraorbital, intrasynovial and intraperitoneal injection, as well as any similar injection or infusion technique. Intravenous administration is preferred. Suitable components of parenteral formulations, and methods of making such formulations, are detailed in various texts, including "Remington's Pharmaceutical Sciences".

The composition of the present invention will be administered to a patient parenterally in the usual manner. The DFO-squaramide conjugate complex may then take anywhere from 1 hour to 24 hours to distribute throughout the body to the target site. Once the desired distribution has been achieved, the patient will be imaged.

Accordingly, the present invention also relates to a radionuclide-labelled conjugate, as defined herein, for use in a method of imaging a patient, the method including:
- administering to a patient the radionuclide-labelled conjugate, as defined herein; and
- imaging said patient.

The present invention also relates to a radionuclide-labelled conjugate, as defined herein, for use in a method of imaging a cell or *in vitro* biopsy sample, the method including:
- administering to a cell or *in vitro* biopsy sample the radionuclide-labelled conjugate, as defined herein; and
- imaging the cell or *in vitro* biopsy sample.

For conjugates of formula (I), the PSMA targeting agent serves to target the conjugate to a desired site *in vivo,* or to a desired site in the cell or in the biopsy sample, particularly a prostate tumour.

For conjugates of formula (II), the sstr2 targeting agent serves to target the conjugate to a desired site *in vivo,* or to a desired site in the cell or in the biopsy sample, particularly a neuroendocrine tumour.

In another aspect, the present invention relates to a radionuclide-labelled conjugate, as defined herein, for use in the treatment of cancer in a patient.

It will be understood, that the specific dose level for any particular patient, and the length of time that the agent will take to arrive at the target site, will depend upon a variety of factors including the activity of the specific compound employed, the age, body weight, general health, sex, diet, time of administration, route of administration, and rate of excretion, and the severity of the particular disorder undergoing therapy.

The term "effective amount" refers to an amount that results in a detectable amount of radiation following administration of the radionuclide-labelled conjugate to a patient. A person skilled in the art will know how much of the radionuclide-labelled conjugate to administer to a patient to achieve the optimal imaging capability without causing problems from a toxicity perspective. The radionuclide-labelled conjugates of the present invention find particular use in assisting clinicians to determine where a cancer is (including whether a target, such a receptor, is homogeneously present on a tumour), what treatment a cancer will respond to (which facilitates treatment selection and determination of optimal dosages), and how much of the treatment will ultimately reach the target site.

Patients may include but are not limited to primates, especially humans, domesticated companion animals such as dogs, cats, horses, and livestock such as cattle, pigs and sheep, with dosages as described herein.

As mentioned above, the radionuclide-labelled conjugates of the present invention are particularly useful for imaging and/or treating tumours (which form as a result of uncontrolled or progressive proliferation of cells). Some such uncontrolled proliferating cells are benign, but others are termed "malignant" and may lead to death of the organism. Malignant neoplasms or "cancers" are distinguished from benign growths in that, in addition to exhibiting aggressive cellular proliferation, they may invade surrounding tissues and metastasize. Moreover, malignant neoplasms are characterized in that they show a greater loss of differentiation (greater "dedifferentiation"), and greater loss of their organization relative to one another and their surrounding tissues. This property is also called "anaplasia". Neoplasms treatable by the present invention also include solid phase tumors/malignancies, i.e. carcinomas, locally advanced tumors and human soft tissue sarcomas. Carcinomas include those malignant neoplasms derived from epithelial cells that infiltrate (invade) the surrounding tissues and give rise to metastastic cancers, including lymphatic metastases.

Adenocarcinomas are carcinomas derived from glandular tissue, or which form recognizable glandular structures. Another broad category of cancers includes sarcomas, which are tumors whose cells are embedded in a fibrillar or homogeneous substance like embryonic connective tissue.

The type of cancer or tumor cells that may be amenable to imaging according to the invention include prostate cancers and neuroendocrine cancers.

It may also be advantageous to administer the radionuclide-labelled conjugates of the present invention with drugs that have anti-cancer activity. Examples of suitable drugs in this regard include fluorouracil, imiquimod, anastrozole, axitinib, belinostat, bexarotene, bicalutamide, bortezomib, busulfan, cabazitaxel, capecitabine, carmustine, cisplatin, dabrafenib, daunorubicin hydrochloride, docetaxel, doxorubicin, eloxati, erlotinib, etoposide, exemestane, fulvestrant, methotrexate, gefitinib, gemcitabine, ifosfamide, irinotecan, ixabepilone, lanalidomide, letrozole, lomustine, megestrol acetate, temozolomide, vinorelbine, nilotinib, tamoxifen, oxaliplatin, paclitaxel, raloxifene, pemetrexed, sorafenib, thalidomide, topotecan, vermurafenib and vincristine.

The radionuclide-labelled conjugates of the present invention can also be used to determine whether a particular tumour has one or more types of receptor, and therefore whether a patient may benefit from a particular therapy. For example, by using lys-urea-glu as a targeting molecule in the radiolabelled conjugate of formula (I), the presence of PSMA on a patient's tumour can be tested for. If the tumour is PSMA-negative (i.e. does not have PSMA cell surface receptor), the imaging agent will not "stick" to the tumour. Alternatively, by using sstr2 as a targeting molecule in the radiolabelled conjugate of formula 9II), the presence of sstr2 on a patient's tumour can be tested for. If the tumour is sstr2-negative (i.e. does not have sstr2 cell surface receptor), the imaging agent will not "stick" to the tumour.

It will be understood that the invention disclosed and defined in this specification extends to all alternative combinations of two or more of the individual features mentioned or evident from the text or drawings. All of these different combinations constitute various alternative aspects of the invention.

In another aspect there is provided a kit or article of manufacture comprising a compound, conjugate or radionuclide conjugate as described herein, a pharmaceutically acceptable salt, diluent or excipient and/or pharmaceutical composition as described above. Further, the kit may comprise instructions for use in any method or use of the invention as described herein.

In other embodiments there is provided a kit for use, or when used, in a therapeutic and/or diagnostic application mentioned above, the kit comprising:
- a container holding a therapeutic or diagnostic composition in the form of an compound, conjugate or radionuclide conjugate as described herein, or pharmaceutical composition;
- a label or package insert with instructions for use in any method or use of the invention as described herein..

In certain embodiments the kit may contain one or more further active principles or ingredients for treatment or diagnosis of cancer.

The kit or "article of manufacture" may comprise a container and a label or package insert on or associated with the container. Suitable containers include, for example, bottles, vials, syringes, blister pack, etc. The containers may be formed from a variety of materials such as glass or plastic. The container holds a therapeutic or diagnostic composition which is effective for treating or imaging the condition and may have a sterile access port (for example the container may be an intravenous solution bag or a vial having a stopper pierceable by a hypodermic injection needle). The label or package insert indicates that the therapeutic composition is used for treating or imaging the condition of choice. In one embodiment, the label or package insert includes instructions for use and indicates that the therapeutic or diagnostic composition can be used to treat a cancer described herein.

### Examples

All reagents and solvents were obtained from standard commercial sources and unless otherwise stated were used as received.

### Example 1 - Radioimaging agents targeting PSMA

Small molecules based on urea-linked dipeptides (Lysine-Urea-Glu) bind with selectivity to prostate-specific membrane antigen (PSMA). The aim of this work is to develop new ⁸⁹Zr-DFOSq conjugated PSMA radiotracers. A family of DFO-sq conjugated mono and dimeric PSMA targeting Lysine-Urea-Glu fragments have been synthesized and tested *in vivo* using ⁸⁹Zr and ⁶⁸Ga PET radionuclides.

***General Experimental and Materials.*** Unless otherwise stated, all reagents were purchased from commercial sources and used without further purification. DFOSq was prepared according to reported procedure (Rudd et al. Chemical Communications 2016, 52 (80), 11889-11892). Flash column chromatography was carried out using silica gel (40-63 microns) as the stationary phase. Analytical TLC was performed on pre-coated silica gel plates (0.25 mm thick, 60F254, Merck, Germany) and visualized under UV light. ESI-MS spectra were recorded on Thermo Fisher OrbiTRAP infusion mass spectrometer. HPLC purification and analysis of non-radioactive samples were performed on a Agilent 1100 series using solvent A = 0.1 % TFA in Milli and solvent B = 0.1% TFA in CH₃CN. For analytical HPLC Phenomenex Luna^{®} 5 µm C18(2) 100 Å, LC Column 150 x 4.6 mm at a flow rate of 1 mL/min was used while Phenomenex Luna^{®} 5 µm C18(2) 100 Å, LC Column 250 x 21 mm at flow rate of 5-8 mL/min was used for preparative HPLC. Radio-HPLC was performed on a Shimadzu SCL-10A VP/LC-10 AT VP system with a Shimadzu SPD-10A VP UV detector followed by a radiation detector (Ortec model 276 photomultiplier base with preamplifier, Ortec 925-SCINT ACE mate preamplifier, BIAS supply and SCA, Bicron 1M 11/2 photomultipliertube). Phenomenex Luna^{®} 5 µm C18(2) 100 Å, LC Column 150 x 4.6 mm using 0.05% TFA buffers in MillQ water and acetonitrile) at a flow rate of 1 mL/min.

***Synthesis of PABA-PSMA ligand.*** To a suspension of resin bound PSMA ligand (lys-urea-glu) (43 mg, 0.1mmol) was added Fmoc-PABA-OH (72 mg, 0.2 mmol), DIEA (70 µL, 0.4mmol) and HBTU (38 mg, 0.1 mmol) was stirred in DMF (5 mL) overnight then the resin was filtered and treated with 20% piperidine in DMF solution for 1 hours then filtered again. The resin was then treated with TFA (1 ml) for 15 min then TFA removed under a stream of nitrogen and ice cold diethyl ether (30 mL) was added to precipitate the product which was collected by centrifugation and purified by HPLC (Phenomenex Luna^{®} 5 µm C18(2) 100 Å, LC Column 250 x 21 mm using 0.1% TFA buffers in MillQ water and acetonitrile) to give PABA-PSMA ligand as white solid (18 mg, 41% yield). ESI-MS: (+ve ion) [M + H⁺] *m*/*z* = 439.1825 (experimental), calculated for [C₁₉H₂₇N₄O₈]⁺: *m*/*z* = 439.1829.

***Synthesis of ED-EDTA-PABA-PSMA ligand.*** PABA-PSMA ligand (6 mg, 0.014 mmol) and EDTA anhydride (3.5 mg, 0.0014) was dissolved in dry DMF (1 mL) in an Eppendorf tube under a stream of nitrogen. The solution was stirred for 5 hours at RT then a large excess of ethylene diamine (50 uL) was added and the mixture was left to stir overnight. The solvent was removed under vacuum and the residue was purified by HPLC (Phenomenex Luna^{®} 5 µm C18(2) 100 Å, LC Column 250 x 21 mm using 0.1% TFA buffers in MillQ water and acetonitrile) to provide ED-EDTA-PABA-PSMA ligand as white solid (2.5 mg, 25% yield). ESI-MS: (+ve ion) [M + H⁺] *m*/*z* = 755.3205 (experimental), calculated for [C₃₁H₄₅N₈O₁₄]⁺: *m*/*z* = 755.3212

*Synthesis of PhPSMA ligand.* To a suspension of resin bound PSMA ligand (43 mg, 0.1 mmol) was added Fmoc-PAMBA-OH (75 mg, 0.2 mmol), DIEA (70 µL, 0.4mmol) and HBTU (38 mg, 0.1 mmol) was stirred in DMF (5 mL) overnight then the resin was filtered and treated with 20% piperidine in DMF solution for 1 hours then filtered again. The resin was then treated with TFA 1 ml for 15 min then the TFA removed under a stream of nitrogen and ice cold diethyl ether (30 mL) was added to precipitate the product which was collected by centrifugation and purified by HPLC (Phenomenex Luna^{®} 5 µm C18(2) 100 Å, LC Column 250 x 21 mm using 0.1% TFA buffers in MillQ water and acetonitrile) to give PhPSMA ligand as white solid (12 mg, 26% yield). ESI-MS: (+ve ion) [M + H⁺] *m*/*z* = 453.1982 (experimental), calculated for [C₂₀H₂₉N₄O₈]⁺: *m*/*z* = 453.1985

*Synthesis of DFOSq-EDTA-PSMA ligand (2).* A mixture of DFOSq (20 mg, 0.026 mmol) in DMSO (100 µL) and ED-EDTA-PABA-PSMA ligand (91 mg, 0.132 mmol) in borate buffer (0.1M, pH 9.0, 900uL) was stirrer at room temperature for 3 days then purified by HPLC (Phenomenex Luna^{®} 5 µm C18(2) 100 Å, LC Column 250 x 21 mm using 0.1% TFA buffers in MillQ water and acetonitrile) to provide (2) as white solid (6 mg, 16% yield). ESI-MS: (+ve ion) [M + H⁺] *m*/*z* = 1393.6483 (experimental), calculated for [C₆₀H₉₃N₁₄O₂₄]⁺: m/z = 1393.6487

*Synthesis of DFOSq-PhPSMA ligand (1).* A mixture of DFOSq (5 mg, 0.011 mmol) in DMSO (100 µL) and PAMBA-PSMA ligand (91 mg, 0.132 mmol) in borate buffer (0.1 M, pH 9.0, 900uL) was stirrer at 37 °C for 6 days then purified by HPLC (Phenomenex Luna^{®} 5 µm C18(2) 100 Å, LC Column 250 x 21 mm using 0.1% TFA buffers in MillQ water and acetonitrile) to provide (1) as white solid (2 mg, 17% yield). ESI-MS: (+ve ion) [M + H⁺] *m*/*z* = 1091.5254 (experimental), calculated for [C₄₉H₇₅N₁₀O₁₈]⁺: m/z = 1091.5261

*Synthesis of Glut-PSMA(OtBu)₃ ligand.* PSMA(OtBu)₃ ligand (50 mg, 0.103 mmol) and glutaric acid anhydride (59 mg, 0.51 mmol) was dissolved in DMF (1 mL) then DIPEA (179 µL, 1.02 mmol) was added. The solution was stirred at room temperature for 1 hour. Cold diethyl ether (40 mL) was then added to the reaction mixture and resulting crude product was isolated by centrifugation followed by HPLC purification (Phenomenex Luna^{®} 5 µm C18(2) 100 Å, LC Column 250 x 21 mm using 0.1% TFA buffers in MillQ water and acetonitrile) to provide Glut-PSMA(OtBu)₃ ligand as white solid (30 mg, 48% yield). ESI-MS: (+ve ion) [M + H⁺] *m*/*z* = 602.3657 (experimental), calculated for [C₂₉H₅₂N₃O₁₀]⁺: *m*/*z* = 602.3653

***Synthesis of PhPSMA(OtBu)₃ ligand.*** PSMA(OtBu)₃ ligand (500 mg, 1.03 mmol), EDL.HCl (393 mg, 2.03 mmol), HOBt (314 mg, 2.03 mmol), Fmoc-PAMBA-OH (450 mg, 1.23 mmol) and DIEA (839 µL, 5.13 mmol) was dissolved in DMF (2 mL) and the solution was stirred at room temperature for 24 hour. Water 50 mL was then added to reaction mixture and resulting precipitates was collected by centrifugation. 20% piperidine in DMF (10 mL) was added to the residue and the solution was stirred for 1 hour then solvent was removed and residue was purified by column chromatography (silica gel, 5% MeOH in DCM) to give PhPSMA(OtBu)₃ ligand as white solid (410 mg, 64%). ESI-MS: (+ve ion) [M + H⁺] *m*/*z* = 621.3862 (experimental), calculated for [C₃₂H₅₃N₄O₈]⁺: *m*/*z* = 621.3863

***Synthesis of Glut-PhPSMA(OtBu)₃ ligand. PhPSMA(OtBu)₃*** ligand (330 mg, 0.532 mmol) and glutaric acid anhydride (303 mg, 2.6 mmol) was dissolved in DCM (10 mL) then a few drops of DIPEA was added. The solution was stirred at room temperature for 4 hour. The reaction mixture was extracted with DCM (50 mL) then solvent removed and the residue was HPLC purified (Phenomenex Luna^{®} 5 µm C18(2) 100 Å, LC Column 250 x 21 mm using 0.1% TFA buffers in MillQ water and acetonitrile) to give Glut-PhPSMA(OtBu)₃ ligand as white solid (236 mg, 60%). ESI-MS: (+ve ion) [M + H⁺] *m*/*z* = 735.4181 (experimental), calculated for [C₃₇H₅₉N₄O₁₁]⁺: *m*/*z* = 735.4180

***Synthesis of DFOSq-Tren.*** DFOSq (100 mg, 0.146 mmol) and tris(2-aminoethyl)amine (237 µL, 1.460 mmol) were dissolved in DMF (2 mL) then the solution was stirred overnight at room temperature. Diethyl ether was then added to reaction mixture and resulting crude product was isolated by centrifugation followed by HPLC purification (Phenomenex Luna^{®} 5 µm C18(2) 100 Å, LC Column 250 x 21 mm using 0.1% TFA buffers in MillQ water and acetonitrile) to give DFOSq-tren as white waxy solid (65 mg, 57%). ESI-MS: (+ve ion) [M + H⁺] *m*/*z* = 785.4860 (experimental), calculated for [C₃₅H₆₅N₁₀O₁₀]⁺: *m*/*z* = 785.4885

***Synthesis of DFOSq-bisPSMA ligand (3).*** DFOSq-tren (20 mg, 0.025 mmol) and FeCl₃ (7 mg, 0.025 mmol) was stirred in DMF (150 µL) at RT then added to a solution of HATU (29 mg, 0.075 mmol), DIPEA (40 µL, 0.248 mmol) and Glut-PSMA(OtBu)₃ ligand (30 mg, 0.050 mmol) in DMF (200 µL). The resulting solution was stirred at RT for 1 hour then cold diethyl ether (10 mL) was added and the resulting precipitate was collected by centrifugation. The residue was dissolved in DMF (100 µL) and a saturated solution of EDTA in DMF:water mixture (1:1, 1 mL) was added and the solution was heated at 40 °C until the color changes from red to pale yellow. The reaction mixture was purified by HPLC (Phenomenex Luna^{®} 5 µm C18(2) 100 Å, LC Column 250 x 21 mm using 0.1% TFA buffers in MillQ water and acetonitrile) and the purified fractions were treated with 20% TFA in DCM to remove OtBu groups. The solvent was removed under a stream of nitrogen and the residue was purified by HPLC to give **(3)** as white solid (9 mg, 22%). ESI-MS: (+ve ion) [M + 2H]⁺ *m*/*z* = 808.4080 (experimental), calculated for [C₆₈H₁₁₆N₁₆O₂₈]²⁺: *m*/*z* = 808.4072

***Synthesis of DFOSq-bisPhPSMA ligand (4).*** DFOSq-tren (10.68 mg, 0.014 mmol) and FeCl₃ (3.68 mg, 0.014 mmol) was stirred in DMF (150 µL) at RT then added to a solution of HATU (12.42 mg, 0.033 mmol), DIPEA (11 µL, 0.068 mmol) and Glut-PhPSMA(OtBu)₃ ligand (20 mg, 0.028 mmol) in DMF (200 µL). The resulting solution was stirred at RT for 1 hour then cold diethyl ether (10 mL) was added and the resulting precipitate was collected by centrifugation. The residue was dissolved in DMF (100 µL) and a saturated solution of EDTA in DMF:water mixture (1:1, 1 mL) was added and the solution was heated at 40 °C until the color changes from red to pale yellow. The reaction mixture was purified by HPLC (Phenomenex Luna^{®} 5 µm C18(2) 100 Å, LC Column 250 x 21 mm using 0.1% TFA buffers in MillQ water and acetonitrile) and the purified fractions were treated with 20% TFA in DCM to remove OtBu groups. The solvent was removed under a stream of nitrogen and the residue was purified by HPLC to give **(4)** as white solid (7 mg, 27%). ESI-MS: (+ve ion) [M + 2H⁺] *m*/*z* = 941.4580 (experimental), calculated for [C₈₅H₁₂₉N₁₈O₃₀]²⁺: *m*/*z* = 941.4594

**⁸⁹Zr *Radiolabelling of* (1).** ⁸⁹Zr in 1 M oxalic acid (50 µL, 66 MBq, Perkin Elmer NEZ308000MC) was diluted with MilliQ water (50 µL) then was neutralized (pH 6-7) with a series of small volume additions of aqueous Na₂CO₃ (1 M, 5 x 5 µL). HEPES buffer (41 µL,1 M, pH 7.0) was then added and the solution allowed to stand for 5 min before pH was tested again. After confirming the mixture had a pH 6-7, 125 µL (50 MBq) of ⁸⁹Zr was added to **(1)** (100 µg in 100 µL, 1.8 nmoles/MBq) and the reaction mixture was left to stand at room temperature for 30 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). The crude tracer was purified by Phenomenex StrataX cartridges (C18, 60 mg) using ethanol as eluent and fractions containing most labelled product (22 MBq) was combined and diluted to 8% ethanol in PBS to final volume of 1.2mL. Six syringes (BD Ultra-Fine^{™}, 0.3 mL) containing approximately 3.0 MBq (approx. peptide mass 6 µg, 5.4 nmoles) in 170 µL were prepared.

**⁸⁹Zr *Radiolabelling of (2).*** ⁸⁹Zr in 1 M oxalic acid (100 µL, 72 MBq , Perkin Elmer NEZ308000MC) was diluted with MilliQ water (100 µL) then was neutralized (pH 6-7) with a series of small volume additions of aqueous Na₂CO₃ (1 M, 80 µL). HEPES buffer (93 µL,1 M, pH 7.0) was then added and the solution allowed to stand for 5 min before pH was tested again. After confirming the mixture had a pH 6-7, **(2)** (500 µg in 500 µL in 0.25M HEPES) was added into the ⁸⁹Zr solution and the reaction mixture was left to stand at room temperature for 60 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). It showed ≥70% radiochemical yield. The crude tracer was purified by Phenomenex StrataX cartridges (C18, 60 mg) using ethanol as eluent and fractions containing most labelled product (20 MBq) was combined and diluted to 8% ethanol in PBS to final volume of 1.2mL. For imaging and biodistribution six syringes (0.3 mL BD Ultra-Fine^{™}) containing approximately 2.6 MBq (approx. 28 µg) each were prepared for injection.

**⁸⁹Zr *Radiolabelling* of (3).** ⁸⁹Zr in 1 M oxalic acid (70 µL, 60 MBq, Perkin Elmer NEZ308000MC) was diluted with MilliQ water (70 µL) then was neutralized (pH 6-7) with a series of small volume additions of aqueous Na₂CO₃ (1 M, 4 x 10 µL). HEPES buffer (59 µL,1 M, pH 7.0) was then added and the solution allowed to stand for 5 min before pH was tested again. After confirming the mixture had a pH 6-7, 190 µL (50 MBq) of ⁸⁹Zr was added to **(3)** (150 µg in 75 µL, 1.8 nmoles/MBq) and the reaction mixture was left to stand at room temperature for 30 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). The crude tracer was purified by Phenomenex StrataX cartridges (C18, 60 mg) using 8% ethanol as eluent and several fractions (≈250 µL) were collected and combined to obtain 17 MBq in ≈1.5 mL of labelled tracer was obtained. Six syringes (BD Ultra-Fine^{™}, 0.3 mL) containing approximately 2.6 MBq (approx. peptide mass 7.8 µg, 4.8 nmol) in 220 µL were prepared.

**⁸⁹Zr *Radiolabelling of (4).*** ⁸⁹Zr in 1 M oxalic acid (70 µL, 62 MBq , Perkin Elmer NEZ308000MC) was diluted with MilliQ water (70 µL) then was neutralized (pH 6-7) with a series of small volume additions of aqueous Na₂CO₃ (1 M, 3 x 10 µL). HEPES buffer (59 µL,1 M, pH 7.0) was then added and the solution allowed to stand for 5 min before pH was tested again. After confirming the mixture had a pH 6-7, **(4)** (220 µg in 22 µL, 1:1 DMSO:MilliQ, 1.8 nmoles/MBq) was added into the ⁸⁹Zr solution (220 µL) and the reaction mixture was left to stand at room temperature for 70 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). It showed ≥70% radiochemical yield. The crude tracer was adsorbed on Phenomenex StrataX (C18, 60 mg) cartridge then 8% ethanol in PBS was used as eluent. Several fractions (≈250 µL) were collected and combined to obtain 17.1 MBq in ≈1.1 mL of labelled tracer with ≥95% radiochemical purity. For imaging and biodistribution six syringes (0.3 mL BD Ultra-Fine^{™}) containing approximately 1.8 MBq (approx. 6.4 µg, 3.3 nmol) each were prepared for injection.

**⁶⁸Ga *Radiolabelling* of (1).** ⁶⁸Ga in HCl (450 µL, 42 MBq) was buffered with sodium acetate (1 M, 50 µL, pH 4.5) then **(1)** (2.9 µg in 2.9 µL, 2.6 nmoles) and the reaction mixture was left to stand at room temperature for 10 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). 10xPBS buffer (55 µL, pH 7.4) was added to the mixture then further diluted with 1xPBS (550 µL, pH 7.4) to give a final peptide concentration of 2.4 µM and six syringes (BD Ultra-Fine^{™}, 0.3 mL) containing approximately 3.5 MBq in 150 µL (approx. peptide mass 0.4 µg, 0.36 nmoles) were prepared.

**⁶⁸Ga *Radiolabelling* of (4).** ⁶⁸Ga in HCl (900 µL, 42 MBq) was buffered with sodium acetate (1 M, 100 µL, pH 4.5) then **(4)** (5 µg in 5 µL, 2.6 nmoles) and the reaction mixture was left to stand at room temperature for 10 min then an aliquot was analysed by radio-HPLC (0-100% of buffer B to A at 1 mL/min over 15 min, A = 0.05% TFA in MilliQ and B = in 0.05% TFA in Acetonitrile, Luna C18 column 4.6 × 150 mm, 5 µm). 10xPBS buffer (110 µL, pH 7.4) was added to the mixture to give a final peptide concentration of 2.4 µM and six syringes (BD Ultra-Fine^{™}, 0.3 mL) containing approximately 4.0 MBq in 150 µL (approx. peptide mass 0.68 µg, 0.36 nmoles) were prepared.

***PET-CT imaging and Biodistribution.*** Male Balb/c nude mice (age 8.5 weeks) or Male NSG mice (age 12 weeks) were inoculated subcutaneously on the right flank with 6 x 10⁶ LNCap cells in PBS:Matrigel (1:1). Mice were weighed and tumours measured twice weekly using electronic callipers. Tumour volume (mm³) was calculated as length x width x height x π/6. Mice were assigned for imaging and biodistribution studies (tumour volumes: 85-450 mm₃). ⁸⁹Zr-DFOSq-PSMA tracers were then administered intravenously by tail vein injection to 6 mice. At 1, 2, 4 and 18 hr post injection three mice were anaesthetised using isoflurane and placed on the imaging bed of a G8 PET/CT scanner (Perkin Elmer). A CT scan was performed and followed immediately by a 10 min static PET scan. PET images were reconstructed using the maximal likelihood and expectation maximization (ML-EM) algorithm. PET Images were analysed using VivoQuant (Invicro) and Tumour SUVmax or Tumour SUVmax/background average (TBR) determined. After imaging at 18hr mice were euthanised and selected tissues were excised, weighed and counted using a Capintec (Captus 4000e) gamma counter. A separate cohort of 3 mice was harvested at 1 hr post injection for biodistribution analysis as above. The data was analysed using GraphPad Prism 7 and differences between tracers analysed using a t-test.

***Cell binding and internalization studies.*** LNCap cells (150,000/well) were plated in RPMI 1640 medium containing 10 % FBS into wells of a 24 well plate pre-coated with 0.05% poly-L-lysine and incubated overnight at 37°C and 5% CO₂. The following day cells were washed twice with PBS and incubated for 1 hour at 37°C in 1 mL per well internalisation buffer (MEM, 1% FBS). The buffer was then replaced with 1 ml per well of a warmed solution containing 0.5 µCi ⁸⁹Zr-DFOsq-PSMA tracers in internalisation buffer. Cells were incubated in triplicate for 5, 15, 30 or 60 min at 37°C and 5% CO₂. At each time point, cells were washed twice with ice-cold PBS and the washes collected for counting (unbound fraction). Cells were then incubated twice for 10 min in saline containing 10 µM PMPA and each wash collected for counting (surface bound fraction). The cells were then solubilised in 1 M NaOH (internalised fraction). Total protein content was determined in this fraction using a BCA protein assay kit (Pierce). A Perkin Elmer 2480 Wizard automatic gamma counter was then used to measure Zr-89 radioactivity in unbound, surface bound and internalised fractions. Surface bound and internalised fractions were expressed as percentage of total added radioactivity per mg of protein. Non-specific membrane binding and internalisation was determined by incubating cells with the ⁸⁹Zr-DFOSq-PSMA tracers in the presence of excess (10 µM) PMPA. ⁸⁹Zr-DFOSq-PSMA tracers uptake at 60 min was also determined in DU145 cells (seeded at 125,000 cells/well), a PSMA negative cell line. The raw data was analysed using GraphPad Prism 7.

### Results

### Synthesis and radiolabelling of PSMA tracers

For the synthesis of monomeric PSMA ligands, the lys-urea-glu urea-linked dipeptide precursor was prepared on resin using solid phase chemistry described in reported literature (Eder et al. Bioconjugate Chemistry 2012, 23 (4), 688-697). The resin bound PSMA precursor was reacted with Fmoc- protected p-aminobenzoic acid (PABA) or p-aminomethylbenzoic acid (PAMBA) to generate resin bound intermediates PABA-PSMA ligand and PhPSMA ligand after cleavage form resin. The PhPSMA ligand was conjugated to DFOSq in 0.1 M borate buffer at pH 9.0 over a period of 6-7 days at room temperature providing the **(1)** ligand in 26% yield. For the synthesis of a EDTA bridged ligand the PABA-PSMA ligand precursor was first reacted with EDTA-anhydride followed by ethylene diamine to provide ED-EDTA-PABA-PSMA ligand building block. The DFOSq conjugation was achieved in 16% yield using the similar method described for **(1)** (Scheme 1).

For dimeric ligands, DFOSq was modified by reacting it with tris(2-aminoethyl)amine to provide DFOSq-tren containing two free amine groups. Two acid functionalized PSMA molecules were prepared as separate building blocks containing different linker molecules. The starting OtBu protected PSMA molecule was prepared according to reported procedures and then reacted with glutaric acid anhydride to produce Glut-PSMA(OtBu)₃ ligand. The intermediate with aromatic linker was prepared in the same way but first by reacting PSMA(OtBu)₃ ligand with 4-aminomethyl benzoic acid followed by glutaric acid anhydride. The DFOSq-tren was coupled with Glut-PSMA(OtBu)₃ ligand or Glut-PhPSMA(OtBu)₃ ligand using a typical HATU/DIEA coupling, however the protection of hydroxymates groups on DFOSq motif were required to achieve the coupling in good yields. Both Fe and OtBu groups were removed in two subsequent steps after coupling using EDTA and 10% TFA in DCM respectively to provide the dimeric PSMA ligands in 22-27% yield.

The radiolabelling of PSMA ligands with ⁸⁹Zr was achieved under mild reaction conditions. ⁸⁹Zr was obtained in 1 M oxalic acid solution which was neutralized with 1 M Na₂CO₃ and buffered with HEPES to a final concentration of 0.25 M. The required peptide mass to achieve maximum radiochemical yield in a reaction time of 30-45 min was optimized using **(1)** ligand. The ligand was labelled from 0.1 µg/MBq to 2 µg/MBq of ⁸⁹Zr and the analytical radio-HPLC shows minimum peptide mass required for radiochemical yield of >95% in 30 min reaction time was 2 µg/MBq (1.8 nmoles/MBq per mice). Both the dimeric ligand were radiolabelled using same equivalent number of moles of each peptide per MBq of neutralized ⁸⁹Zr to achieve similar radiochemical yield other than **(2)** which required fivefold excess peptide amount as compared to other tracers to achieve radiotracer with radiochemical purity >98% as lower peptide mass resulted in multiple radiolabelled products. The radiolabelling with ⁶⁸Ga was achieved at much lower peptide concentrations (approx. 5 µg/mL of ⁶⁸Ga eluate, 0.36 nmoles/MBq per mice) within 10 min of reaction time at room temperature providing tracers in high radiochemical yield and purity requiring no purification of the crude tracer.

### PET-CT imaging and Biodistribution

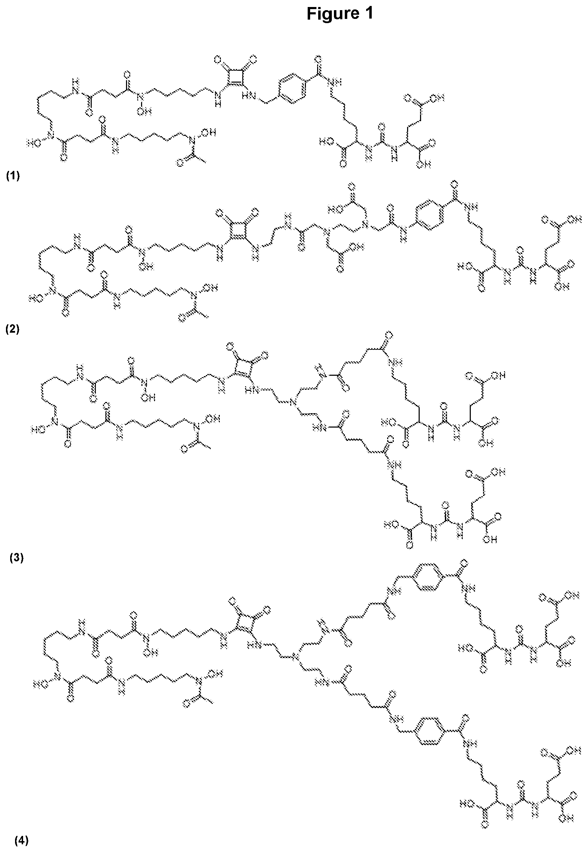

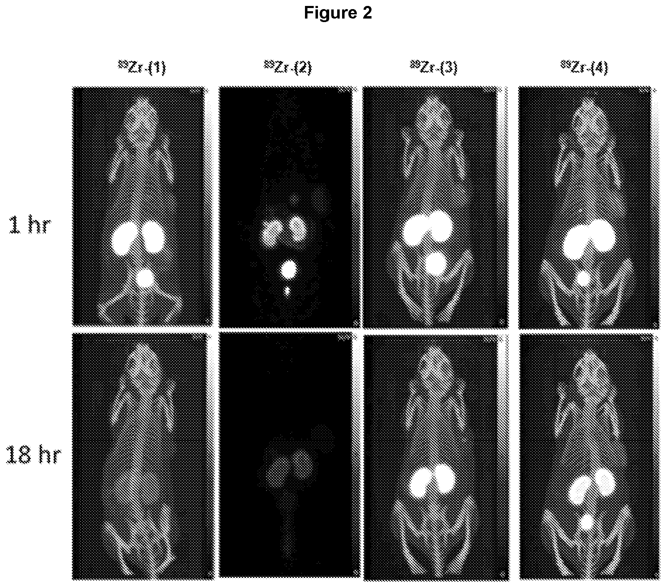

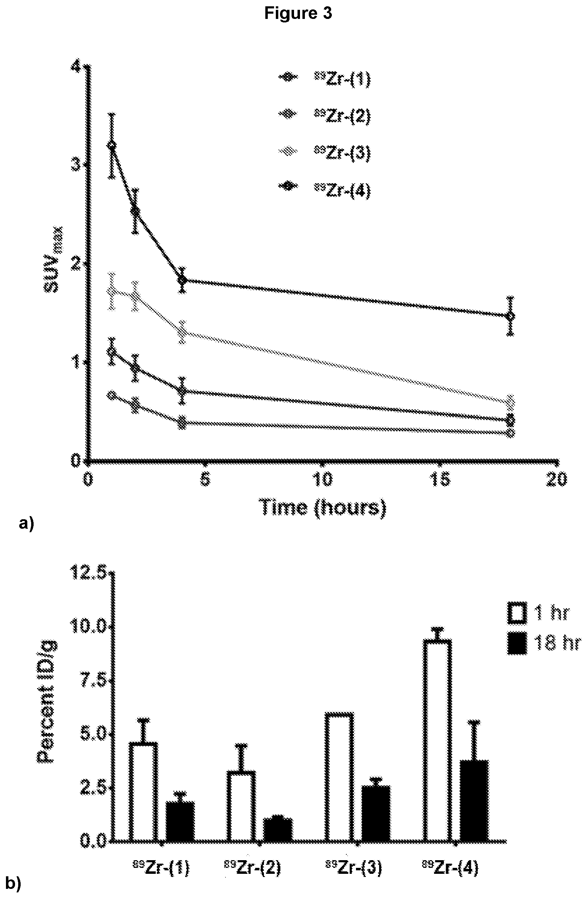

*⁸⁹Zr Labelled tracers:* The ⁸⁹Zr radiolabelled PSMA tracers were injected intravenously via tail vein to LNCaP (human PSMA expressing prostate cancer cell line) tumour bearing mice. At 1, 2, 4 and 18 h post-injection the mice were anaesthetised with isoflurane and imaged over 10 min. The representative PET images of mice injected with ⁸⁹Zr radiolabelled PSMA tracers at 1 and 18 hr are shown in Figure 1 and quantitation of the Tumour SUVₘₐₓ for each tracer is shown in Figure 2a. The PET images for all tracers shows intense uptake seen in the kidneys with lower uptake in tumour for monomeric tracers while both dimeric tracers shows higher uptake. There was a trend for higher tumour SUVₘₐₓ for **(4)** tracer compared to all other tracers at all time points. Similarly, the biodistribution results showed a trend to higher tumour %ID/g for **(4)** compared to others. A comparison of tumour uptake values is shown in Figure 2b and the tissue biodistribution data for the tracers at 1 and 18 hr is summarised in Figure 3. All tracers were significantly cleared from the tumour at 18 hr **((1)** 5.4 to 2.2 %ID/g, **(2)** 3.2 to 1.0 %ID/g, **(3)** 5.9 to 2.5 %ID/g and **(4)** 9.3 to 3.7 %ID/g) and these data are supported by the imaging data Figure 2a where the 1 hr uptake of all tracers were significantly reduced at 18 hr by 55-65%.

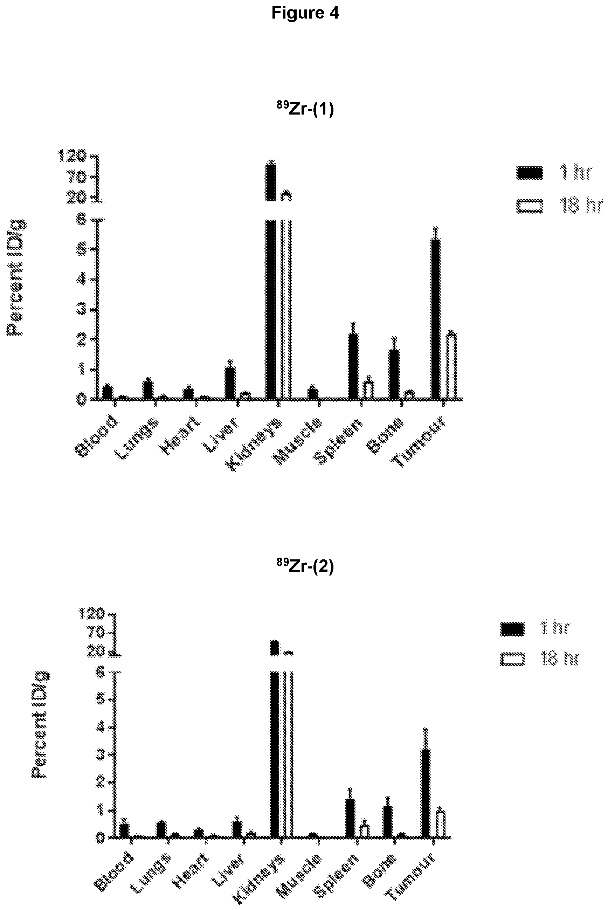

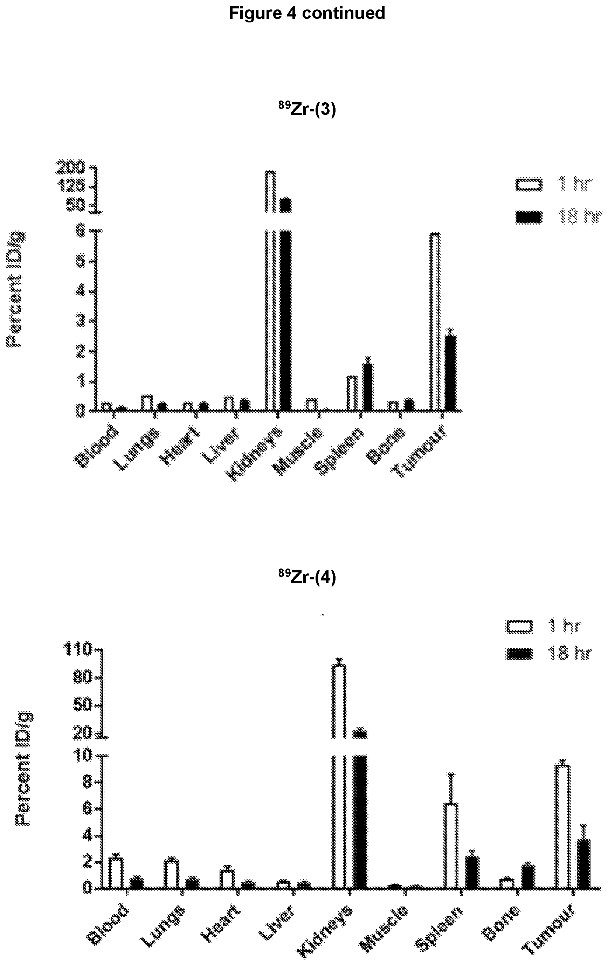

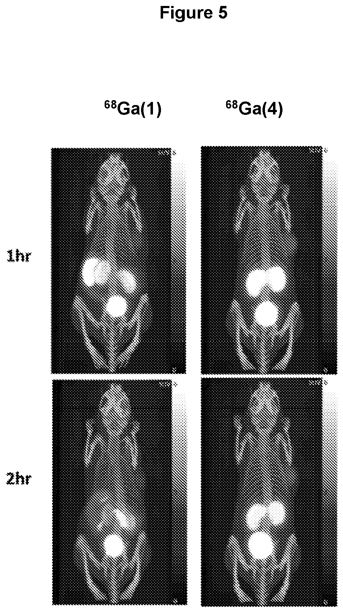

*⁶⁸Ga Labelled tracers:* Representative PET images of mice injected with ⁶⁸Ga**(1)** and ⁶⁸Ga**(4)** are shown in Figure 4 and quantitation of the tumour SUVₘₐₓ is shown in Figure 5a. The PET images show high uptake of ⁶⁸Ga**(1)** into kidney, gall bladder and gut with moderate uptake into LNCap tumours. ⁶⁸Ga**(4)** was associated with high kidney uptake, moderate tumour uptake and no gut uptake. Tumour uptake of ⁶⁸Ga**(4)** was 1.6 and 1.8 fold higher than ⁶⁸Ga**(1)** at 1 and 2 hr p.i., respectively. This is consistent with the biodistribution results where tumour %ID/g for ⁶⁸Ga**(4)** was higher than ⁶⁸Ga**(1)** at 1 hr (10.8 ± 1.3 vs 6.5 ± 0.4) and at 2.5 hr (8.6± 1.0 vs 4.1 ± 0.5) p.i. The tissue biodistribution for the tracers at 1 and 2 hr is summarised in Figure 5b. Significant tumour clearance of ⁶⁸Ga**(1)** was evident at 2.5 hr p.i. (6.5 ± 0.4%ID/g at 1 hr vs 4.1± 0.5 %ID/g at 2.5 hr). In contrast, there was no significant change in ⁶⁸Ga**(4)** tumour retention at 1 and 2.5 p.i. (10.8 ± 1.3 % ID/g vs 8.6 ± 0.9 %ID/g, Figure 5b). These data are supported by the imaging data (Figure 5a) where the 1 hr uptake of ⁶⁸Ga**(1)** was reduced by 13% and ⁶⁸Ga**(4)** uptake by 9% at 2 hr, respectively.

### Cell binding and internalization studies

The surface binding of **(1)** in the presence and absence of excess PMPA was similar at all timepoints (2 % AR/mg protein). While internalised activity increased over the 60 minute incubation to 7% AR/mg protein, internalisation up to 5 % AR/mg protein was seen in the presence of excess PMPA. Minimal binding was seen in the DU145, PSMA negative cell line. At 60 min <6% of added radioactivity/mg protein (%AR/mg) was cell surface bound but over >39% %AR/mg was internalised. Internalised activity increased over the course of the 60-minute study. Internalised and surface bound activity were effectively blocked with excess PMPA with surface bound activity was <2.5 % and internalised activity at < 13% AR/mg protein. Minimal binding was seen in the DU145, PSMA negative cell line.

A variety of new mono- and dimeric PSMA conjugates were designed and synthesized. The length and lipophilicity of the linker between the chelator part and the PSMA binding motif was surprisingly found to be an important factor to control the physiological properties and binding affinities of the tracer. The PSMA conjugates were readily radiolabelled with ⁸⁹Zr to provide radiolabelled tracer in high radiochemical yield and purity. The *in vivo* data correlates very well with the *in vitro* studies. ⁸⁹ZrDFOSq-bisPhPSMA showed specific binding and rapid internalisation into LNCap cells *in vitro.* Based on the results obtained from ⁸⁹Zr studies, two tracers were selected to investigate the ⁶⁸Ga radiolabelling and in vivo studies. ⁶⁸Ga**(1)** was associated with higher gut uptake than ⁶⁸Ga**(3)** and significantly cleared from LNCaP tumours at 2.5 hr post injection. ⁶⁸Ga**(4)** showed higher tumour uptake by both PET imaging and biodistribution than the monomeric analogue and these results are consistent with the data obtained for ⁸⁹Zr studies.

**Table 1 In vivo (imaging and biodistribution) and in vitro (cell uptake) data for the ⁸⁹Zr tracers**

| | **Tumour Uptake %ID/g** | | | **Tumour SUVₘₐₓ** | | | **Cell Uptake %AR/mg protein (60 min)** | |
|---|---|---|---|---|---|---|---|---|
| **Tracer** | 1h pi | 18h pi | Clearance % | 1h pi | 18h pi | Clearance % | Surface bound | internalized |
| **⁸⁹Zr(1)*** | 4.6±0.45 | 1.7±0.18 | 61 | 1.1±0.12 | 0.4±0.05 | 63 | 2.8±0.73 | 7.1±0.94 |
| **⁸⁹Zr(2)** | 3.2±0.72 | 0.9±0.09 | 69 | 0.7±0.03 | 0.3±0.01 | 57 | - | - |
| **⁸⁹Zr(3)**** | 5.9 | 2.5±0.22 | 58 | 1.7±0.12 | 0.6±0.06 | 65 | 2.2±0.14 | 3.8±0.39 |
| **⁸⁹Zr(4)** | 9.3±0.33 | 3.7±1.07 | 60 | 3.2±0.30 | 1.5±0.18 | 54 | 5.8±0.47 | 39.1±3.2 |

| | | | | | | | | |
|---|---|---|---|---|---|---|---|---|
| * n=6 for biodistribution and PET imaging at each time point ** n=1 for biodistribution at 1hr time point | | | | | | | | |

**Table 2 In vivo (imaging and biodistribution) and in vitro (cell uptake) data for the ⁶⁸Ga Labelled PSMA tracers**

| | **Tumour Uptake %ID/g** | | | **Tumour SUVₘₐₓ** | | |
|---|---|---|---|---|---|---|
| **Tracer** | 1h pi | 2.5 h pi | Clearance % | 1h pi | 2h pi | Clearance % |
| **⁶⁸Ga(1)** | 6.5±0.38 | 4.1±0.51 | 36 | 1.0±0.02 | 0.8±0.05 | 17 |
| **⁶⁸Ga(4)** | 10.8±1.26 | 8.6±0.93 | 20 | 1.6±0.10 | 1.5±0.09 | 7 |

Several PSMA tracers based on lys-urea-glu urea-linked dipeptide moiety were designed, synthesized and strategically bio-conjugated to DFOSq ligand in a mono- and dimeric arrangement of PSMA binding motif. The ligands can be readily radiolabelled with ⁸⁹Zr and ⁶⁸Ga radionuclides under mild condition providing high radiochemical yield and purity. The tracers showed potential of diagnostic imaging of prostate cancer based on PET-CT and biodistribution analyses.

### Example 2 - Radio imaging agents targeting SSTR2 in Neuroendocrine Tumours

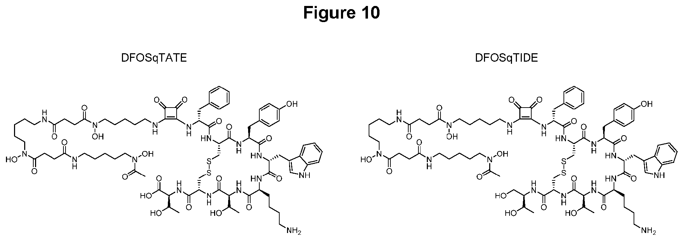

The inventors developed and compared two new radio tracers based on ⁸⁹ZrDFO-sq conjugated somatostatin analogues such as octreotide and octreotate (Figure 10). Both peptides bind to somatostatin subtype 2 receptors (sstr2) that are overexpressed in many types of neuroendocrine tumours.

### Material and Methods

***General Experimental and Materials.*** Unless otherwise stated, all reagents were purchased from commercial sources and used without further purification. Flash column chromatography was carried out using silica gel (40-63 microns) as the stationary phase. Analytical TLC was performed on pre-coated silica gel plates (0.25 mm thick, 60F254, Merck, Germany) and visualized under UV light. HPLC purification and analysis of non-radioactive samples were performed on an Agilent 1100 series using solvent A = 0.1 % TFA in Milli and solvent B = 0.1% TFA in CH₃CN. For analytical HPLC Phenomenex Luna^{®} 5 µm C18(2) 100 Å, LC Column 150 x 4.6 mm at a flow rate of 1 mL/min was used while Phenomenex Luna^{®} 5 µm C18(2) 100 Å, LC Column 250 x 21 mm at flow rate of 5-8 mL/min was used for preparative HPLC. Radio-HPLC was performed on a Shimadzu SCL-10A VP/LC-10 AT VP system with a Shimadzu SPD-10A VP UV detector followed by a radiation detector (Ortec model 276 photomultiplier base with preamplifier, Ortec 925-SCINT ACE mate preamplifier, BIAS supply and SCA, Bicron 1M 11/2 photomultiplier tube). Phenomenex Luna^{®} 5 µm C18(2) 100 Å, LC Column 150 x 4.6 mm using 0.05% TFA buffers in MillQ.

***General synthesis of lysine side chain protected Tyr³-Octreotide*/*Octreotate peptides.*** Tyr³-octreotide or Tyr³-octreotate linear peptide with sequence [D-Phe-Cys(Acm)-Tyr-(tBu)-D(Trp)-Lys(Boc)-Thr(tBu)-Cys(Acm)-Thr-(tBu)-OL or OH] was prepared by standard Fmoc automated solid phase peptide synthesis on a CEM Liberty Blue^{™} automated microwave peptide synthesizer. For octreotate peptide the first amino acid Fmoc-Thr(OtBu)-OH was loaded on Wang resin while for octreotide commercially available Fmoc-Threninol(tBu)-2-CI-Trt resin was used. Each coupling cycle used 1 eq. of HATU and 5 eq. DIPEA and 20% piperidine in DMF for deprotection step with no final deprotection of N-terminus Fmoc group. The resin bound linear peptide was cyclized using iodine (1 mg/mg of resin) in DMF (20 mL). The resin cleavage was performed using a 5 mL solution of triisopropylsilane (2.5%), distilled water (2.5%) and 3,6-dioxa-1,8-octanedithiol (2.5%), thioanisole (2.5%) and TFA (90%) with gentle shaking for 2 h at RT. The mixture was then filtered and sparged with N₂ to reduce the volume then ether (40 mL) added to precipitate the peptide which was collected after centrifugation. The crude peptide material purified by semi-preparative reverse phase HPLC (Phenomenex Luna^{®} 5 µm C18(2) 100 Å, LC column 250 x 21 mm using 0.1% TFA buffers in MillQ water and acetonitrile). The identity of peptides was confirmed by ESI-MS. The purified cyclic peptides were dissolved in DMF (1 mL) and treated with di-tert-butyldicarbonate (5 eq.) in the presence of DIPEA (1 eq.) for 4 hours at RT to protect the lysine side chain with Boc group. Cold diethyl ether was added to reaction mixture to precipitate the product which was collected by centrifugation. The residue was treated with 20% piperidine in DMF (1 mL) for 30 min at RT then precipitated with cold diethyl ether and the residue was collected by centrifugation and purified by semi-preparative reverse phase HPLC (Phenomenex Luna^{®} 5 µm C18(2) 100 Å, LC Column 250 x 21 mm using 0.1% TFA buffers in MillQ water and acetonitrile). ESI-MS: Lys(Boc)TIDE (+ve ion) [M + H⁺] *m*/*z* = 1135.4952 (experimental), calculated for [C₅₄H₇₅N₁₀O₁₃S₂]⁺: *m*/*z* = 1135.4956; Lys(Boc)TATE (+ve ion) [M + H⁺] *m*/*z* = 1149.4739 (experimental), calculated for [C₅₄H₇₃N₁₀O₁₄S₂]⁺: *m*/*z* = 1149.4749.

*General procedure for the DFOSq conjugation of Octreotide*/*Octreotate peptides.* The lysine(Boc) side chain protected octreotide and octreotate peptides and DFO-Sq (5 eq.) were dissolved in DMSO (100 µL) then borate buffer (0.1 M, pH 9.0, 900 uL) was added. The solution was slowly agitated at room temperature for seven days then neutralized with 10% TFA and purified semi-preparative reverse phase HPLC (Phenomenex Luna^{®} 5 µm C18(2) 100 Å, LC column 250 x 21 mm using 0.1% TFA buffers in MillQ water and acetonitrile). The purified fractions were treated with 20% TFA in DCM for 30 min at RT then TFA was removed under N₂ flow followed by HPLC (Phenomenex Luna^{®} 5 µm C18(2) 100 Å, LC column 250 x 21 mm using 0.1% TFA buffers in MillQ water and acetonitrile). ESI-MS: DFOSqTIDE (+ve ion) [M + H⁺] *m*/*z =* 1673.7716 (experimental), calculated for [C₇₈H₁₁₃N₁₆O₂₁S₂]⁺: m/z = 1673.7708; DFOSqTATE (+ve ion) [M + H⁺] *m*/*z* = 1687.7500 (experimental), calculated for [C₇₈H₁₁₁N₁₆O₂₂S₂]⁺: m/z = 1687.7500.

***⁸⁹Zr** Radiolabelling **DFOSqTIDE.*** ⁸⁹Zr in 1 M oxalic acid (60 µL, 60 MBq , Perkin Elmer NEZ308000MC) was diluted with MilliQ water (60 µL) then was neutralized (pH 6-7) with a series of small volume additions of aqueous Na₂CO₃ (1 M, 4 x 10 µL). HEPES buffer (54 µL,1 M, pH 7.0) was then added and the solution allowed to stand for 5 min before pH was tested again. After confirming the mixture had a pH 6-7 a solution of DFO-Sq-TIDE (120 µg in 60 µL of 0.25 M HEPES buffer, 10% DMSO, 1.18 nmol/MBq) was added and the reaction mixture was left to stand at room temperature for 30 min then an aliquot was analysed by radio-HPLC (20-100% of buffer B to A at 1 mL/min over 15 min, A = 0.05% TFA in MilliQ and B = in 0.05% TFA in Acetonitrile, Luna C18 column 4.6 × 150 mm, 5 µm). The crude tracer was purified by Phenomenex StrataX cartridges (C18, 60 mg) using ethanol as eluent. Several fractions (≈100 µL) were collected and the fraction containing most activity was diluted in sterile filtered PBS to a final ethanol concentration of 8% (v/v). Six syringes (BD Ultra-Fine^{™}, 0.3 mL) containing approximately 2.2 MBq (4.4 µg of peptide, 165 µL, 2.6 nmol) each was prepared.

***⁸⁹Zr** Radiolabelling **DFOSqTATE.*** ⁸⁹Zr in 1 M oxalic acid (70 µL, 56 MBq , Perkin Elmer NEZ308000MC) was diluted with MilliQ water (70 µL) then was neutralized (pH 6-7) with a series of small volume additions of aqueous Na₂CO₃ (1 M, 4 x 10 µL). HEPES buffer (62 µL,1 M, pH 7.0) was then added and the solution allowed to stand for 5 min before pH was tested again. After confirming the mixture had a pH 6-7 a solution of DFO-Sq-TATE (112 µg in 56 µL of 0.25 M HEPES buffer, 10% DMSO, 1.19 nmol/MBq) was added and the reaction mixture was left to stand at room temperature for 30 min then an aliquot was analysed by radio-HPLC (20-100% of buffer B to A at 1 mL/min over 15 min, A = 0.05% TFA in MilliQ and B = in 0.05% TFA in Acetonitrile, Luna C18 column 4.6 × 150 mm, 5 µm). The crude tracer was purified by Phenomenex StrataX cartridges (C18, 60 mg) using ethanol as eluent. Several fractions (≈100 µL) were collected and the fraction containing most activity was diluted in sterile filtered PBS to a final ethanol concentration of 8% (v/v). Six syringes (BD Ultra-Fine^{™}, 0.3 mL) containing approximately 2.5 MBq (5 µg of peptide, 150 µL, 2.9 nmol) each was prepared.

*PET-CT imaging and Biodistribution of Octreotate*/*tide Tracers.* Female Balb/c nude mice (age 9 weeks) were inoculated subcutaneously on the right flank with 3 x 10⁶ AR42J cells in PBS:Matrigel (1:1). Mice were weighed and tumours measured twice weekly using electronic callipers. Tumour volume (mm³) was calculated as length² x width/2. 12 mice were assigned for imaging and biodistribution studies (tumour volumes: 170-550 mm³). DFOSqTIDE (2.5 MBq) and DFOSqTATE (2.2 MBq) were administered intravenously by tail vein injection to mice on. At 1, 2, 4 and 18 hr post injection three mice were anaesthetised using isoflurane and placed on the imaging bed of a G8 PET/CT scanner (Perkin Elmer). A CT scan was performed which was immediately followed by a 10 min static PET scan. PET images were reconstructed using the maximal likelihood and expectation maximization (ML-EM) algorithm. PET images were analysed using VivoQuant (Invicro) and Tumour SUVmax, Tumour SUVmax/background average (TBR) and Tumour SUVmax/liver average (TLR) determined. After imaging at 18hr mice were euthanised and selected tissues were excised, weighed and counted using a Capintec (Captus 4000e) gamma counter. A separate cohort of 3 mice was harvested at 1 hr post injection for biodistribution analysis. The data was analysed using GraphPad Prism 7 and differences between tracers analysed using a t-test.

***⁶⁸Ga** Radiolabelling of DFOSqTIDE.* ⁶⁸Ga^{III} in HCl (630 µL, 44 MBq) was buffered with sodium acetate (1 M, 70 µL, pH 4.5) then H₃DFOSq-TIDE (6 µg in 6 µL DMSO, 3.3 nmoles) and the reaction mixture was left to stand at room temperature for 15 min then an aliquot was analysed by radio-HPLC (0-95% of buffer B to A at 1 mL/min over 15 min, A = 0.05% TFA in MilliQ and B = in 0.05% TFA in Acetonitrile, Luna C18 column 4.6 × 150 mm, 5 µm). The traces showed more >98% radiochemical yield with a radiochemical purity of >98%. The reaction mixture was diluted with MilliQ water (470 µL) then buffered with 10 × PBS buffer (130 µL, pH 7.4) to to a final vollume of 1.3 mL. Six syringes (BD Ultra-Fine^{™}, 0.3 mL) containing approximately 3.5 MBq in 200 µL (approx. peptide mass 1 µg, 0.5 nmoles) were prepared.

**⁶⁸*****Ga** Radiolabelling of **DFOSqTATE.*** ⁶⁸Ga^{III} in HCl (900 µL, 40 MBq) was buffered with sodium acetate (1 M, 100 µL, pH 4.5) then H₃DFOSq-TATE (6 µg in 6 µL DMSO, 3.3 nmoles) and the reaction mixture was left to stand at room temperature for 15 min then an aliquot was analysed by radio-HPLC (0-95% of buffer B to A at 1 mL/min over 15 min, A = 0.05% TFA in MilliQ and B = in 0.05% TFA in Acetonitrile, Luna C18 column 4.6 × 150 mm, 5 µm). The traces showed more >95% radiochemical yield with a radiochemical purity of >95%. The reaction mixture was diluted with MilliQ water (100 µL) then buffered with 10 × PBS buffer (110 µL, pH 7.4) to to a final vollume of 1.2 mL. Six syringes (BD Ultra-Fine^{™}, 0.3 mL) containing approximately 2.3 MBq in 200 µL (approx. peptide mass 1.0 µg, 0.5 nmoles) were prepared.

### Results and Discussion

### Synthesis and radiolabelling or DFOSqTIDE/ DFOSqTATE

The resin bound linear Tyr³-octreotide/tate peptides were prepared using standard automated solid phase peptide synthesis techniques with Fmoc protected amino acids, using HATU/DIPEA coupling on Wang and chlorotrityl resin with no final Fmoc deprotection step. The deprotection of Fmoc groups were achieved with 20% piperidine in DMF on the solid phase for each cycle. The cyclization through intramolecular disulfide bridge between the second and seventh cysteine residues were achieved via in-situ deprotection of acetamido methyl (Acm) on cysteine residues followed disulphide bond formation using iodine in DMF. Following cyclization, final cleavage from solid support and deprotection of remaining protecting groups were achieved using standard trifluoracetic acid cocktail (Scheme 3). The bioconjugation of DFOsq is desired specifically on N-terminus D-Phe unit as the D(Trp)-Lys-Thr part of the peptide plays important role in receptor binding so the lysine unit was protected using Boc anhydride followed by final Fmoc deprotection on D-Phe unit using 20% piperidine in DMF. The resulting Lys(Boc)-peptides shows better solubility in borate buffer and the coupling with DFOSq was achieved by incubating the peptide and DFOSq in 0.1 M borate buffer (pH 9.0) with 10% DMSO over 7 days. The final DFOsq conjugated peptide assembly was obtained after the deprotection of Boc group from lysine with TFA followed by HPLC purification.

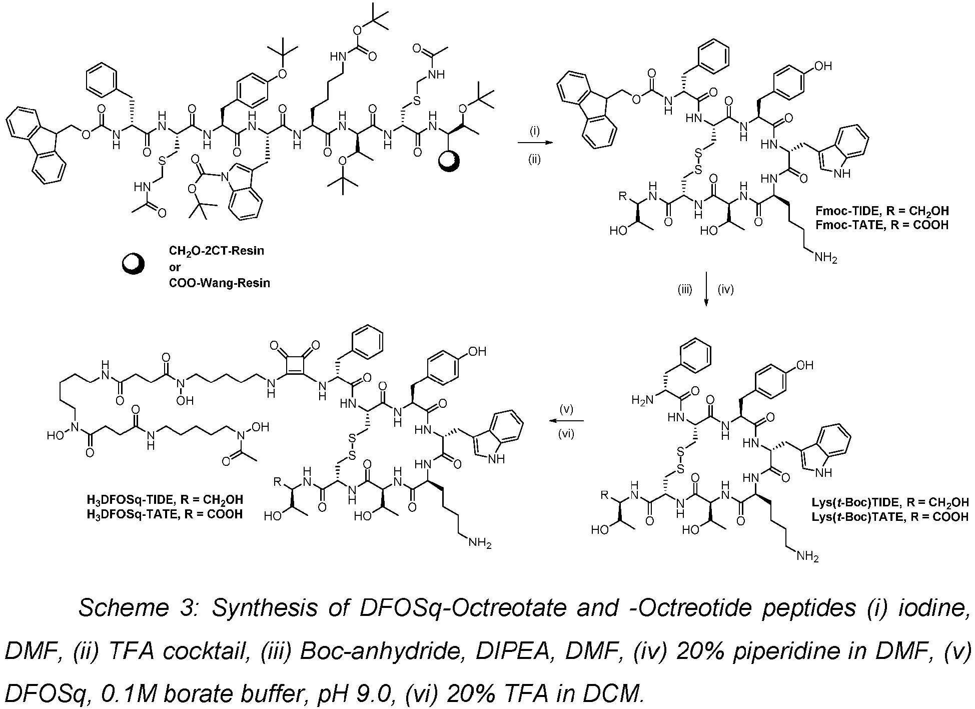

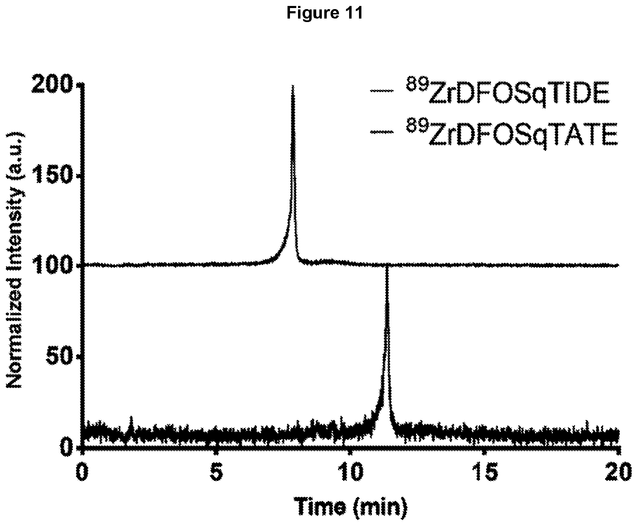

The radiolabelling of both peptides with ⁸⁹Zr was achieved under mild reaction conditions. ⁸⁹Zr was obtained in 1 M oxalic acid solution which was neutralized with 1 M Na₂CO₃ and buffered with HEPES to a final concentration of 0.25 M. The peptides were labelled at a concentration of 2 µg/MBq of ⁸⁹Zr and the analytical radio-HPLC shows radiochemical yield of >95% in 30-60 min reaction time. The crude tracers were purified with Phenomenex StaraX C18 cartridges using ethanol as eluent producing the tracers in >98% radiochemical purity (Figure 11).

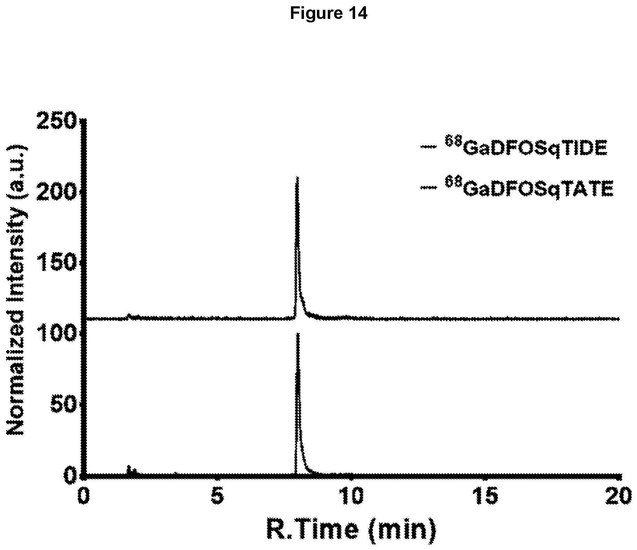

Radiolabelling of both DFOSq-TATE and DFO-TIDE with [⁶⁸Ga]Ga^{III} was achieved at room temperature in 10 minutes. An aqueous mixture of [⁶⁸Ga]Ga^{III}, obtained from elution of a ⁶⁸Ge/⁶⁸Ga generator with 0.05 M HCl, was partially neutralised to pH 4-5 with sodium acetate buffer (1 M, pH 4.5). Even with the use of relatively low peptide mass (approx. 150 ng/MBq of [⁶⁸Ga]Ga^{III}) it was possible to obtain ⁶⁸GaDFOSqTATE and ⁶⁸GaDFOSq-TIDE in high radiochemical yield (≥98%) and purity ((≥98%) requiring no purification of the crude tracer for *in vivo* experiments (Figure 14).

### PET-CT imaging and Biodistribution of ⁸⁹Zr-DFOSqTIDE/ DFOSqTATE

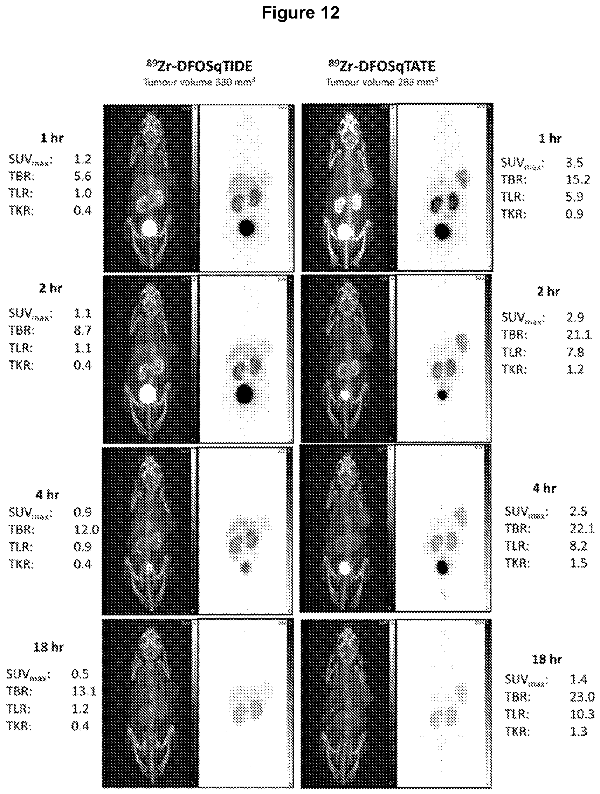

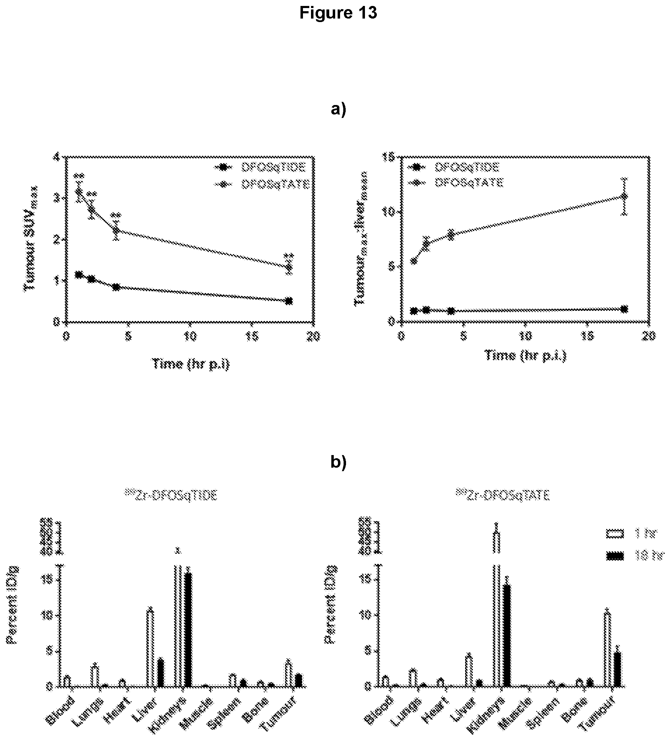

AR42J (rat pancreatic cancer cell line) tumour bearing Balb/c nude mice as described above were injected intravenously via tail vein injection with ⁸⁹ZrDFOSqTATE or ⁸⁹ZrDFOSqTIDE (2-3 MBq). At 1, 2, 4 and 18 h post-injection the mice were anaesthetised with isoflurane and imaged over 10 min. At 1 hr after injection of DFOSqTIDE and DFOSqTATE, uptake into kidney, tumour and liver was observed. Representative PET images of mice injected with DFOSqTIDE and DFOSqTATE are shown in Figure 12 and quantitation of the tumour SUVₘₐₓ for each radiotracer is summarised in Figure 13a.

These findings are consistent with the biodistribution results where tumour %ID/g for DFOSqTATE was higher than DFOSqTIDE at 1 hr (10.4±0.5 vs 3.4 ±0.4, P<0.001) and 18 hr (4.9±0.8 vs 1.7±0.1, P<0.05). The imaging data shows high uptake of DFOSqTIDE by the liver resulting in a lower tumour to liver ratio than for DFOSqTATE. The biodistribution data confirms the higher accumulation of DFOSqTIDE in the liver compared with DFOSqTATE (10.8±0.4 vs 4.3 ± 0.4 %ID at 1 hr post injection, P<0.01). The biodistribution data in Figure 13b demonstrates both radiotracers were cleared significantly from the tumour at 18 hr, For DFOSqTIDE the %ID/g was reduced from 3.4± 0.4 at 1 hr to 1.5 ± 0.1 at 18 hr (P < 0.05) and DFOSqTATE from 10.4± 0.5 at 1 hr to 4.9 ± 0.8 at 18 hr (P<0.01). These data are supported by the imaging data (Fig. 12) where the 1 hr uptake of DFOSqTIDE was reduced by 55 % at 18 hr (P < 0.01) and DFOSqTATE uptake by 58% (P < 0.01).

In summary, ⁸⁹Zr-DFO-SqTATE exhibited higher AR42J tumour uptake and lower liver accumulation than 89Zr-DFO-Sq-TIDE in Balb/c nude mice. Both radiotracers were cleared significantly from AR42J tumours by 18 hr post injection.

**Table 3. In vivo (imaging and biodistribution) and in vitro (cell uptake) data for the ⁸⁹Zr Labelled tracers**

| | **Tumour Uptake %ID/g** | | | **Tumour SUVₘₐₓ** | | |
|---|---|---|---|---|---|---|
| **Tracer** | 1h pi | 18h pi | Clearance % | 1h pi | 18h pi | Clearance % |
| **⁸⁹ZrDFOSqTIDE** | 3.4±0.4 | 1.7±0.1 | 50 | 1.1±0.07 | 0.5±0.01 | 55 |
| **⁸⁹ZrDFOSqTATE** | 10.4±0.5 | 4.9±0.8 | 53 | 3.1±0.2 | 1.3±0.15 | 58 |

Both the peptides can be readily radiolabelled with ⁶⁸Ga under mild reaction conditions. ⁶⁸Ga was obtained as HCl solution which was buffered with sodium acetate (1 M, pH 4.5) to raise the pH to 4.0 before labelling. The analytical radio-HPLC of the reaction mixture after 10 min shows complete labelling in high radiochemical purity requiring no purification (Figure 14).

Two new SSTR2 binding Tyr³- octreotate and octreotide peptides were designed, synthesized and strategically bio-conjugated to DFOSq ligand to N-terminus. The peptides are easy to synthesize and can be readily radiolabelled with ⁸⁹Zr and ⁶⁸Ga radionuclides under mild condition providing high radiochemical yield and purity. Both peptides showed potential of diagnostic imaging of neuroendocrine tumours based on PET-CT and biodistribution analyses.

### PET-CT imaging and Biodistribution of ⁶⁸GADFOSqTIDE/ DFOSqTATE

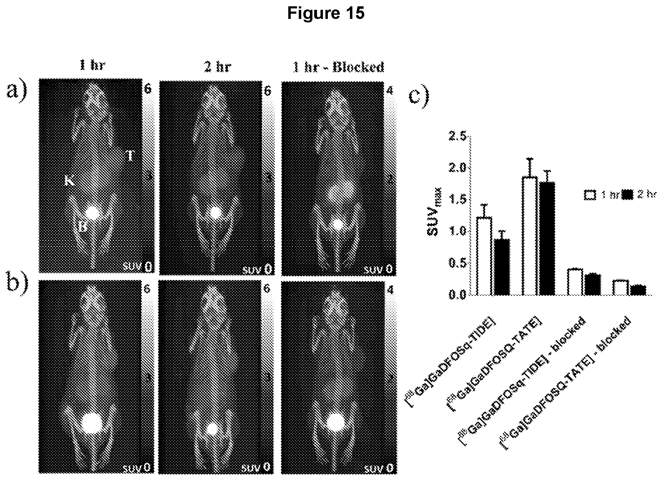

⁶⁸GaDFOSq-TIDE and ⁶⁸GaDFOSq-TATE tracers were injected (2-3 MBq, 1 µg, 0.5 nmol) intravenously via tail vein to AR42J (rat pancreatic cancer cell line) tumour bearing Balb/c nude mice. PET images were acquired at 1 and 2 h post-injection (Figure 15a and b). Inspection of the PET images reveals clear delineation of the tumor for both tracers but ⁶⁸GaDFOSq-TATE has higher tumor uptake. Quantification of the uptake in the images using Standard Uptake Value (SUVₘₐₓ = radioactivity in a tissue/injected activity/BW) confirms the higher uptake for⁶⁸GaDFOSq-TATE (1 h; SUVₘₐₓ 1.8 ± 0.2, compared to 1.21 ± 0.20, Figure 15c). The higher tumor uptake of ⁶⁸GaDFOSqTATE was also associated with a higher degree of tumor retention at 2 h post injection with only a 5% reduction in SUVₘₐₓ whereas the tumor SUVₘₐₓ for ⁶⁸GaDFOSqTIDE reduced by 30% ⁶⁸GaDFOSqTATE at 2 h post injection. Both tracers clear rapidly from the blood and there is low uptake in bone consistent with stable gallium-68 complexes as well as low uptake in muscle. Both tracers have significant uptake in the kidneys and bladder. Addition of an excess of the respective nonradioactive peptides (20 times, 20 µg, 11.1 nmol) results in a significant reduction in tumour uptake suggesting the uptake of both tracers in the tumor is receptor mediated.

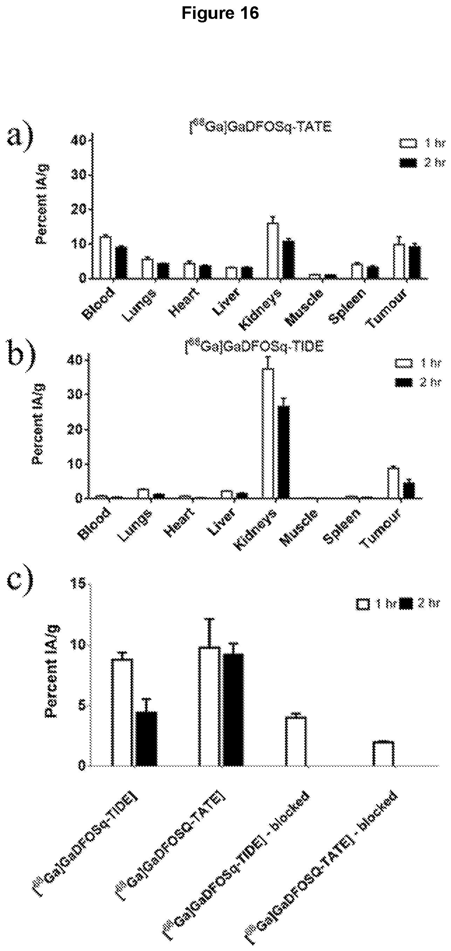

The high tumor uptake was confirmed by an *ex vivo* biodistibution study in the same mouse model where mice were injected with either ⁶⁸GaDFOSq-TIDE and ⁶⁸GaDFOSq-TATE (2-3 MBq, 1 µg, 0.5 nmol) and then euthanised at either 1 h or 2 h after administration. The amount of injected activity per gram of tissue (%IA/g) in the tumor and major organs was quantified (Figure 16). At 1 hour post injection administration of ⁶⁸GaDFOSqTATE (9.80 ± 2.33 %IA/g) resulted in higher uptake in the tumour than ⁶⁸GaDFOSqTIDE (8.81 ± 1.03 %IA/g respectively) consistent with the PET images. The initial tumour uptake of ⁶⁸GaDFOSqTIDE reduced from 8.81 ± 1.03 %IA/g at 1 h post injection to 4.4 ± 1.1 %IA/g at 2 h post injection . In contrast, the high tumor uptake of ⁶⁸GaDFOSqTATE at 1 h post injection (9.80 ± 2.33 %ID/g) is retained at 2 h post injection (9.22 ± 0.92 %ID/g) consistent with the PET images. ⁶⁸GaDFOSqTIDE displays a higher degree of uptake in the kidneys (1 h, 37.56 ± 3.50 %IA/g) than ⁶⁸GaDFOSqTATE (1h, 15.98 ± 3.27 %IA/g).

## Claims

1. A compound of formula (I) or pharmaceutically acceptable derivative thereof: 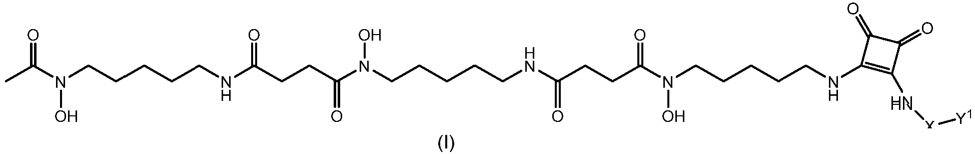 or a pharmaceutically-acceptable salt thereof, wherein X is selected from the group consisting of:
aryl optionally substituted with C₁-C₁₀ alkyl, ethylenediaminetetraacetic acid (EDTA); and
(C₁-C₁₀ alkyl)NR⁷(Y²)R⁸, wherein R⁷ and R⁸ may be independently selected from C₁-C₁₀ alkyl or C₁-C₁₀ alkyl phenyl, wherein C₁-C₁₀ alkyl may be interrupted by n amido groups, wherein n is 0 - 3; and
Y¹ and Y² are independently selected from the group consisting of: carboxylic acid, ester, anhydride, and amine; and
wherein the pharmaceutically acceptable derivative of formula (I) is:

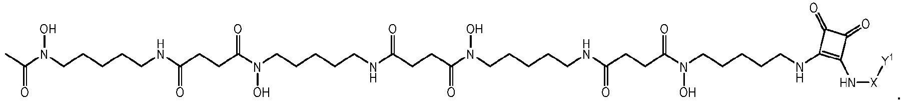

2. The compound of claim 1 or pharmaceutically acceptable derivative thereof, wherein X is selected from the group consisting of: phenyl, benzyl, EDTA functionalised phenyl and (C₁-C₁₀ alkyl)NR⁷(Y²)R⁸.

3. The compound of claim 1 or 2 or pharmaceutically acceptable derivative thereof, wherein Y¹ and Y² are independently amine or carboxylic acid.

4. The compound of any one of claims 1 to 3 wherein the compound is selected from the group consisting of: 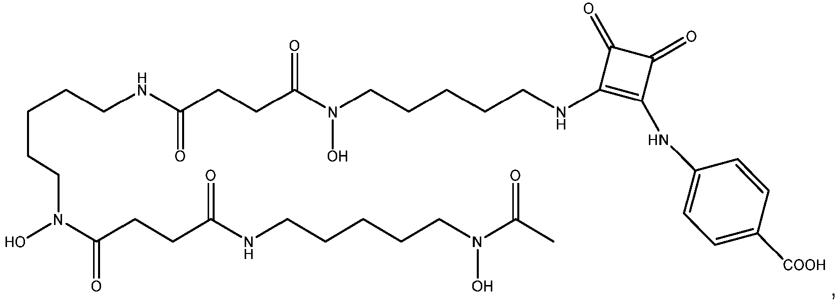 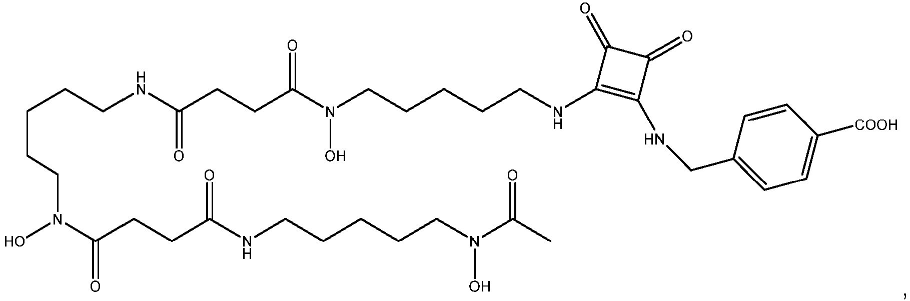 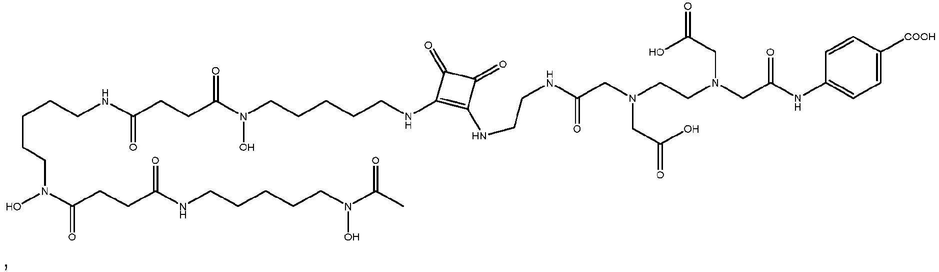 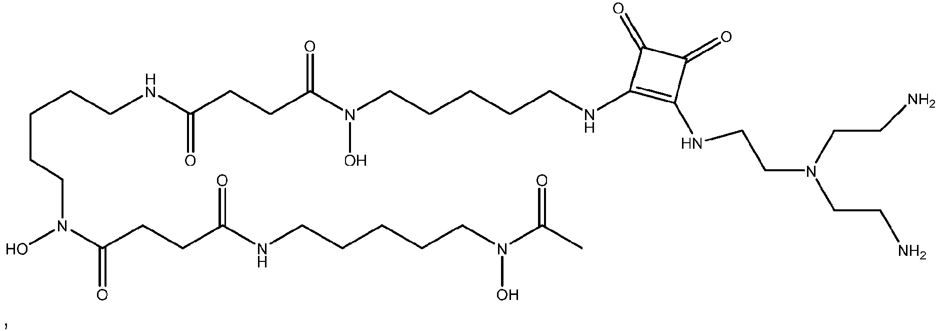 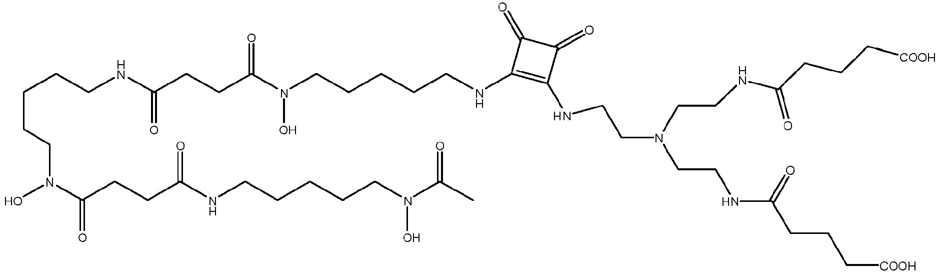 , and

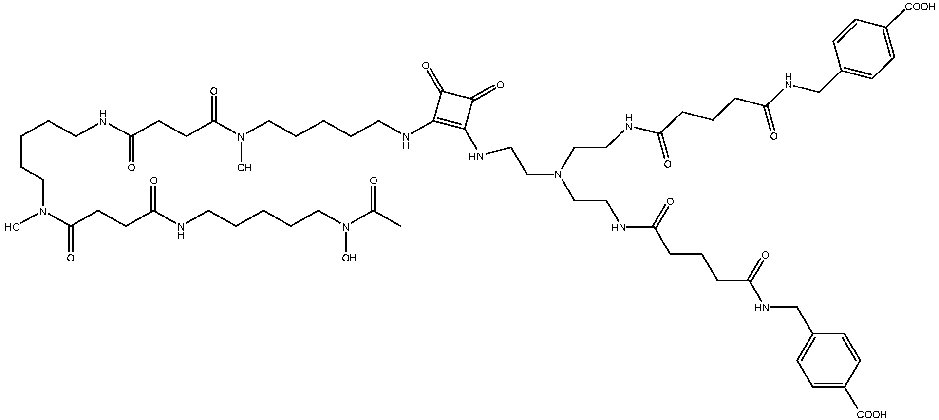

5. The compound of any one of claims 1 to 4, wherein the pharmaceutically acceptable derivative is selected from the group consisting of: 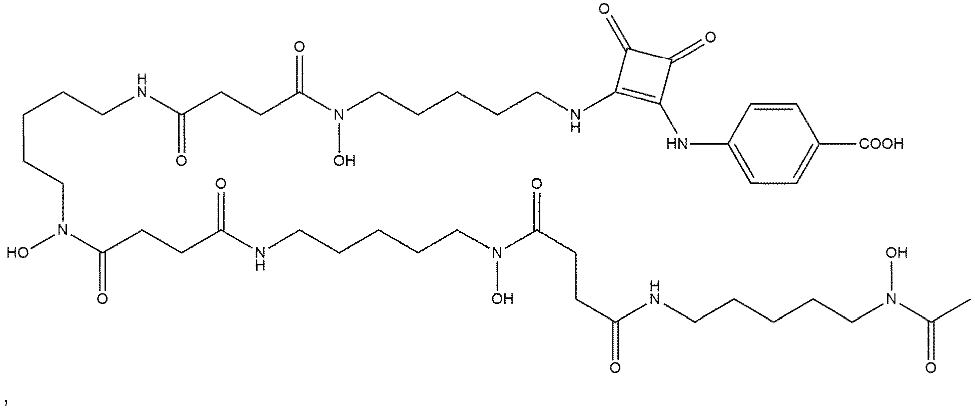 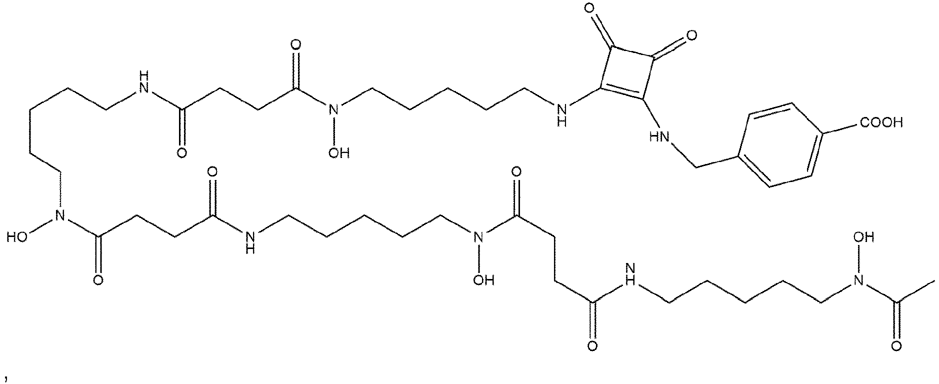 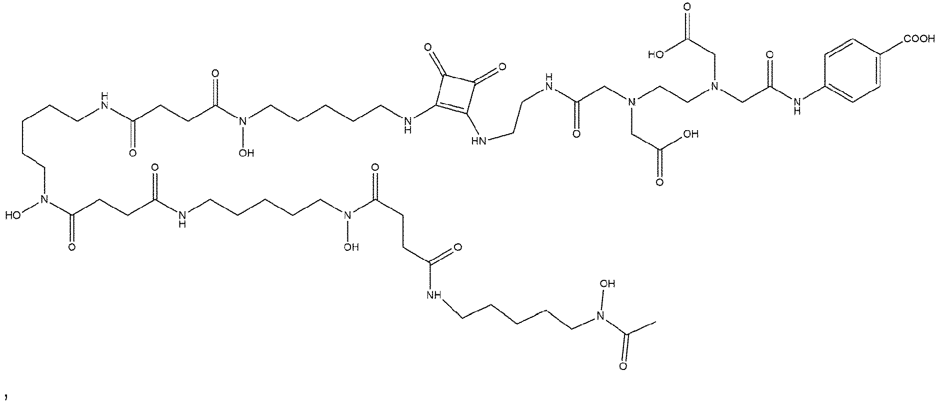 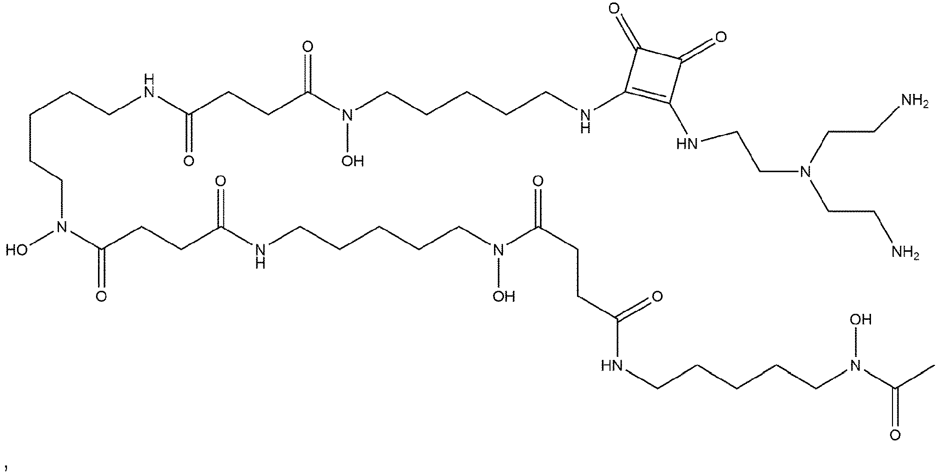 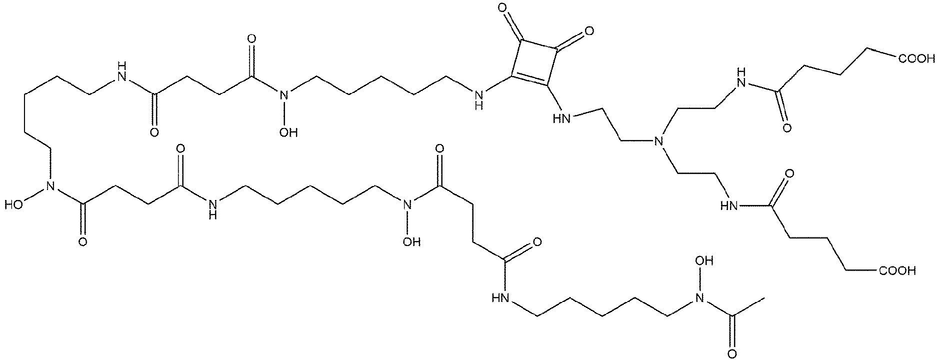 , and

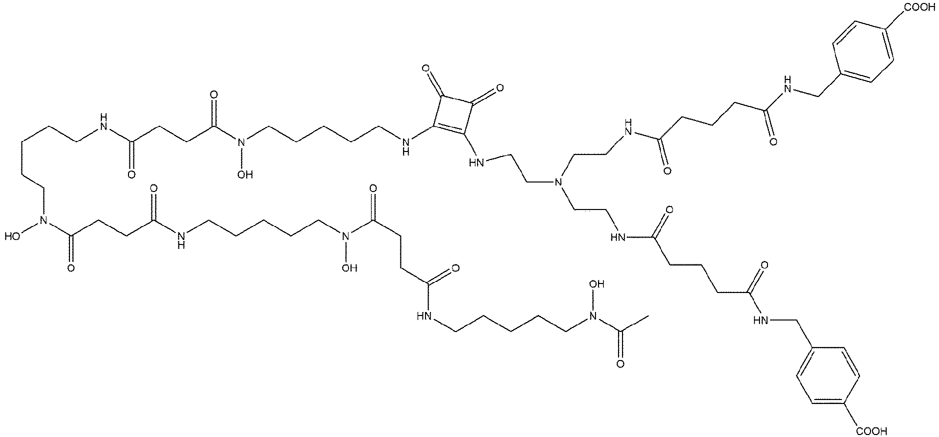

6. A conjugate comprising:
- a compound of formula (I) or pharmaceutically acceptable derivative thereof according to any one of claims 1 to 5, and
- a PSMA targeting agent.

7. The conjugate of claim 6, wherein the conjugate is selected from the group consisting of: 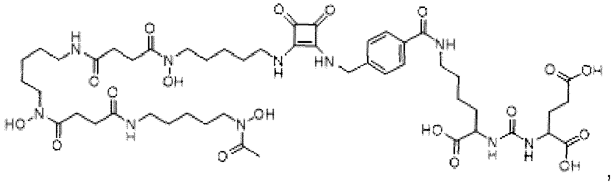 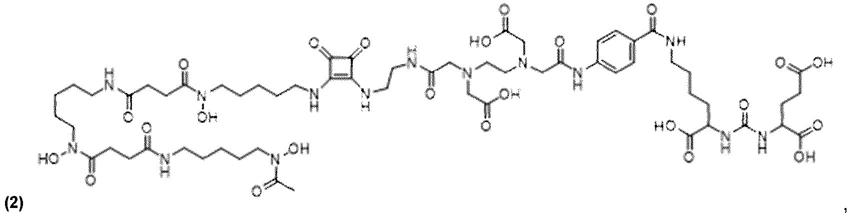 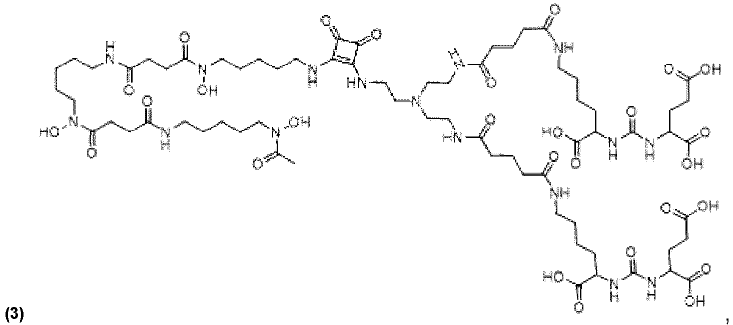 and

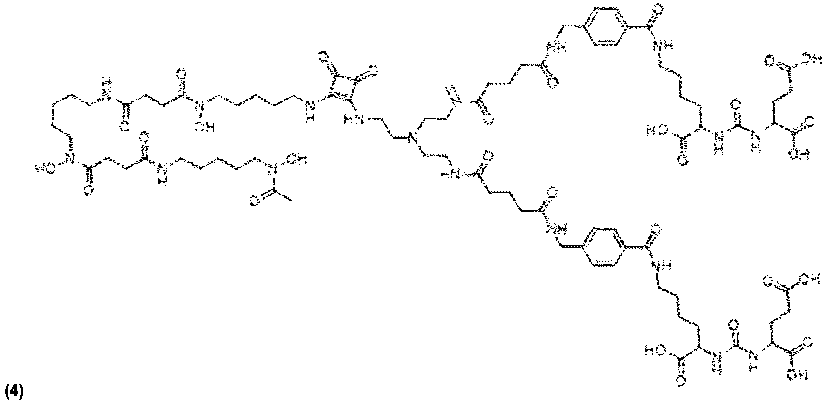

8. A conjugate of formula (II) or pharmaceutically acceptable derivative thereof: 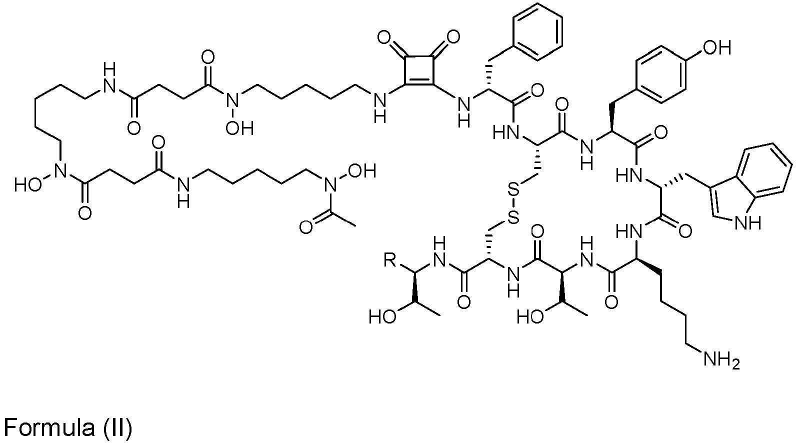 or a pharmaceutically acceptable salt thereof, wherein R is CH₂OH or COOH; and wherein the pharmaceutically acceptable derivative of formula (II) is

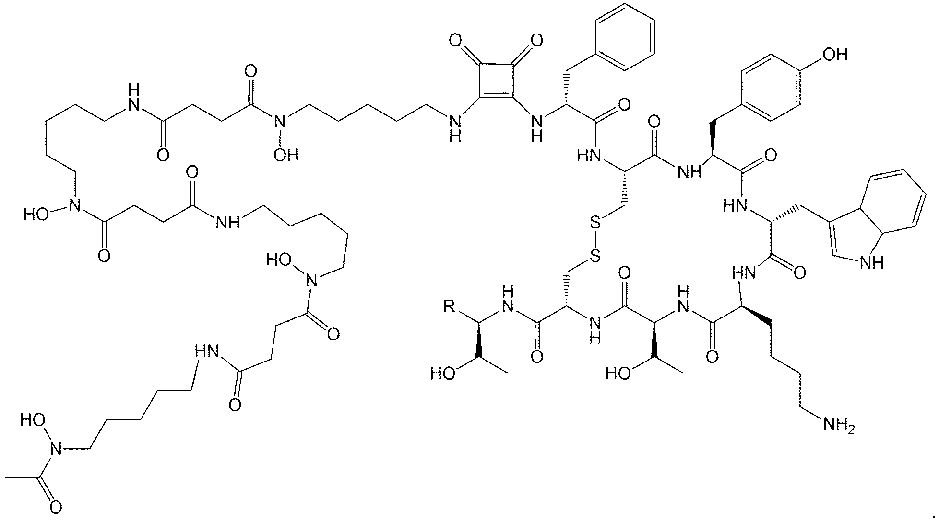

9. A radionuclide-labelled conjugate comprising:
- a conjugate or pharmaceutically acceptable derivative thereof of any one of claims 6 to 8, and
- a radionuclide complexed thereto,
wherein the radionuclide is selected from radioisotopes of zirconium, gallium, lutetium, holmium, scandium, titanium, indium and niobium, preferably wherein the radionuclide is a radioisotope of zirconium (preferably ⁸⁹Zr), gallium (preferably ⁶⁸Ga) or indium (preferably ¹¹¹In), more preferably wherein the radionuclide is a radioisotope of zirconium (preferably ⁸⁹Zr).

10. A pharmaceutical composition comprising the radionuclide-labelled conjugate of claim 9, and one or more pharmaceutically acceptable carrier substances, excipients and/or adjuvants, preferably wherein the pharmaceutical composition further comprises a drug having anti-cancer activity.

11. The radionuclide-labelled conjugate of claim 9 for use in a method of imaging a patient, the method including:
- administering to a patient the radionuclide-labelled conjugate; and
- imaging said patient.

12. The radionuclide-labelled conjugate for use of claim 11, wherein the targeting agent serves to target the conjugate to a desired site *in vivo,* preferably wherein the desired site is a tumour.

13. The radionuclide-labelled conjugate of claim 9 for use in a method of imaging a cell or *in vitro* biopsy sample, the method including:
- administering to a cell or *in vitro* biopsy sample the radionuclide-labelled conjugate; and
- imaging the cell or *in vitro* biopsy sample.

14. The radionuclide-labelled conjugate of claim 9 for use in the treatment of cancer in a patient.

15. A kit for use in the method or use of any one of claims 11 to 14, the kit comprising:
- a compound of any one of claims 1 to 5, a conjugate of any one of claims 6 to 8, a radionuclide-labelled conjugate of claim 9, or a pharmaceutical composition of claim 10; and
- optionally, a label or package insert with instructions for use in the method or use of any one of claims 11 to 14.

## Patentansprüche

1. Verbindung der Formel (I) oder pharmazeutisch unbedenkliches Derivat davon: 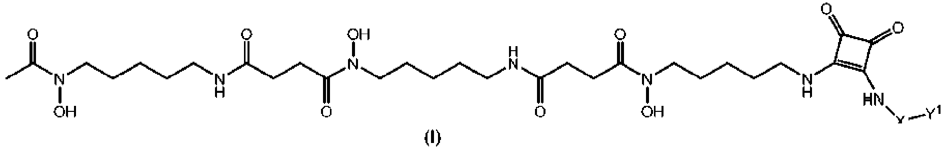 oder pharmazeutisch unbedenkliches Salz davon, wobei X ausgewählt ist aus der Gruppe bestehend aus:
Aryl optional substituiert mit C₁-C₁₀-Alkyl, Ethylendiamintetraessigsäure (EDTA); und
(C₁-C₁₀-Alkyl)NR⁷(Y²)R⁸, wobei R⁷ und R⁸ unabhängig ausgewählt sein können aus C₁-C₁₀-Alkyl oder C₁-C₁₀-Alkylphenyl, wobei C₁-C₁₀-Alkyl durch n Amidogruppen unterbrochen sein kann, wobei n 0-3 ist; und
Y¹ und Y² unabhängig ausgewählt sind aus der Gruppe bestehend aus: Carbonsäure, Ester, Anhydrid und Amin; und
wobei das pharmazeutisch unbedenkliche Derivat der Formel (I) wie folgt ist:

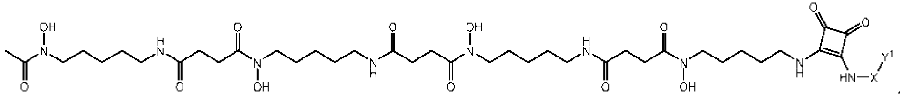

2. Verbindung nach Anspruch 1 oder pharmazeutisch unbedenkliches Derivat davon, wobei X ausgewählt ist aus der Gruppe bestehend aus: Phenyl, Benzyl, EDTA-funktionalisiertem Phenyl und (C₁-C₁₀-Alkyl)NR⁷(Y²)R⁸.

3. Verbindung nach Anspruch 1 oder 2 oder pharmazeutisch unbedenkliches Derivat davon, wobei Y¹ und Y² unabhängig Amin oder Carbonsäure sind.

4. Verbindung nach einem der Ansprüche 1 bis 3, wobei die Verbindung ausgewählt ist aus der Gruppe bestehend aus: 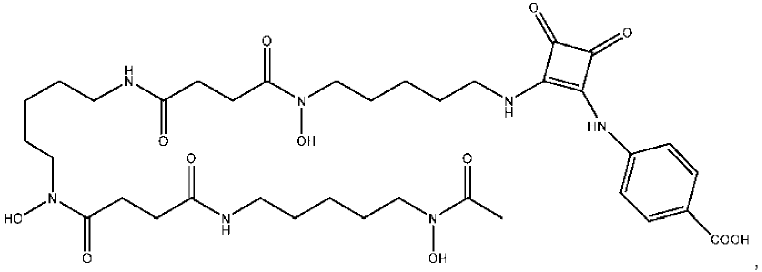 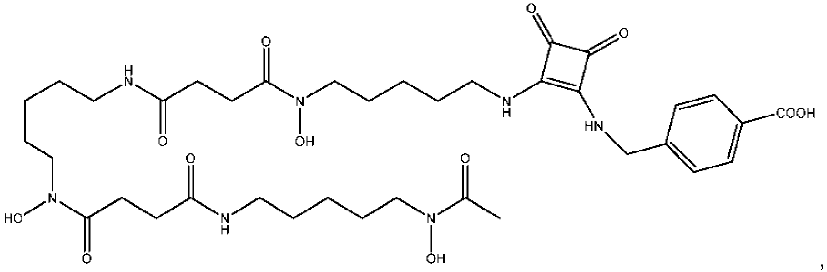 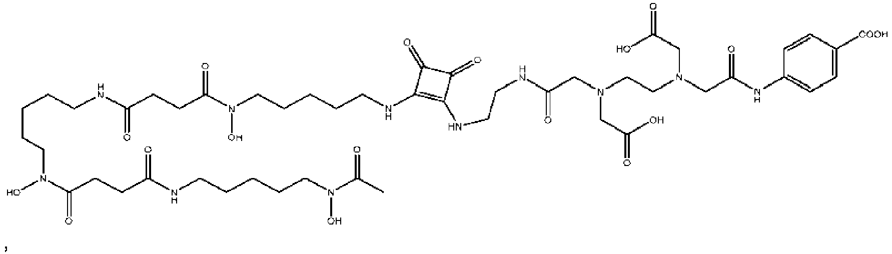 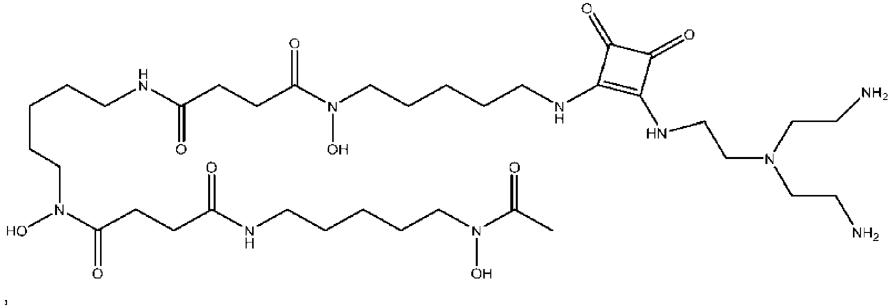 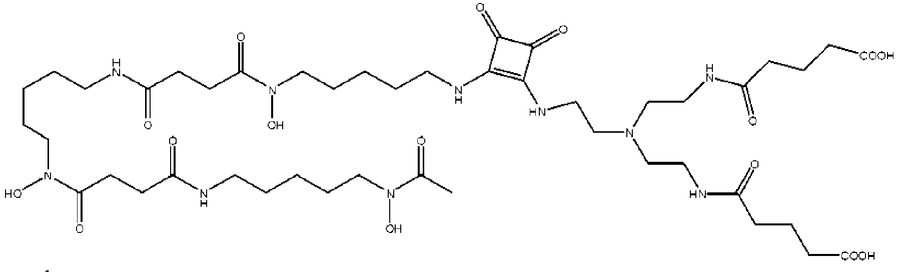 , und

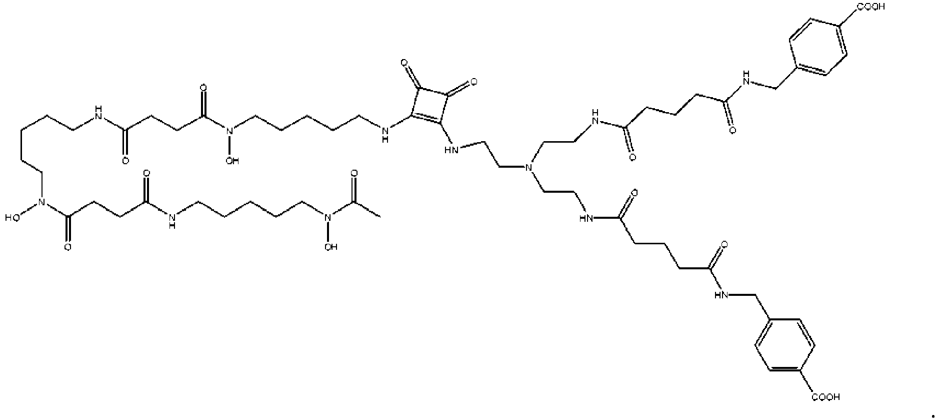

5. Verbindung nach einem der Ansprüche 1 bis 4, wobei das pharmazeutisch unbedenkliche Derivat ausgewählt ist aus der Gruppe bestehend aus: 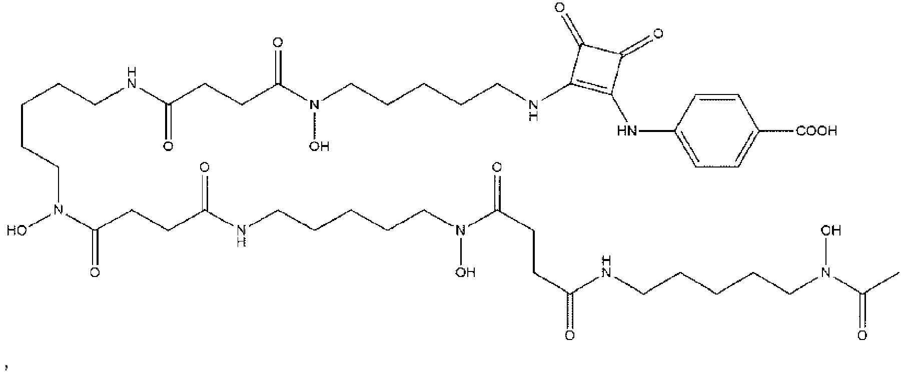 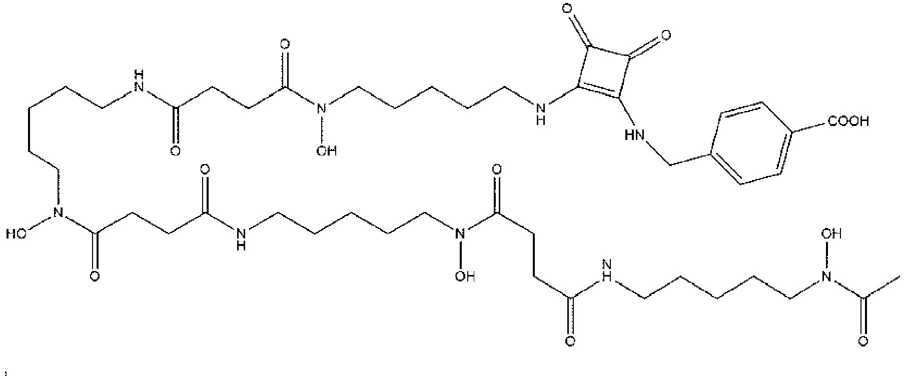 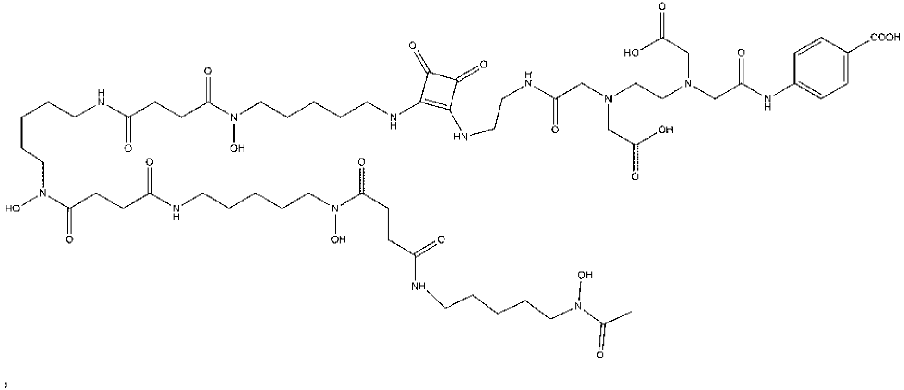 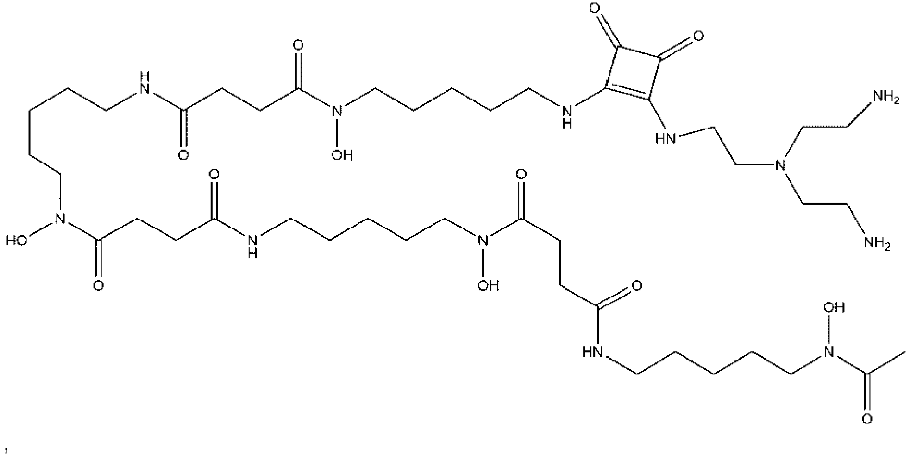 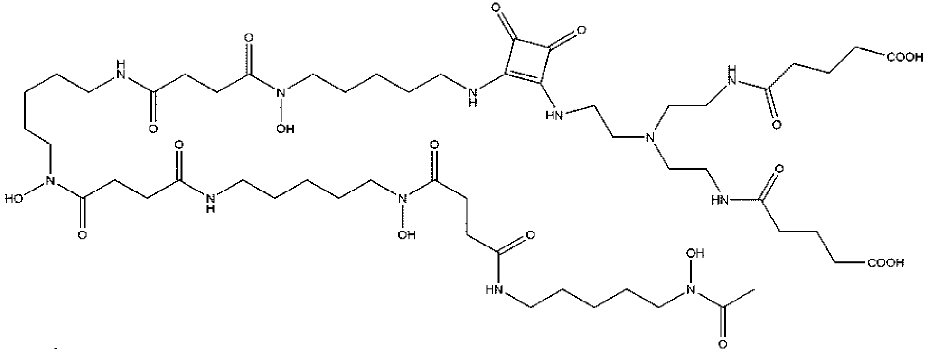 , und

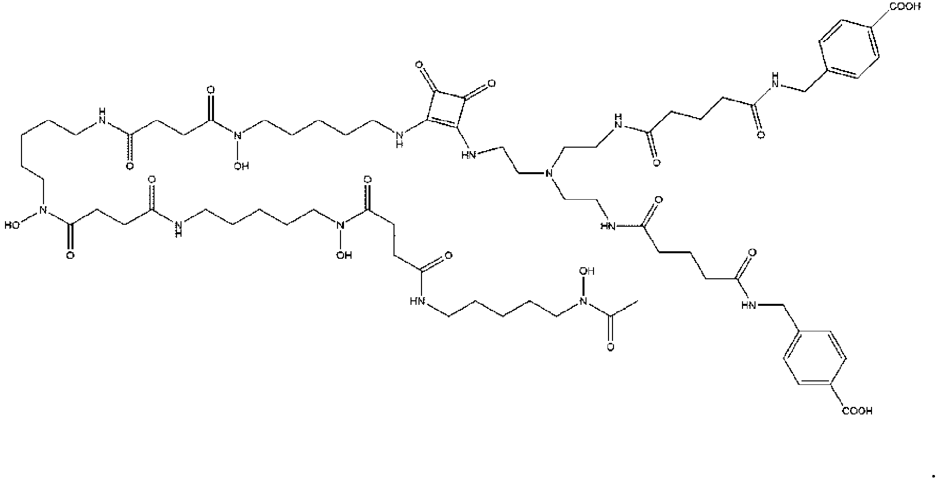

6. Konjugat, umfassend:
- eine Verbindung der Formel (I) oder ein pharmazeutisch unbedenkliches Derivat davon nach einem der Ansprüche 1 bis 5, und
- ein PSMA-Targeting-Mittel.

7. Konjugat nach Anspruch 6, wobei das Konjugat ausgewählt ist aus der Gruppe bestehend aus: 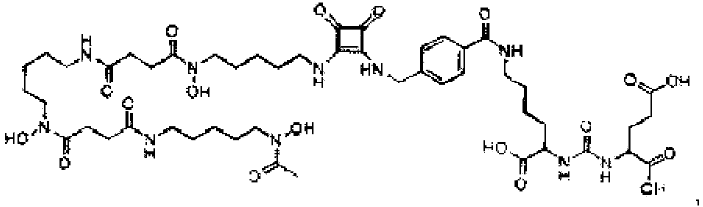 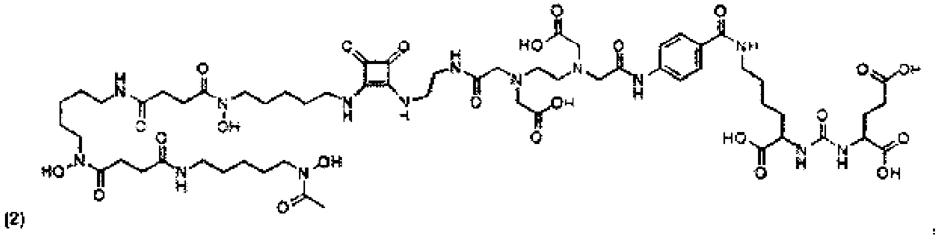 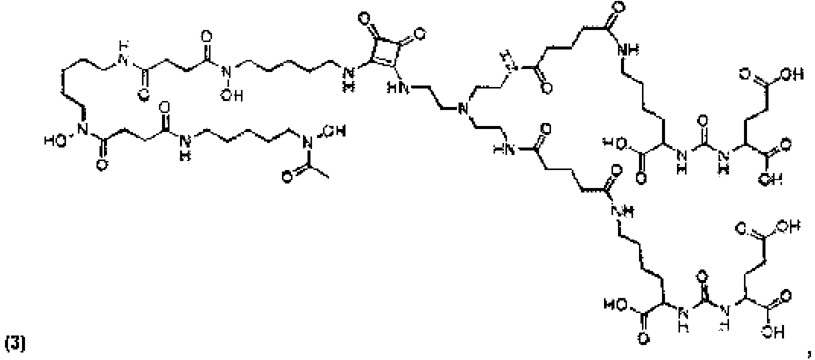 und 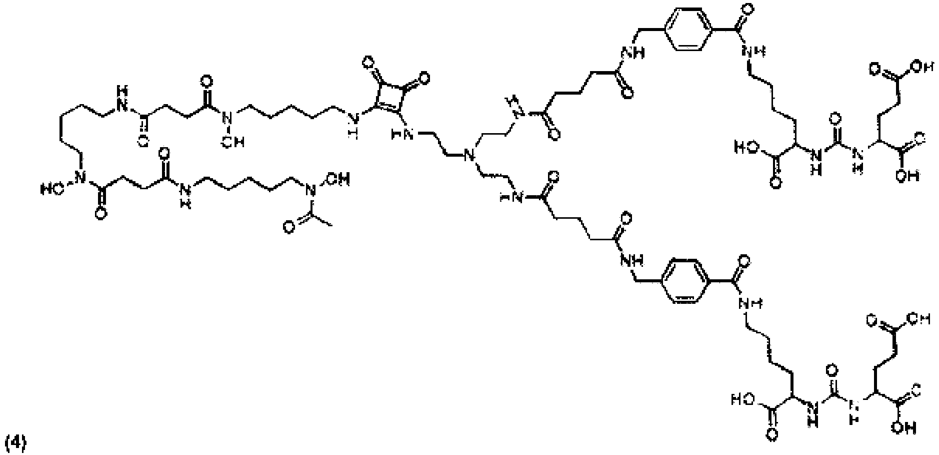 .

8. Konjugat der Formel (II) oder pharmazeutisch unbedenkliches Derivat davon: 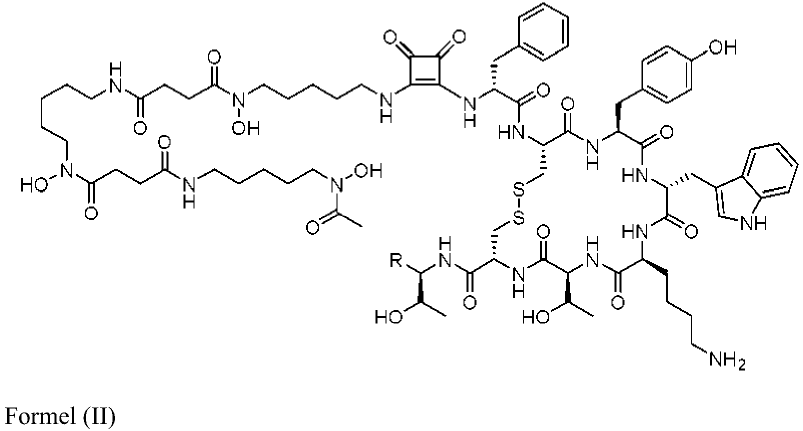
oder pharmazeutisch unbedenkliches Salz davon, wobei R CH₂OH oder COOH ist; und
wobei das pharmazeutisch unbedenkliche Derivat der Formel (II) wie folgt ist

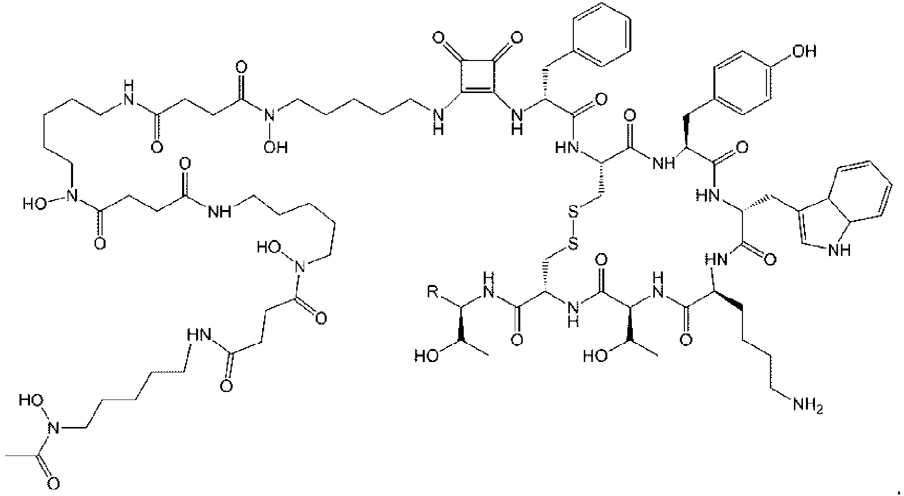

9. Radionuklidmarkiertes Konjugat, umfassend:
- ein Konjugat oder pharmazeutisch unbedenkliches Derivat davon nach einem der Ansprüche 6 bis 8, und
- ein Radionuklid, das daran komplexiert ist,
wobei das Radionuklid ausgewählt ist aus Radioisotopen von Zirkonium, Gallium, Lutetium, Holmium, Scandium, Titan, Indium und Niob, wobei bevorzugt das Radionuklid ein Radioisotop von Zirkonium (bevorzugt ⁸⁹Zr), Gallium (bevorzugt ⁶⁸Ga) oder Indium (bevorzugt ¹¹¹In) ist, wobei bevorzugter das Radionuklid ein Radioisotop von Zirkonium (bevorzugt ⁸⁹Zr) ist.

10. Pharmazeutische Zusammensetzung, umfassend das radionuklidmarkierte Konjugat nach Anspruch 9 und eine oder mehrere pharmazeutisch unbedenkliche Trägersubstanzen, Hilfsstoffe und/oder Adjuvantien, wobei bevorzugt die pharmazeutische Zusammensetzung ferner ein Arzneimittel mit Antikrebsaktivität umfasst.

11. Radionuklidmarkiertes Konjugat nach Anspruch 9 zur Verwendung in einem Verfahren zur Bildgebung eines Patienten, wobei das Verfahren Folgendes beinhaltet:
- Verabreichen des radionuklidmarkierten Konjugats an einen Patienten; und
- Bildgeben des Patienten.

12. Radionuklidmarkiertes Konjugat zur Verwendung nach Anspruch 11, wobei das Targeting-Mittel dazu dient, das Konjugat auf eine gewünschte Stelle *in vivo* zu targetieren, wobei bevorzugt die gewünschte Stelle ein Tumor ist.

13. Radionuklidmarkiertes Konjugat nach Anspruch 9 zur Verwendung in einem Verfahren zum Bildgeben einer Zelle oder *In-vitro*-Biopsieprobe, wobei das Verfahren Folgendes beinhaltet:
- Verabreichen des radionuklidmarkierten Konjugats an eine Zelle oder *In-vitro-*Biopsieprobe; und
- Bildgeben der Zelle oder *In-vitro*-Biopsieprobe.

14. Radionuklidmarkiertes Konjugat nach Anspruch 9 zur Verwendung bei der Behandlung von Krebs bei einem Patienten.

15. Kit zur Verwendung in dem Verfahren oder der Verwendung nach einem der Ansprüche 11 bis 14, wobei das Kit Folgendes umfasst:
- eine Verbindung nach einem der Ansprüche 1 bis 5, ein Konjugat nach einem der Ansprüche 6 bis 8, ein radionuklidmarkiertes Konjugat nach Anspruch 9 oder eine pharmazeutische Zusammensetzung nach Anspruch 10; und
- optional eine Markierung oder Packungsbeilage mit Anweisungen zur Verwendung in dem Verfahren oder der Verwendung nach einem der Ansprüche 11 bis 14.

## Revendications

1. Composé de formule (I), ou dérivé pharmaceutiquement acceptable de celui-ci : 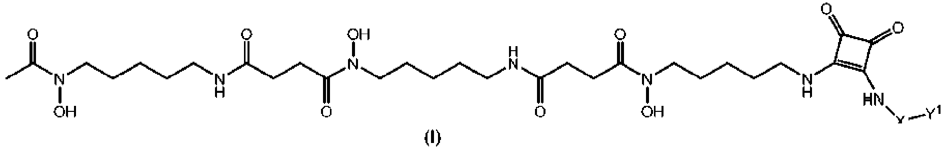 ou un sel pharmaceutiquement acceptable de celui-ci, dans lequel X est choisi dans le groupe constitué par :
un aryle éventuellement substitué par un alkyle en C₁-C₁₀, l'acide éthylènediaminetétraacétique (EDTA) ; et
(alkyle en C₁-C₁₀)NR⁷(Y²)R⁸, dans lequel R⁷ et R⁸ peuvent être indépendamment choisis parmi un alkyle en C₁-C₁₀ ou un alkyle en C₁-C₁₀-phényle, dans lequel l'alkyle en C₁-C₁₀ peut être interrompu par n groupes amido, dans lequel n vaut 0 à 3 ; et
Y¹ et Y² sont indépendamment choisis dans le groupe constitué par : un acide carboxylique, un ester, un anhydride et une amine ; et
dans lequel le dérivé pharmaceutiquement acceptable de formule (I) est :

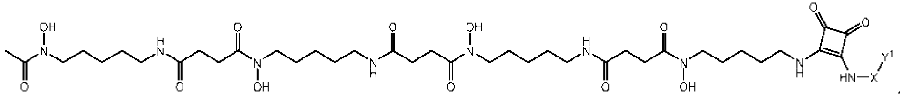

2. Composé de la revendication 1 ou dérivé pharmaceutiquement acceptable de celui-ci, dans lequel X est choisi dans le groupe constitué par : un phényle, un benzyle, un phényle fonctionnalisé par EDTA et (alkyle en C₁-C₁₀)NR⁷(Y²)R⁸.

3. Composé de l'une des revendications 1 ou 2 ou dérivé pharmaceutiquement acceptable de celui-ci, dans lequel Y¹ et Y² sont indépendamment une amine ou un acide carboxylique.

4. Composé de l'une quelconque des revendications 1 à 3, dans lequel le composé est choisi dans le groupe constitué par : 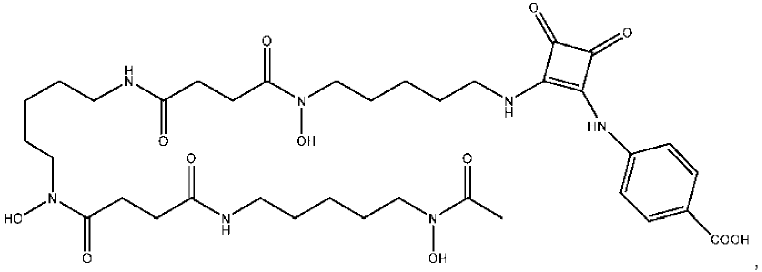 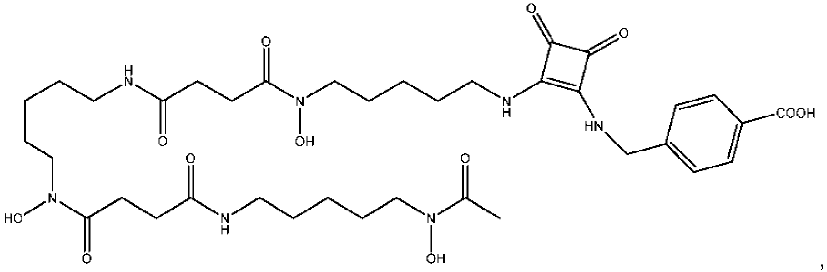 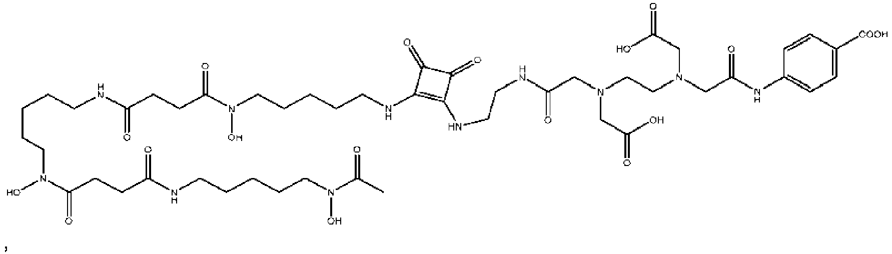 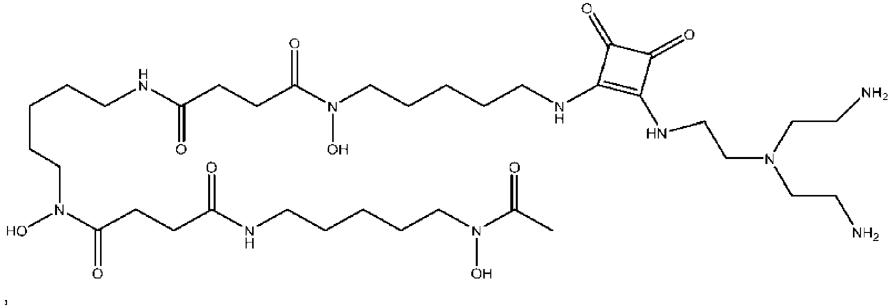 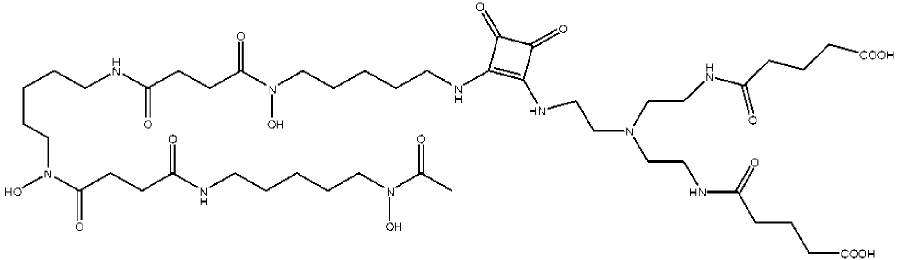 , et

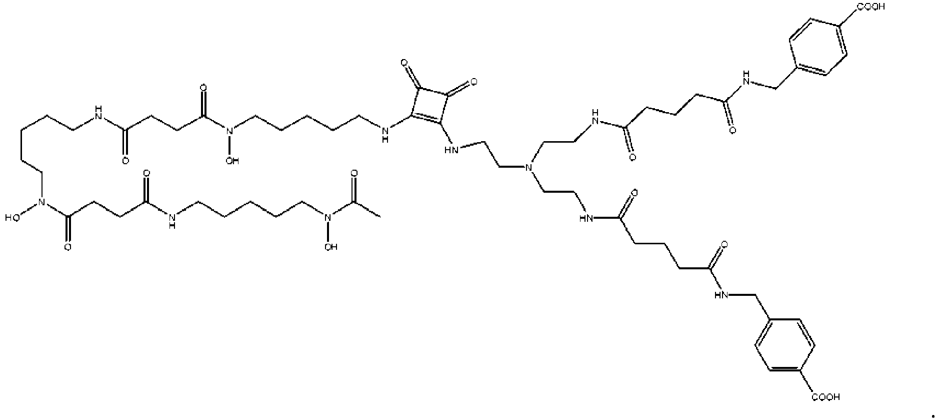

5. Composé de l'une quelconque des revendications 1 à 4, dans lequel le dérivé pharmaceutiquement acceptable est choisi dans le groupe constitué par : 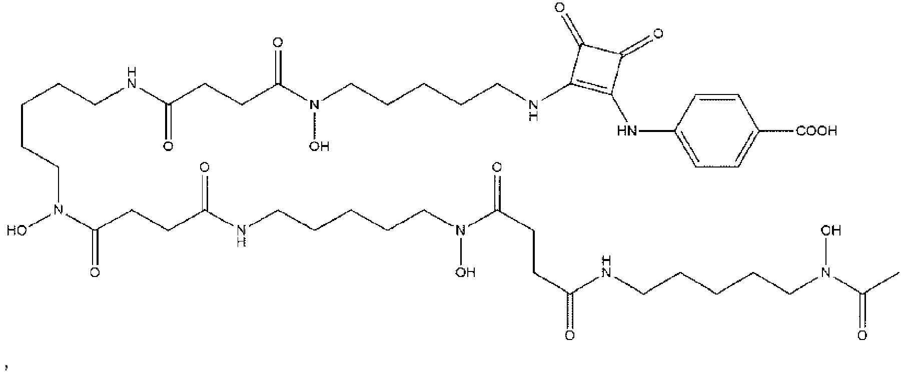 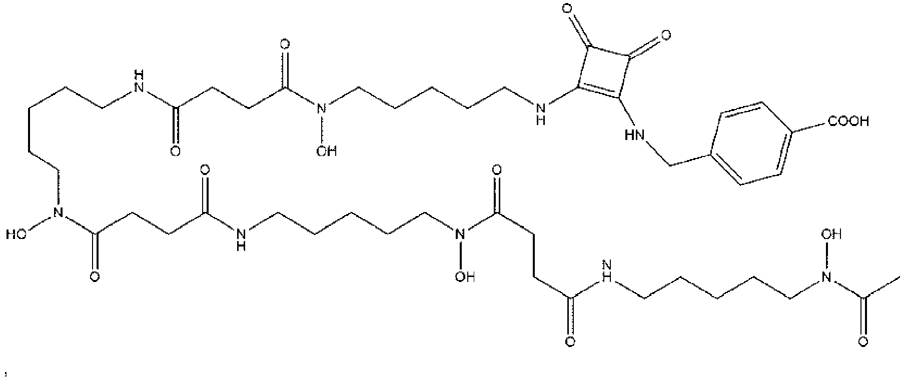 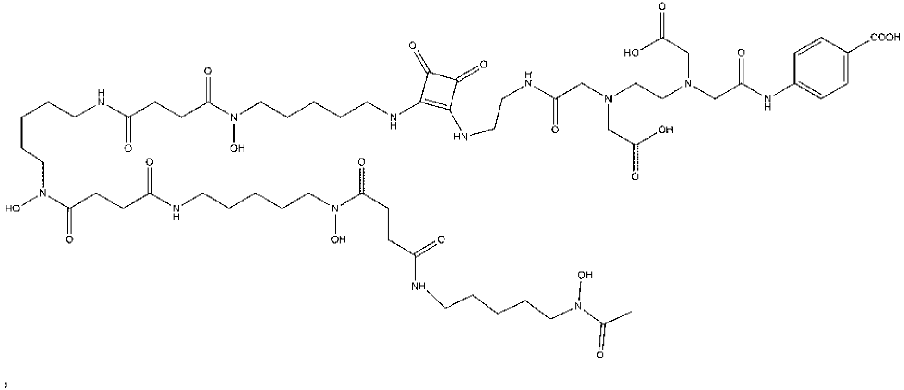 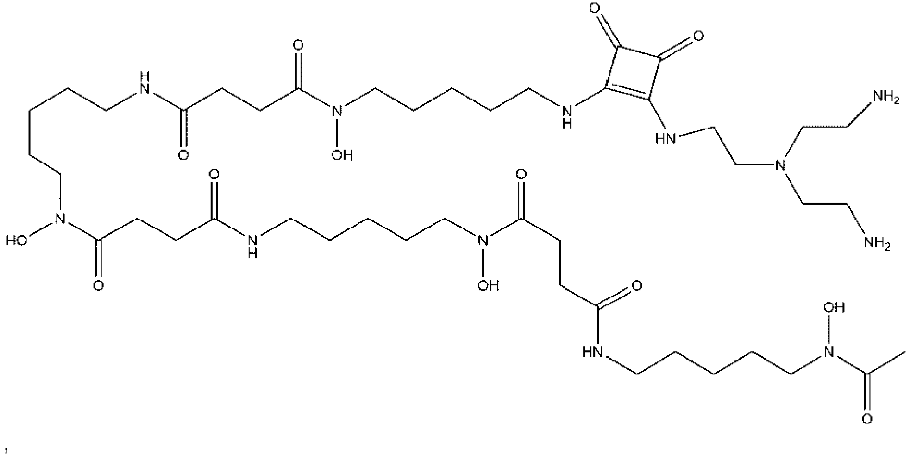 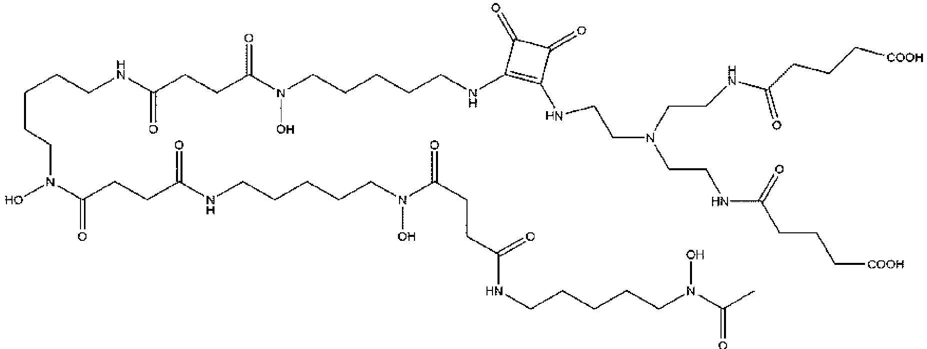 , et

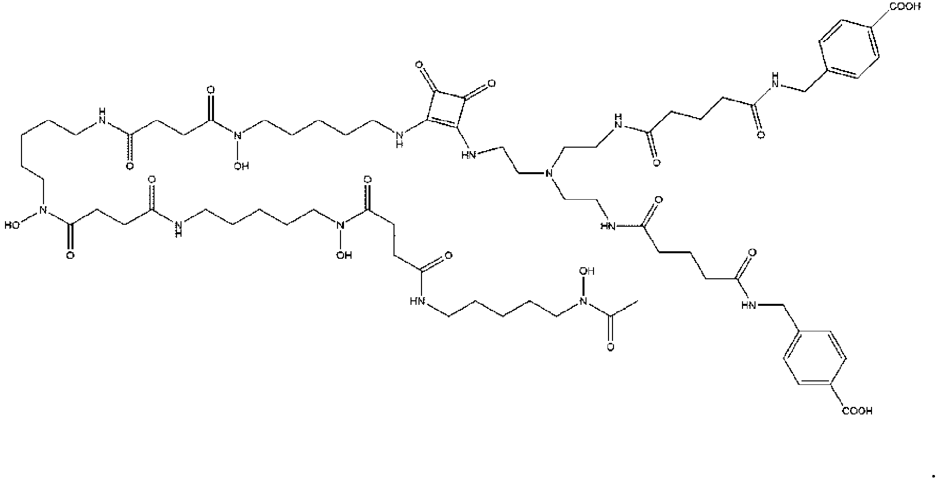

6. Conjugué comprenant :
- un composé de formule (I) ou un dérivé pharmaceutiquement acceptable de celui-ci selon l'une quelconque des revendications 1 à 5, et
- un agent de ciblage de PSMA.

7. Conjugué de la revendication 6, dans lequel le conjugué est choisi dans le groupe constitué par : 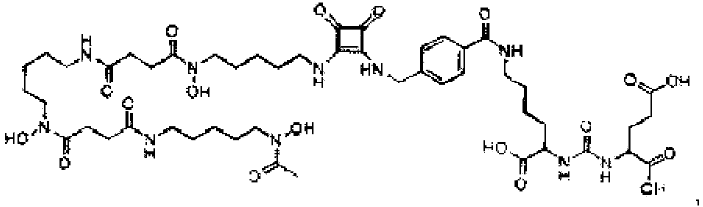 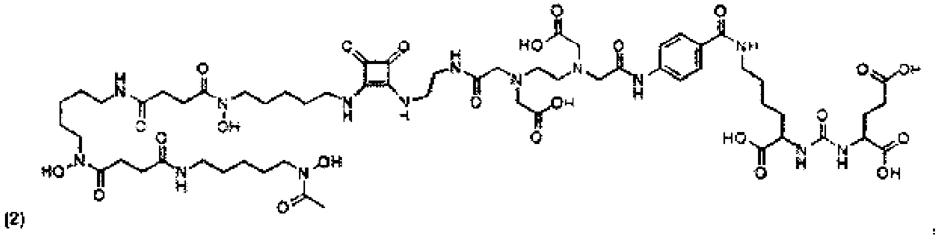 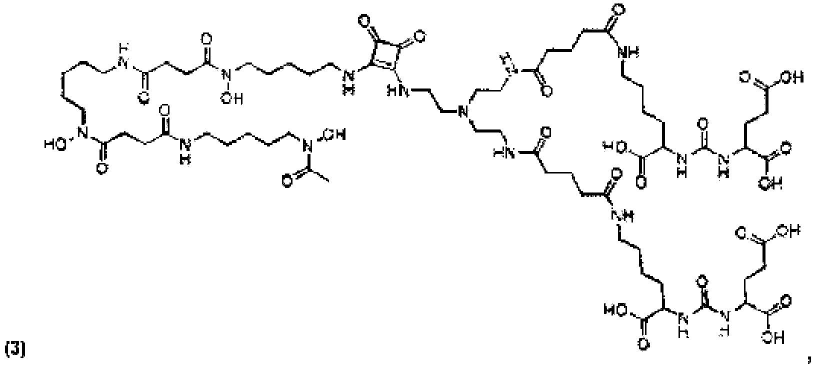 et

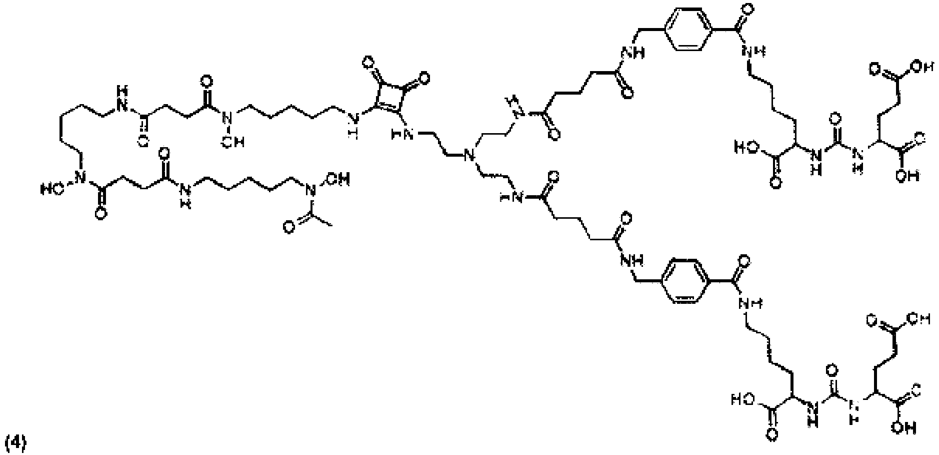

8. Conjugué de formule (II), ou dérivé pharmaceutiquement acceptable de celui-ci : 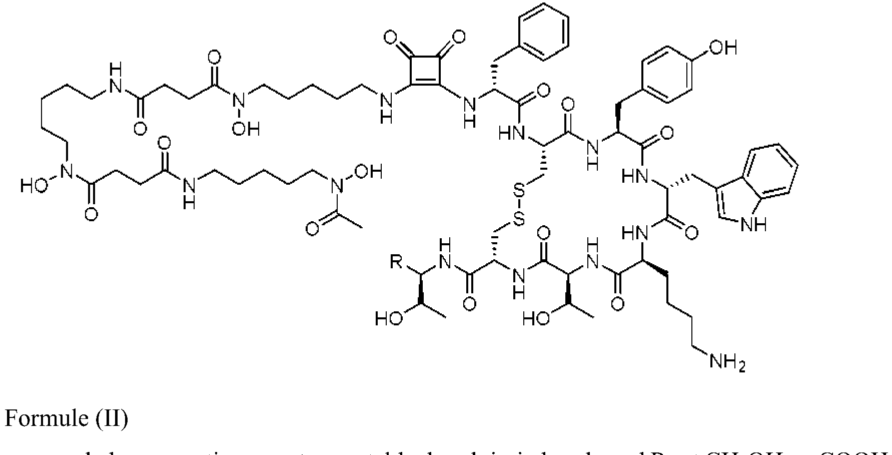
ou un sel pharmaceutiquement acceptable de celui-ci, dans lequel R est CH₂OH ou COOH ; et
dans lequel le dérivé pharmaceutiquement acceptable de formule (II) est

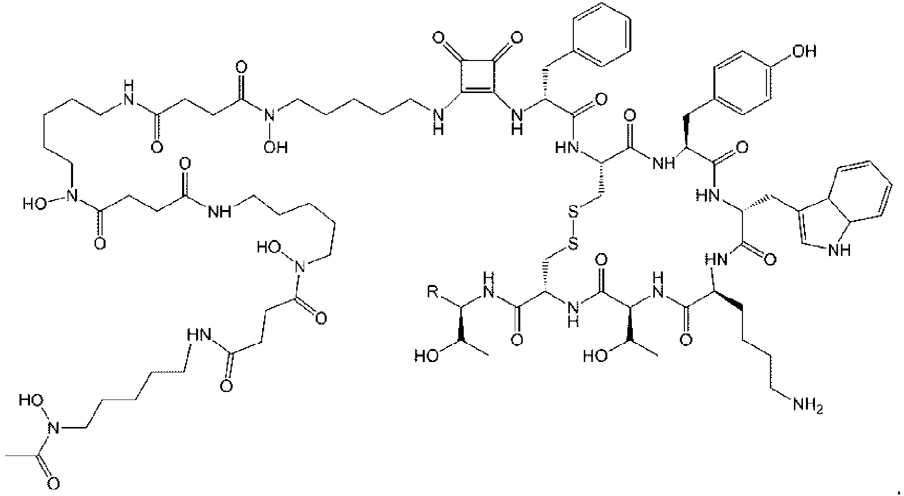

9. Conjugué marqué par un radionucléide comprenant :
- un conjugué ou un dérivé pharmaceutiquement acceptable de celui-ci de l'une quelconque des revendications 6 à 8, et
- un radionucléide complexé à celui-ci,
dans lequel le radionucléide est choisi parmi des radio-isotopes de zirconium, de gallium, de lutétium, d'holmium, de scandium, de titane, d'indium et de niobium, de préférence dans lequel le radionucléide est un radio-isotope de zirconium (de préférence ⁸⁹Zr), de gallium (de préférence ⁶⁸Ga) ou d'indium (de préférence ¹¹¹In), idéalement dans lequel le radionucléide est un radio-isotope de zirconium (de préférence ⁸⁹Zr).

10. Composition pharmaceutique comprenant le conjugué marqué par un radionucléide de la revendication 9 et une ou plusieurs substances porteuses, un ou plusieurs excipients et/ou adjuvants pharmaceutiquement acceptables, de préférence dans laquelle la composition pharmaceutique comprend en outre un médicament présentant une activité anticancéreuse.

11. Conjugué marqué par un radionucléide de la revendication 9, destiné à être utilisé dans un procédé d'imagerie d'un patient, le procédé comprenant :
- l'administration à un patient du conjugué marqué par un radionucléide ; et
- l'imagerie dudit patient.

12. Conjugué marqué par un radionucléide destiné à être utilisé selon la revendication 11, dans lequel l'agent de ciblage sert à cibler le conjugué vers un site souhaité *in vivo,* de préférence dans lequel le site souhaité est une tumeur.

13. Conjugué marqué par un radionucléide de la revendication 9, destiné à être utilisé dans un procédé d'imagerie d'une cellule ou d'un échantillon de biopsie *in vitro*, le procédé comprenant :
- l'administration à une cellule ou à un échantillon de biopsie *in vitro* du conjugué marqué par un radionucléide ; et
- l'imagerie de la cellule ou de l'échantillon de biopsie *in vitro*.

14. Conjugué marqué par un radionucléide de la revendication 9, destiné à être utilisé dans le traitement d'un cancer chez un patient.

15. Kit destiné à être utilisé dans le procédé ou l'utilisation de l'une quelconque des revendications 11 à 14, le kit comprenant :
- un composé de l'une quelconque des revendications 1 à 5, un conjugué de l'une quelconque des revendications 6 à 8, un conjugué marqué par un radionucléide de la revendication 9, ou une composition pharmaceutique de la revendication 10 ; et
- éventuellement, une étiquette ou une notice contenant des instructions d'utilisation dans le procédé ou l'utilisation de l'une quelconque des revendications 11 à 14.
